# EUROPEAN PATENT APPLICATION

(11) **EP 4 700 031 A1**
(43) Date of publication of application: **25.02.2026**
(21) Application number: 24793028.2
(22) Date of filing: 18.04.2024
(51) Int. Cl.: C07F 9/50, C07F 7/08, C07F 11/00, C07F 19/00, C07C 2/34, C07C 11/02, C07C 11/107, B01J 31/18, B01J 31/14

(54) **LIGAND COMPOUND, ORGANIC CHROMIUM COMPOUND, AND CATALYST COMPOSITION COMPRISING SAME**

(30) Priority: 19.04.2023 KR 20230051231
(71) Applicant: LG Chem, Ltd., Yeongdeungpo-gu Seoul 07336 (KR)
(72) Inventor: LIM, Won Taeck, Daejeon 34122 (KR); CHO, Yeon Ho, Daejeon 34122 (KR); KIM, Seok Sun, Daejeon 34122 (KR); JUNG, Seung Hwan, Daejeon 34122 (KR); SHIN, Eun Ji, Daejeon 34122 (KR); SA, Seok Pil, Daejeon 34122 (KR); KIM, Tae Hee, Daejeon 34122 (KR); MIN, Se Hye, Daejeon 34122 (KR)
(74) Representative: Goddar, Heinz J.
(86) International application number: PCT/KR2024/005209
(87) International publication number: WO 2024/219843

(57) **Abstract**

The present invention relates to a ligand compound having a novel structure, which exhibits high 1-hexene and 1-octene selectivity while exhibiting high catalytic activity and enables ethylene oligomerization with excellent efficiency, an organic chromium compound, a catalyst composition containing the organic chromium compound, and a method for ethylene oligomerization using the same.

## Description

### TECHNICAL FIELD

### Cross-reference to Related Applications

The present invention claims the benefit of Korean Patent Application No. 10-2023-0051231, filed on April 19, 2023, in the Korean Intellectual Property Office, the disclosure of which is incorporated in the present specification in its entirety by reference.

### TECHNICAL FIELD

The present invention relates to a ligand compound, an organic chromium compound, a catalyst composition containing the organic chromium compound, and an ethylene oligomerization method using the same.

### BACKGROUND ART

A linear alpha-olefin such as hexene or 1-octene is used as a cleaning agent, a lubricant, or a plasticizer, and it is used as a comonomer to control the polymer density, particularly in the production of linear low-density polyethylene (LLDPE).

In the conventional manufacturing process of linear low-density polyethylene (LLDPE), copolymerization was allowed to occur together with an alpha-olefin, for example, a comonomer such as 1-hexene or 1-octene, in order to control the density by forming branches in the polymer backbone together with ethylene.

Therefore, in order to manufacture LLDPE having a high comonomer content, there was a problem in that the price of the comonomer accounted for a large portion of the manufacturing cost. Various attempts have been made to solve such a problem.

Such a linear alpha-olefin was mainly produced through the Shell Higher Olefin Process. However, in this method, alpha-olefins having various lengths are simultaneously synthesized according to the Schultz-Flory distribution, and thus there was the inconvenience of having to go through an additional separation process to obtain a specific alpha-olefin.

To solve this problem, a method in which 1-hexene is selectively synthesized through a trimerization reaction of ethylene or 1-octene is selectively synthesized through a tetramerization reaction of ethylene has been proposed. In addition, much research has been being carried out on catalyst systems that enable this selective oligomerization of ethylene.

### Documents of Related Art

### Patent Document

Patent Document 1: US 5064802 B2

### DISCLOSURE OF THE INVENTION

### TECHNICAL PROBLEM

An object to be achieved by the present invention is to provide a ligand compound having a novel structure, which exhibits high 1-hexene and 1-octene selectivity while exhibiting high catalytic activity and enables ethylene oligomerization with excellent efficiency, an organic chromium compound, and a catalyst composition containing the same.

### TECHNICAL SOLUTION

In order to achieve the above object, the present invention provides a ligand compound, an organic chromium compound, a catalyst composition, and an ethylene oligomerization method.
(1) The present invention provides a ligand compound represented by Chemical Formula 1. 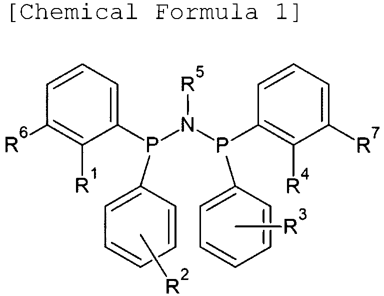 In Chemical Formula 1, R¹ is a halogen group, an alkyl group having 1 to 10 carbon atoms, which is substituted with a halogen group or unsubstituted, an alkoxy group having 1 to 10 carbon atoms, which is substituted with a halogen group or unsubstituted, an alkylthio group having 1 to 10 carbon atoms, which is substituted with a halogen group or unsubstituted, an alkyl sulfonate group having 1 to 10 carbon atoms, which is substituted with a halogen group or unsubstituted, a trialkylsilyl group (alkyl groups of the trialkylsilyl group are each independently an alkyl group having 1 to 10 carbon atoms), or binds to R⁶ to form a monocyclic or polycyclic aromatic hydrocarbon ring or a monocyclic or polycyclic heterocyclic ring; R⁶ is hydrogen in a case where R⁶ does not bind to R¹ to form a monocyclic or polycyclic aromatic hydrocarbon ring or a monocyclic or polycyclic heterocyclic ring; R² and R³ are each independently hydrogen, an alkyl group having 5 to 20 carbon atoms, a cycloalkyl group having 5 to 20 carbon atoms, an alkoxyalkyl group having 2 to 20 carbon atoms, an arylalkoxyalkyl group having 7 to 30 carbon atoms, or a trialkylsilyl group (alkyl groups of the trialkylsilyl group are each independently an alkyl group having 1 to 10 carbon atoms), where R² and R³ are not hydrogen at the same time; R⁴ is a halogen group, an alkyl group having 1 to 10 carbon atoms, which is substituted with a halogen group or unsubstituted, an alkoxy group having 1 to 10 carbon atoms, which is substituted with a halogen group or unsubstituted, an alkylthio group having 1 to 10 carbon atoms, which is substituted with a halogen group or unsubstituted, an alkyl sulfonate group having 1 to 10 carbon atoms, which is substituted with a halogen group or unsubstituted, a trialkylsilyl group (alkyl groups of the trialkylsilyl group are each independently an alkyl group having 1 to 10 carbon atoms), or binds to R⁷ to form a monocyclic or polycyclic aromatic hydrocarbon ring or a monocyclic or polycyclic heterocyclic ring; R⁷ is hydrogen in a case where R⁷ does not bind to R⁴ to form a monocyclic or polycyclic aromatic hydrocarbon ring or a monocyclic or polycyclic heterocyclic ring; and R⁵ is an alkyl group having 1 to 20 carbon atoms, an alkyl group having 1 to 20 carbon atoms, which is substituted with an aryl group having 6 to 10 carbon atoms, a cycloalkyl group having 5 to 10 carbon atoms, or a cycloalkyl group having 5 to 10 carbon atoms, which is fused with an aryl group having 6 to 10 carbon atoms.
(2) The present invention provides the ligand compound according to (1),
   wherein R¹ is fluorine, an alkyl group having 1 to 5 carbon atoms, which is substituted with fluorine or unsubstituted, an alkoxy group having 1 to 5 carbon atoms, which is substituted with fluorine or unsubstituted, an alkylthio group having 1 to 5 carbon atoms, which is substituted with fluorine or unsubstituted, an alkyl sulfonate group having 1 to 5 carbon atoms, which is substituted with fluorine or unsubstituted, a trialkylsilyl group (alkyl groups of the trialkylsilyl group are each independently an alkyl group having 1 to 5 carbon atoms), or binds to R⁶ to form a monocyclic or polycyclic heterocyclic ring, and
   R⁴ is fluorine, an alkyl group having 1 to 5 carbon atoms, which is substituted with fluorine or unsubstituted, an alkoxy group having 1 to 5 carbon atoms, which is substituted with fluorine or unsubstituted, an alkylthio group having 1 to 5 carbon atoms, which is substituted with fluorine or unsubstituted, an alkyl sulfonate group having 1 to 5 carbon atoms, which is substituted with fluorine or unsubstituted, a trialkylsilyl group (alkyl groups of the trialkylsilyl group are each independently an alkyl group having 1 to 5 carbon atoms), or binds to R⁷ to form a monocyclic or polycyclic heterocyclic ring.
(3) The present invention provides the ligand compound according to (1) or (2),
   wherein R¹ is a fluoro group, a trifluoromethyl group, a methoxy group, a methyl group, a trifluoromethoxy group, a trifluoromethylthio group, a methylthio group, a methyl sulfonate group, or a trimethylsilyl group, or binds to R⁶ to form furan or dibenzofuran, and
   R⁴ is a fluoro group, a trifluoromethyl group, a methoxy group, a methyl group, a trifluoromethoxy group, a trifluoromethylthio group, a methylthio group, a methyl sulfonate group, or a trimethylsilyl group, or binds to R⁷ to form furan or dibenzofuran.
(4) The present invention provides the ligand compound according to any one of (1) to (3),
   wherein R² and R³ are each independently an alkyl group having 8 to 12 carbon atoms, a tripropylsilyl group, or a tributylsilyl group.
(5) The present invention provides the ligand compound according to any one of (1) to (4),
   wherein R² and R³ are each independently an n-decyl group, a tripropylsilyl group, or a tributylsilyl group.
(6) The present invention provides the ligand compound according to any one of (1) to (5),
   wherein R⁵ is an alkyl group having 3 to 5 carbon atoms, an alkyl group having 1 to 5 carbon atoms, which is substituted with an aryl group having 6 to 10 carbon atoms, a cycloalkyl group having 5 to 8 carbon atoms, or a cycloalkyl group having 5 to 8 carbon atoms, which is fused with an aryl group having 6 to 10 carbon atoms.
(7) The present invention provides the ligand compound according to any one of (1) to (6),
   wherein the ligand compound represented by Chemical Formula 1 is represented by any one of Chemical Formula 2 to Chemical Formula 5. 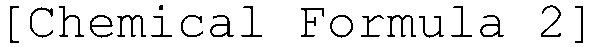 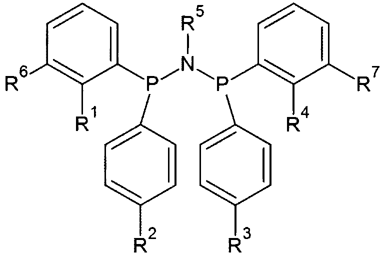 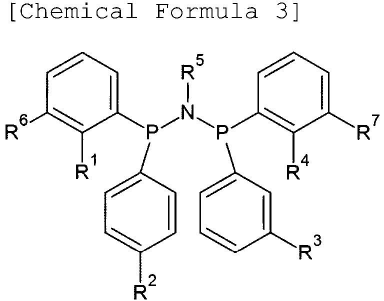 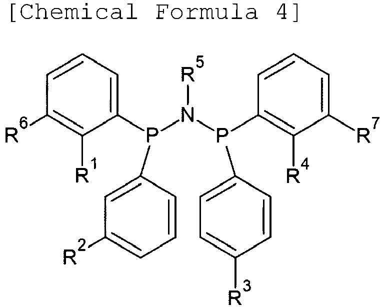 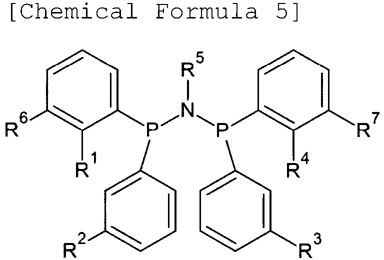 In Chemical Formula 2 to Chemical Formula 5, R¹ to R⁷ are respectively the same as those defined in (1) to (6).
(8) The present invention provides the ligand compound according to any one of (1) to (7), wherein the ligand compound represented by Chemical Formula 1 is represented by any one of Chemical Formula 2-1 to Chemical Formula 2-76 or Chemical Formula 5-1 to Chemical Formula 5-20. 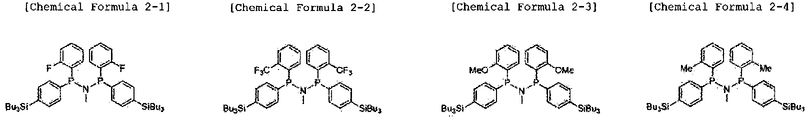 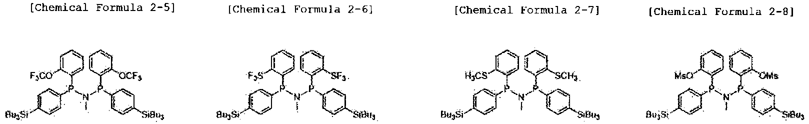 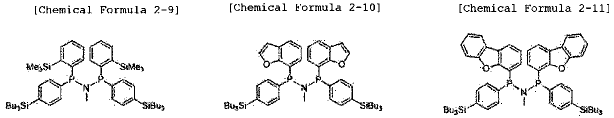 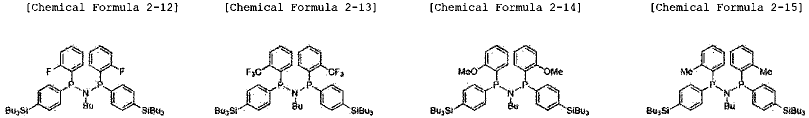 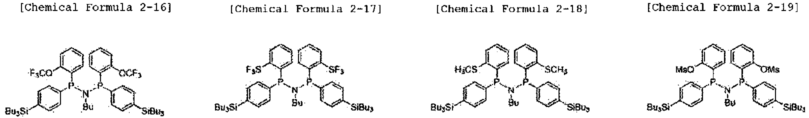 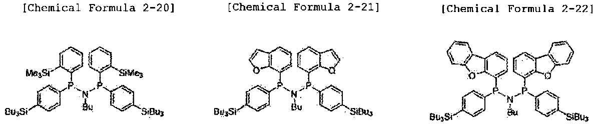 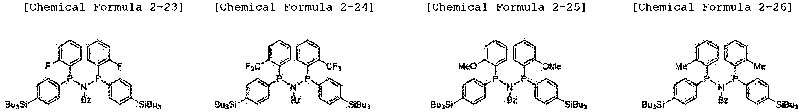 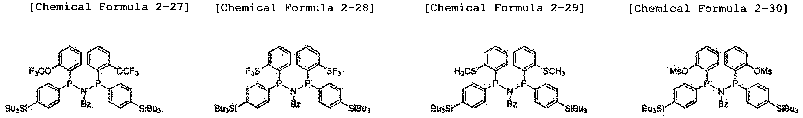 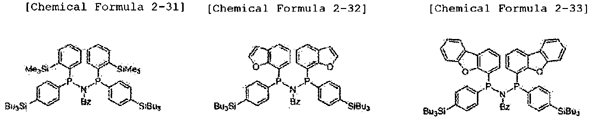 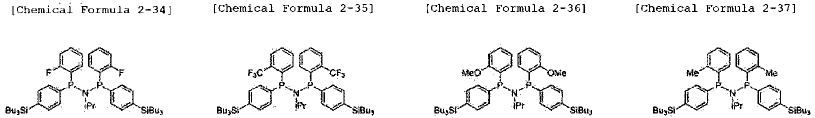 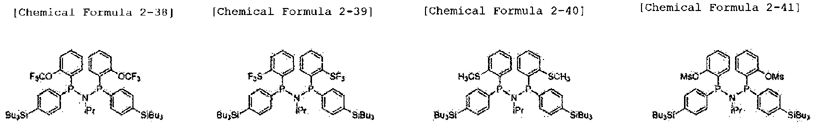 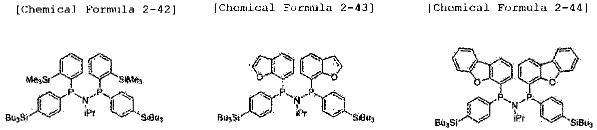 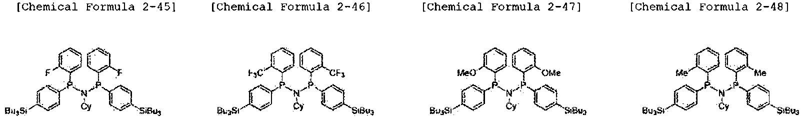 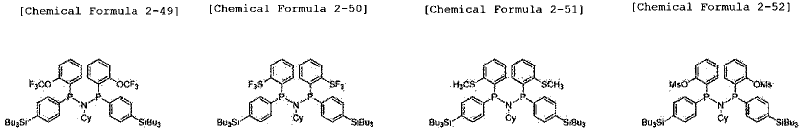 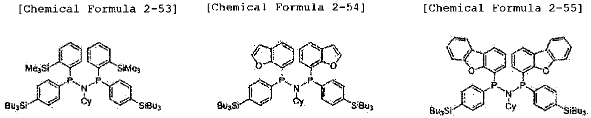 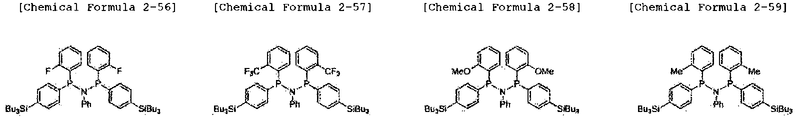 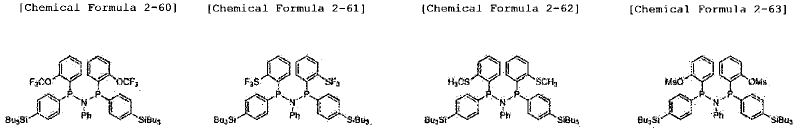 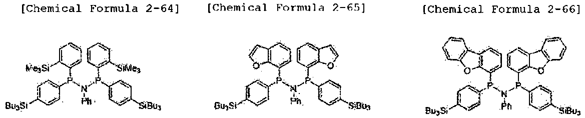 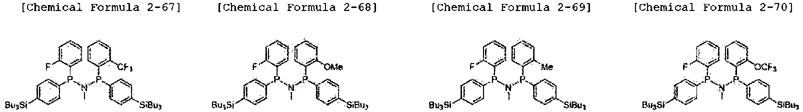 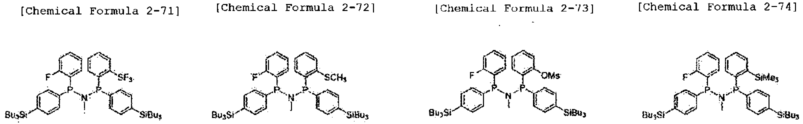 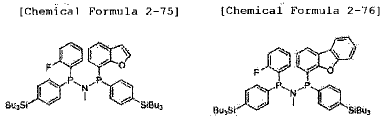 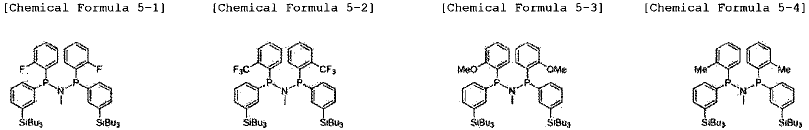 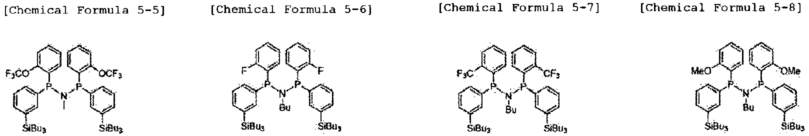 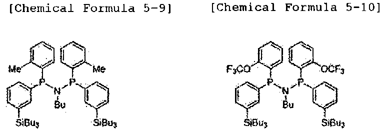 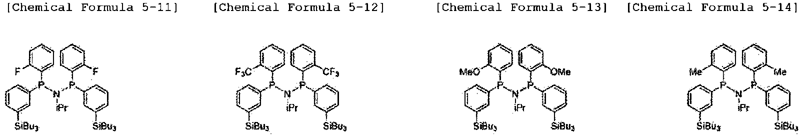 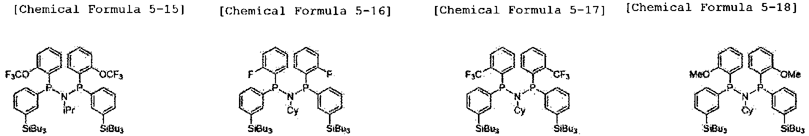 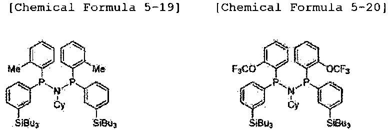
(9) The present invention provides an organic chromium compound including the ligand compound according to any one of (1) to (8), and chromium coordinated to the ligand compound.
(10) The present invention provides the organic chromium compound according to (9), wherein the organic chromium compound has a form in which an unshared electron pair of any one or more of N and two P's is coordinated to chromium in the ligand compound represented by Chemical Formula 1.
(11) The present invention provides a catalyst composition including the ligand compound according to any one of (1) to (8), chromium, and a co-catalyst.
(12) The present invention provides the catalyst composition according to (11), wherein the chromium is derived from a chromium source, and the chromium source contains one or more selected from the group consisting of chromium (III) acetylacetonate, chromium (III) chloride tetrahydrofuran, chromium (III) 2-ethylhexanoate, chromium (III) acetate, chromium (III) butyrate, chromium (III) pentanoate, chromium (III) laurate, chromium (III) tris(2,2,6,6-tetramethyl-3.5-heptanedionate), and chromium (III) stearate.
(13) The present invention provides the catalyst composition according to (11) or (12), wherein the co-catalyst is at least one or more selected from the group consisting of compounds represented by Chemical Formula 6 to Chemical Formula 9.

   [Chemical Formula 6] - [Al(R¹³)-O]ₐ-

   In Chemical Formula 6, R¹³'s are each independently a halogen group, a hydrocarbyl group having 1 to 20 carbon atoms, or a hydrocarbyl group having 1 to 20 carbon atoms, which is substituted with a halogen group, and a is an integer of 2 or more.

   [Chemical Formula 7] E(R¹⁴)₃

   In Chemical Formula 7, E is aluminum or boron, and R¹⁴'s are each independently hydrogen, a halogen group, a hydrocarbyl group having 1 to 20 carbon atoms, or a hydrocarbyl group having 1 to 20 carbon atoms, which is substituted with a halogen group,

   [Chemical Formula 8] [L-H]⁺[G(Y)₄]⁻

   [Chemical Formula 9] [L]⁺[G(Y)₄]⁻

   In Chemical Formulae 8 and 9, L is a neutral or cationic Lewis acid, [LH]⁺ is Brønsted acid, G is a Group 13 element, and Y's are each independently a substituted or unsubstituted alkyl group having 1 to 20 carbon atoms or a substituted or unsubstituted aryl group having 6 to 20 carbon atoms, where in a case where the alkyl group or the aryl group is substituted, a substituent is a halogen group, a hydrocarbyl group having 1 to 20 carbon atoms, an alkoxy group having 1 to 20 carbon atoms, or an aryloxy group having 6 to 20 carbon atoms.
(14) The present invention provides a production method for a linear alpha-olefin including a step (S10) of oligomerizing ethylene in the presence of the catalyst composition according to any one of (11) to (13).
(15) The present invention provides the production method for a linear alpha-olefin according to (14), wherein the linear alpha-olefin is 1-hexene, 1-octene, or a mixture thereof.

### ADVANTAGEOUS EFFECTS

In a case where ethylene oligomerization is carried out using an organic chromium compound and a catalyst composition, which contain the ligand compound according to the present invention, it is possible to produce a linear alpha-olefin with high 1-hexene and 1-octene selectivity while maintaining excellent productivity due to high catalytic activity.

### MODE FOR CARRYING OUT THE INVENTION

Hereinafter, the present invention will be described in more detail to facilitate understanding of the present invention.

The terms and words, which are used in the description of the present specification and claims, are not to be restrictively construed in their ordinary or dictionary sense and are to be construed in the sense and with the concepts consistent with the technical ideas of the present invention based on the principle that an inventor may properly define concepts of terms to describe the invention of the inventor in the best way.

### Ligand Compound

The present invention provides a ligand compound applicable to a catalyst that is used in an ethylene oligomerization reaction. In a case where the ligand compound is applied to an ethylene oligomerization reaction, specifically, to a catalyst composition for forming a linear alpha-olefin formation, high selectivity for a linear alpha-olefin is obtained while excellent catalytic activity is exhibited. In particular, as compared with the existing PNP catalysts as well as a catalyst including a symmetrical ligand compound, the amount of the produced solid polyethylene is small even under the same reaction conditions, and thus a linear alpha-olefin can be produced more efficiently.

According to an embodiment of the present invention, an organic chromium compound coordinated with the ligand compound can be used in the production of the linear alpha-olefin using ethylene, and it is possible to form, with high selectivity, an alpha-olefin having a liquid form, specifically, 1-hexene or 1-octene having a liquid form since the oligomerization reaction proceeds in a reaction under conditions in terms of ethylene. This is because the selectivity for an alpha-olefin having a specific length increases through a transition state in which a metal-containing ring compound (metallacycle) is formed in the oligomerization reaction of ethylene.

According to an embodiment of the present invention, the ligand compound includes a diphosphino aminyl moiety, and an aryl having a specific substituent is linked to a terminal of the diphosphino aminyl moiety, and thus it can have by itself a form that can serve as a strong electron donating group. Due to such structural characteristics, the ligand compound can be applied to an ethylene oligomerization catalyst system to exhibit high activity, and in particular, it can exhibit high selectivity for 1-hexene, 1-octene, and the like. This may be due to the interaction between the respective adjacent chromium active site, and this is because, in particular, in a case where an aryl substituted with a specific substituent is linked to the phosphorus (P) atom of diphosphino aminyl, the electron density increases at the (P) atom and the nitrogen (N) atom which are contained in diphosphino aminyl, and then the electrical and three-dimensional properties of the entire ligand compound change. As a result, a change occurs in the bond between the ligand and the chromium atom, which makes the structure of the catalyst more stable. In addition, the energy of the transition state (activation energy) is changed as compared with the existing metallacycloheptane or metallacyclononane form, which makes it possible to form the alpha-olefin with higher activity and selectivity and makes it possible to further reduce the amounts of by-products such as a solid alpha-olefin having a high molecular weight, such as polyethylene wax (PE wax).

According to an embodiment of the present invention, the ligand compound is characterized in that a phenyl located at a terminal of the diphosphino aminyl moiety has, at the ortho position as a substituent, a halogen group, an alkyl group having 1 to 10 carbon atoms, which is substituted with a halogen group or unsubstituted, an alkoxy group having 1 to 10 carbon atoms, which is substituted with a halogen group or unsubstituted, an alkylthio group having 1 to 10 carbon atoms, which is substituted with a halogen group or unsubstituted, an alkyl sulfonate group having 1 to 10 carbon atoms, which is substituted with a halogen group or unsubstituted, or a trialkylsilyl group (alkyl groups of the trialkylsilyl group are each independently an alkyl group having 1 to 10 carbon atoms), or fused with phenyl to form a monocyclic or polycyclic aromatic hydrocarbon ring or a monocyclic or polycyclic heterocyclic ring, and has, at each of the meta position and the para position as a substituent, an alkyl group having a specific number of carbon atoms or a silyl group substituted with an alkyl group having a specific number of carbon atoms. The substituent substituted at the ortho position of the phenyl increases the steric strain around the metal atom, which makes it possible to increase the selectivity for 1-hexene. In addition, it protects the metal atom or directly forms a coordinate bond, which makes it possible to play a role in improving the stability of the metal complex compound. At the same time, the substituents substituted at the meta and para positions of the phenyl increase the solubility of the ligand compound and the metal complex compound in various polymerization solvents, which makes it possible to improve activity and selectivity. Accordingly, in a case where the ligand compound is used, a chromium catalyst having high stability, excellent activity, and excellent selectivity can be produced.

According to an embodiment of the present invention, the ligand compound can further improve catalyst stability and activity since a bulky substituent such as a cycloalkyl group or a phenyl group is bonded to the nitrogen atom to which two phosphorus atoms are bonded, and the bulky substituent bonded to nitrogen prevents the rotation of the bond between nitrogen and phosphorus. In this case, the activity, stability, and selectivity of the catalyst change depending on the three-dimensional properties of the substituent bonded to the nitrogen atom. In a case where the steric strain of the substituent bonded to the nitrogen atom is too high, there is a problem that the ligand synthesis and the formation of a metal complex compound are difficult, and the generated complex compound is unstable. In addition, in a case where the steric strain of the substituent bonded to the nitrogen atom is too high, there is a problem that access to a raw material such as ethylene is difficult, and thus the activity of the catalyst is reduced. In addition, in a case where the steric strain of the substituent bonded to the nitrogen atom is too low, the rotation of the bond between the nitrogen atom and the phosphorus atom is not prevented, and the metal center atom is not protected, which causes a decrease in the activity and stability of the catalyst. That is, in a case where the steric strain of the substituent bonded to the nitrogen atom is too high or too low, the activity of the catalyst is lowered, and the stability thereof is reduced, which causes a problem that the amount of the produced by-products such as polyethylene wax increases. As a result, it is very important to select a substituent bonded to the nitrogen atom, which has an appropriate level of steric strain with respect to the substituent bonded to the phosphorus atom. In the ligand compound according to the present invention, the steric strain around the PNP functional group increases as a substituent is introduced at the ortho position of the phenyl group bonded to the phosphorus atom. Therefore, in a case where a substituent represented by R⁵ in Chemical Formula 1 is introduced so that the steric strain of the substituent bonded to the nitrogen atom does not increase too much, yield and selectivity can be improved at the time of the oligomerization reaction of ethylene, by using a catalyst composition containing the ligand compound according to the present invention. In particular, in a case where a substituent having a form of a secondary alkyl group is introduced as the substituent represented by R⁵ in Chemical Formula 1, the efficiency is further improved, and regarding a substituent having a form of a primary alkyl group, an appropriate steric strain can be formed by introducing an aryl group such as a phenyl group at the position of the 1st carbon or the 2nd carbon in order to compensate for the low steric strain, which makes it possible to improve yield and selectivity.

According to an embodiment of the present invention, the ligand compound may be represented by Chemical Formula 1.

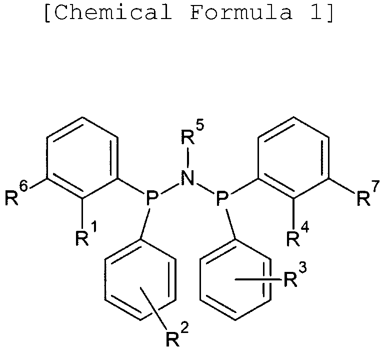

In Chemical Formula 1, R¹ is a halogen group, an alkyl group having 1 to 10 carbon atoms, which is substituted with a halogen group or unsubstituted, an alkoxy group having 1 to 10 carbon atoms, which is substituted with a halogen group or unsubstituted, an alkylthio group having 1 to 10 carbon atoms, which is substituted with a halogen group or unsubstituted, an alkyl sulfonate group having 1 to 10 carbon atoms, which is substituted with a halogen group or unsubstituted, a trialkylsilyl group (alkyl groups of the trialkylsilyl group are each independently an alkyl group having 1 to 10 carbon atoms), or binds to R⁶ to form a monocyclic or polycyclic aromatic hydrocarbon ring or a monocyclic or polycyclic heterocyclic ring; R⁶ is hydrogen in a case where R⁶ does not bind to R¹ to form a monocyclic or polycyclic aromatic hydrocarbon ring or a monocyclic or polycyclic heterocyclic ring; R² and R³ are each independently hydrogen, an alkyl group having 5 to 20 carbon atoms, a cycloalkyl group having 5 to 20 carbon atoms, an alkoxyalkyl group having 2 to 20 carbon atoms, an arylalkoxyalkyl group having 7 to 30 carbon atoms, or a trialkylsilyl group (alkyl groups of the trialkylsilyl group are each independently an alkyl group having 1 to 10 carbon atoms), where R³ and R⁴ are not hydrogen at the same time; R⁴ is a halogen group, an alkyl group having 1 to 10 carbon atoms, which is substituted with a halogen group or unsubstituted, an alkoxy group having 1 to 10 carbon atoms, which is substituted with a halogen group or unsubstituted, an alkylthio group having 1 to 10 carbon atoms, which is substituted with a halogen group or unsubstituted, an alkyl sulfonate group having 1 to 10 carbon atoms, which is substituted with a halogen group or unsubstituted, a trialkylsilyl group (alkyl groups of the trialkylsilyl group are each independently an alkyl group having 1 to 10 carbon atoms), or binds to R⁷ to form a monocyclic or polycyclic aromatic hydrocarbon ring or a monocyclic or polycyclic heterocyclic ring; R⁷ is hydrogen in a case where R⁷ does not bind to R⁴ to form a monocyclic or polycyclic aromatic hydrocarbon ring or a monocyclic or polycyclic heterocyclic ring; and R⁵ is an alkyl group having 1 to 20 carbon atoms, an alkyl group having 1 to 20 carbon atoms, which is substituted with an aryl group having 6 to 10 carbon atoms, a cycloalkyl group having 5 to 10 carbon atoms, or a cycloalkyl group having 5 to 10 carbon atoms, which is fused with an aryl group having 6 to 10 carbon atoms.

In the present invention, the "halogen group" means fluorine (-F), chlorine (-Cl), bromine (-Br), and iodine (-I).

In the present invention, the phrase "substituted with a halogen group" may mean that at least one hydrogen atom bonded to a carbon atom of each substituent is substituted with a halogen group atom, and in a case where the number of carbon atoms of each substituent is referred to, it may mean the number of carbon atoms of each substituent.

In the present invention, the "alkyl group" may mean a linear or branched hydrocarbon residue, and specific examples thereof may include a methyl group, an ethyl group, an n-propyl group, an isopropyl group, an n-butyl group, an isobutyl group, a t-butyl group, an n-pentyl group, an isopentyl group, and a hexyl group, depending on the number of carbon atoms defined.

In the present invention, the "alkylthio group" may mean an alkylthio group in which any one substituent around the sulfide bond as the center is an alkyl group, and in a case where the number of carbon atoms is referred to, it may mean the number of carbon atoms of the alkyl group.

In the present invention, the "alkyl sulfonate group" may mean an alkyl sulfonate group in which the sulfur atom of the sulfonate is substituted with an alkyl group, and in a case where the number of carbon atoms is referred to, it may mean the number of carbon atoms of the alkyl group.

In the present invention, the "trialkylsilyl group" is represented by -SiR₃, where each R independently means an alkyl group that serves as a substituent, and in a case where the number of carbon atoms of the trialkylsilyl group is referred to, it may mean the sum of the number of carbon atoms of R's.

In the present invention, the "monocyclic aromatic hydrocarbon ring" may mean an aromatic hydrocarbon consisting of one ring, such as a benzene ring, and the "polycyclic aromatic hydrocarbon ring" may mean an aromatic hydrocarbon consisting of two or more rings, such as a naphthalene ring.

In the present invention, the "monocyclic heterocyclic ring" may mean a heterocyclic ring consisting of one ring containing a hetero atom, such as furan, thiophene, pyrrole, or pyridine, and the "polycyclic heterocyclic ring" may mean a heterocyclic ring consisting of two or more rings containing a hetero atom, such as benzofuran, dibenzofuran, benzothiophene, dibenzothiophene, benzopyrrole, or dibenzopyrrole. The heterocyclic ring may form an aliphatic or aromatic ring.

In the present invention, the "cycloalkyl group" may mean a hydrocarbon residue having a ring form, and specific examples may include a cyclopentyl group, a cyclohexyl group, a cycloheptyl group, and a cyclooctyl group, depending on the number of carbon atoms defined.

In the present invention, the "alkoxyalkyl group" may mean an alkyl group substituted with an alkoxy group, and in a case where the number of carbon atoms of the alkoxyalkyl group is referred to, it may mean the sum of the number of carbon atoms of the alkoxy group and the alkyl group.

In the present invention, the "arylalkoxyalkyl group" may mean an alkyl group substituted with an alkoxy group containing an aryl group as a substituent, and in a case where the number of carbon atoms of the arylalkoxyalkyl group is referred to, it may mean the sum of the number of carbon atoms of the alkoxy group and the alkyl group.

In the present invention, unless otherwise specified, "aryl" refers to an optionally substituted benzene ring or refers to a ring system that can be formed by fusing one or more optional substituents. The exemplary optional substituent includes a substituted alkyl group having 1 to 2 carbon atoms, a substituted alkenyl group having 2 to 3 carbon atoms, a substituted alkynyl group having 2 to 3 carbon atoms, a heteroaryl group, a heterocyclic group, an aryl group, alkoxy optionally having 1 to 3 fluorine substituents, aryloxy, aralkoxy, acyl, aroyl, heteroaroyl, acyloxy, aroyloxy, heteroaroyloxy, sulphanyl, sulfinyl, sulfonyl, aminosulfonyl, sulfonylamino, carboxyamide, aminocarbonyl, carboxy, oxo, hydroxy, mercapto, amino, nitro, cyano, halogen, or ureido. This ring or ring system may be optionally fused to an aryl ring (for example, a benzene ring), a carbocyclic ring, or a heterocyclic ring, which optionally has one or more substituents. It may include, but not limited to, phenyl, naphthyl, tetrahydronaphthyl, biphenyl, indanyl, anthracyl, and phenanthryl, and substituted derivatives thereof.

In the present invention, the abbreviation "Me" means a methyl group, the abbreviation "iPr" means an isopropyl group, the abbreviation "Bu" means an n-butyl group, the abbreviation "Cy" means a cyclohexyl group, the abbreviation "Ph" means a phenyl group, the abbreviation "Bz" means a benzyl group, and the abbreviation "MsO-" or "-OMs" means a methyl sulfonate group (MeO₂SO-).

According to an embodiment of the present invention, R¹ may be such a group that is a fluoro group, a chloro group, a bromo group, an alkyl group having 1 to 5 carbon atoms, which is substituted with fluorine or unsubstituted, an alkoxy group having 1 to 5 carbon atoms, which is substituted with fluorine or unsubstituted, an alkylthio group having 1 to 5 carbon atoms, which is substituted with fluorine or unsubstituted, an alkyl sulfonate group having 1 to 5 carbon atoms, which is substituted with fluorine or unsubstituted, a trialkylsilyl group (alkyl groups of the trialkylsilyl group are each independently an alkyl group having 1 to 5 carbon atoms), or that binds to R⁶ to form a monocyclic or polycyclic heterocyclic ring. As a specific example, R¹ may be such a group that is a fluoro group, a trifluoromethyl group, a methoxy group, a methyl group, a trifluoromethoxy group, a trifluoromethylthio group, a methylthio group, a methyl sulfonate group, or a trimethylsilyl group, or that binds to R⁶ to form furan or dibenzofuran.

According to an embodiment of the present invention, R⁴ may be such a group that is a fluoro group, a chloro group, a bromo group, an alkyl group having 1 to 5 carbon atoms, which is substituted with fluorine or unsubstituted, an alkoxy group having 1 to 5 carbon atoms, which is substituted with fluorine or unsubstituted, an alkylthio group having 1 to 5 carbon atoms, which is substituted with fluorine or unsubstituted, an alkyl sulfonate group having 1 to 5 carbon atoms, which is substituted with fluorine or unsubstituted, a trialkylsilyl group (alkyl groups of the trialkylsilyl group are each independently an alkyl group having 1 to 5 carbon atoms), or that binds to R⁷ to form a monocyclic or polycyclic heterocyclic ring. As a specific example, R⁴ may be such a group that is a fluoro group, a trifluoromethyl group, a methoxy group, a methyl group, a trifluoromethoxy group, a trifluoromethylthio group, a methylthio group, a methyl sulfonate group, or a trimethylsilyl group, or that binds to R⁷ to form furan or dibenzofuran.

According to an embodiment of the present invention, R¹ and R⁴ may be the same or different from each other.

According to an embodiment of the present invention, R² and R³ may be each independently an alkyl group having 5 to 12 carbon atoms, a cycloalkyl group having 5 to 12 carbon atoms, an alkoxyalkyl group having 6 to 12 carbon atoms, an arylalkoxyalkyl group having 10 to 20 carbon atoms, or a trialkylsilyl group, where alkyl groups of the trialkylsilyl group may be each independently may be an alkyl group having 1 to 5 carbon atoms). As a specific example, R² and R³ may be each independently an alkyl group having 8 to 12 carbon atoms, a tripropylsilyl group, or a tributylsilyl group, and as a more specific example, R² and R³ may be each independently a tripropylsilyl group, a tributylsilyl group, or an n-decyl group. That is, the ligand compound may be such that the phenyl located at a terminal of the diphosphino aminyl moiety has substituents of R¹ and R⁴ at two ortho positions, and at the same time, has, at the meta or para position as a substituent, an alkyl group having 10 carbon atoms or a silyl group substituted with an alkyl group having 3 or 4 carbon atoms.

According to an embodiment of the present invention, R⁵ may be such a group that is an alkyl group having 1 to 10 carbon atoms, an alkyl group having 1 to 10 carbon atoms, which is substituted with an aryl group having 6 to 10 carbon atoms, a cycloalkyl group having 5 to 10 carbon atoms, or a cycloalkyl group having 5 to 10 carbon atoms, which is fused with an aryl group having 6 to 10 carbon atoms. As a specific example, R⁵ may be such a group that is an alkyl group having 3 to 5 carbon atoms, an alkyl group having 1 to 5 carbon atoms, which is substituted with an aryl group having 6 to 10 carbon atoms, a cycloalkyl group having 5 to 8 carbon atoms, or a cycloalkyl group having 5 to 8 carbon atoms, which is fused with an aryl group having 6 to 10 carbon atoms.

According to an embodiment of the present invention, the ligand compound represented by Chemical Formula 1 may be such a compound that is represented by any one of Chemical Formula 2 to Chemical Formula 5.

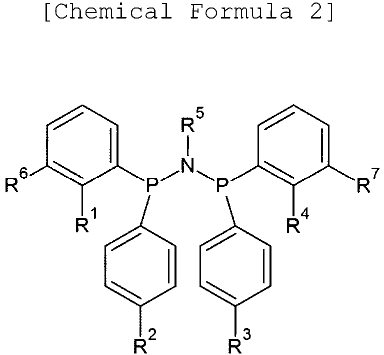

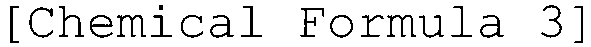

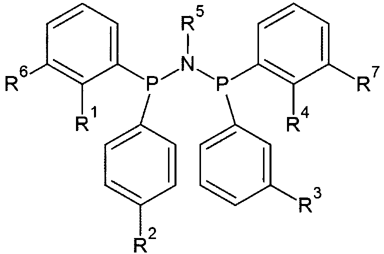

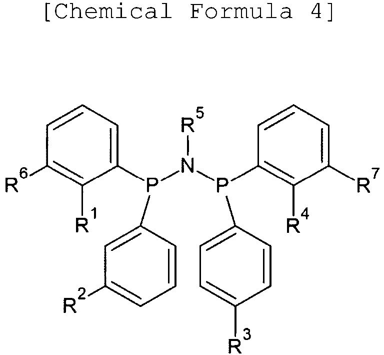

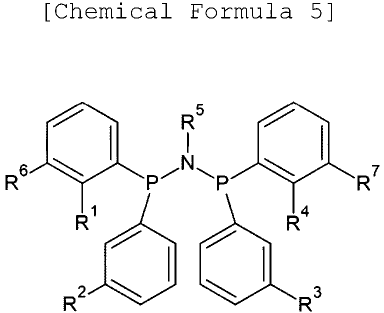

In Chemical Formula 2 to Chemical Formula 5, R¹ to R⁷ are respectively the same as those defined in Chemical Formula 1.

According to an embodiment of the present invention, the ligand compound represented by Chemical Formula 1 may be represented by any one of Chemical Formula 2-1 to Chemical Formula 2-76 or Chemical Formula 5-1 to Chemical Formula 5-20.

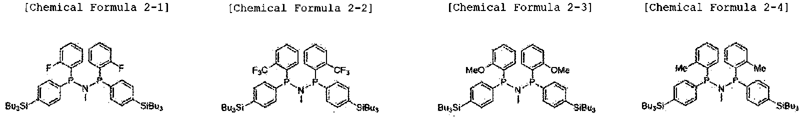

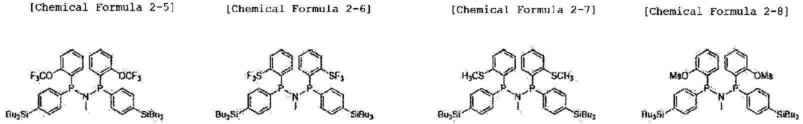

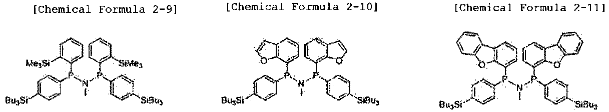

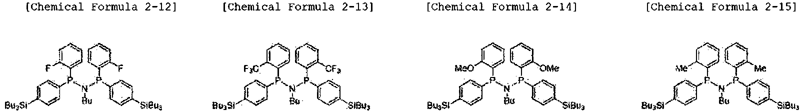

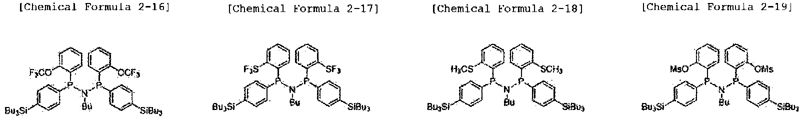

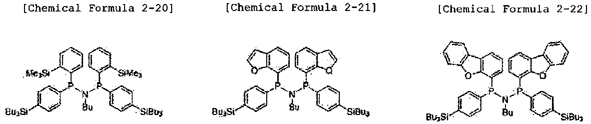

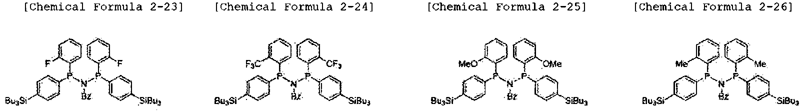

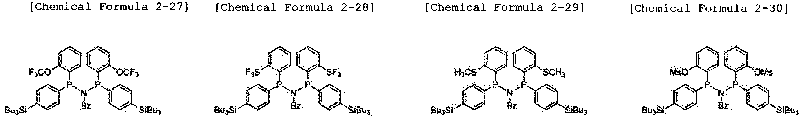

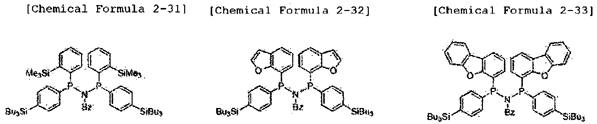

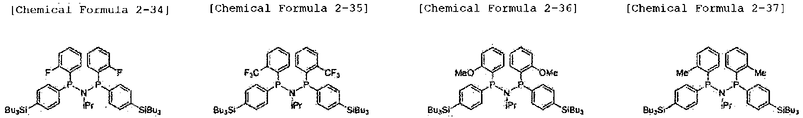

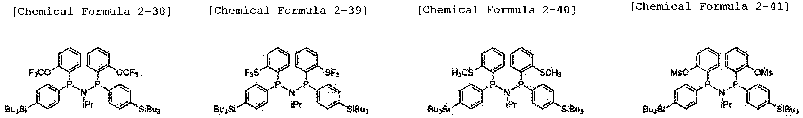

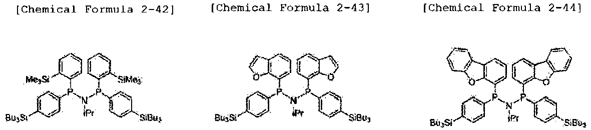

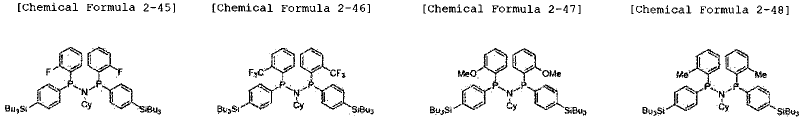

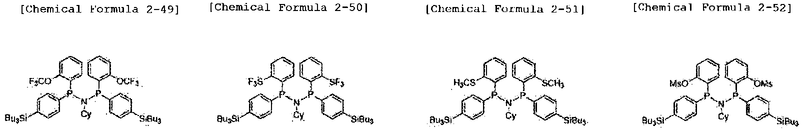

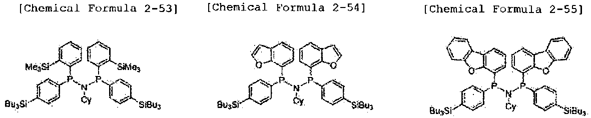

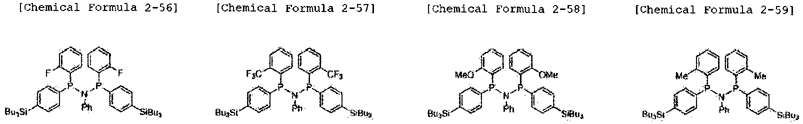

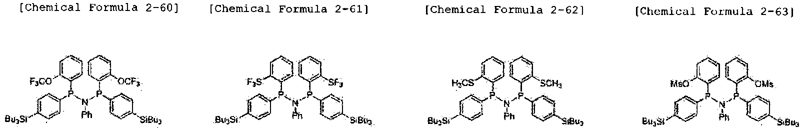

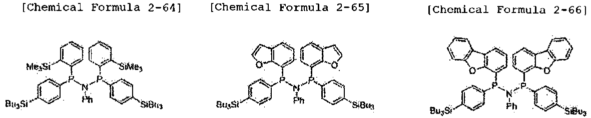

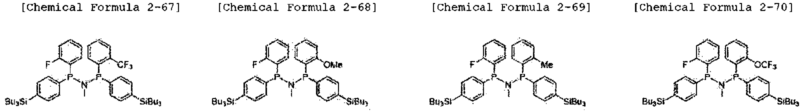

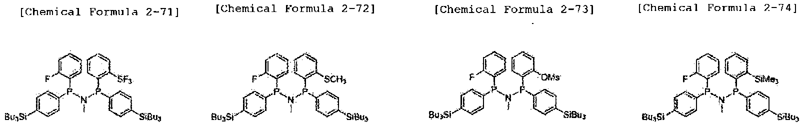

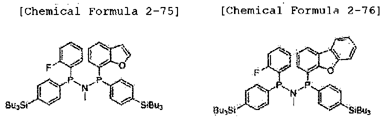

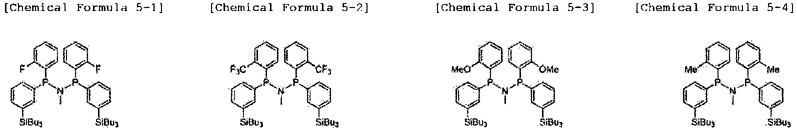

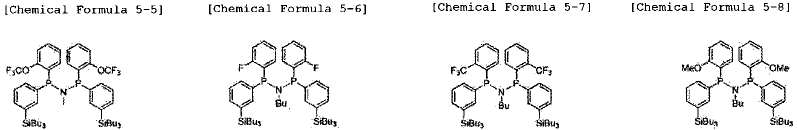

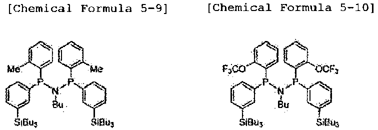

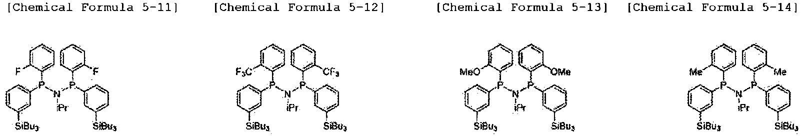

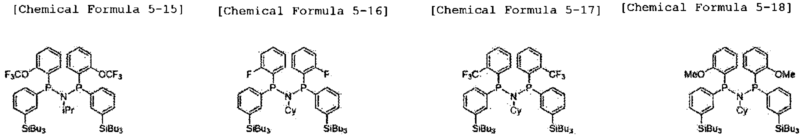

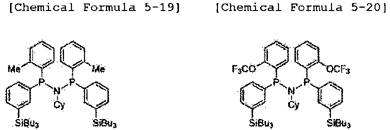

According to an embodiment of the present invention, in addition to the above-described specific examples, the ligand compound may be embodied in various combinations within a range that satisfies the conditions described above, and any compound represented by Chemical Formula 1 can be applied as the ligand compound according to the present invention.

### Organic chromium compound and catalyst composition

The present invention provides an organic chromium compound containing the ligand compound represented by Chemical Formula 1, and chromium (CR) coordinated to the ligand compound.

According to an embodiment of the present invention, the organic chromium compound is a chromium complex compound of the ligand compound and may have a form in which chromium of a chromium source forms a coordinate bond with an unshared electron pair of any one or more of N and two P's in the ligand compound represented by Chemical Formula 1. That is, such a structure in which the phosphorus atom or nitrogen atom of the diphosphino aminyl moiety provides an unshared electron pair to the chromium atom, in particular, a bidentated state in which two pairs of unshared electron pairs are coordinated may be desirable. Such an organic chromium compound can be applied to a catalyst system for the oligomerization reaction of ethylene to exhibit excellent catalytic activity and high selectivity for 1-hexene or 1-octene.

In the present invention, the "catalyst composition" means such a catalyst composition that is in a state of being capable of being obtained as an active catalyst composition by adding, simultaneously or in any order, three components including a chromium source, a ligand compound, and a co-catalyst, or two components of a transition metal compound and a co-catalyst. Here, the catalyst composition may also be referred to as a catalyst system, and in the present invention, the catalyst composition and catalyst system have the same meaning. The three components or two components of the catalyst composition may be added in the presence or absence of a solvent and a monomer and may be used in a supported or non-supported state.

The present invention provides a catalyst composition containing the ligand compound, chromium, and a co-catalyst. As described above, the ligand compound represented by Chemical Formula 1 and chromium may be coordinated to form an organic chromium compound. That is, the catalyst system may be a three components-based catalyst system containing chromium, the ligand compound represented by Chemical Formula 1, and a co-catalyst, or may be a two components-based catalyst system containing the organic chromium compound and a co-catalyst. As a specific example, the catalyst composition may be such a composition that contains the ligand compound, an organic chromium compound containing chromium coordinated to the ligand compound, and a co-catalyst. In addition, the catalyst composition may be such (one) that contains a chromium compound in which a part of the components of the co-catalyst are bonded to the organic chromium compound.

According to an embodiment of the present invention, the chromium may be derived from a chromium source, and the chromium source may be an organic or inorganic chromium compound in which the oxidation state of chromium is 0 to 6. As a specific example, the chromium source may be a chromium metal or a compound in which any organic or inorganic radical is bonded to chromium. Here, the organic radical may be, for example, an alkyl, alkoxy, ester, ketone, amide, or carboxylate radical, which has 1 to 20 carbon atoms per radical, and the inorganic radical may be a halide, a sulfate, an oxide, or the like.

According to an embodiment of the present invention, the chromium source is a compound that exhibits high activity in olefin oligomerization, is easy to be used, and is easily available, and it may be one or more compounds selected from the group consisting of chromium (III) acetylacetonate, chromium (III) chloride tetrahydrofuran, chromium (III) 2-ethylhexanoate, chromium (III) acetate, chromium (III) butyrate, chromium (III) pentanoate, chromium (III) laurate, chromium (III) tris(2,2,6,6-tetramethyl-3.5-heptanedionate), and chromium (III) stearate.

According to an embodiment of the present invention, the co-catalyst may be at least one or more selected from the group consisting of compounds represented by Chemical Formula 6 to Chemical Formula 9.

[Chemical Formula 6] -[Al(R¹³)-O]ₐ-

In Chemical Formula 6, R¹³'s are each independently a halogen group, a hydrocarbyl group having 1 to 20 carbon atoms, or a hydrocarbyl group having 1 to 20 carbon atoms, which is substituted with a halogen group, and a is an integer of 2 or more.

[Chemical Formula 7] E(R¹⁴)₃

In Chemical Formula 7, E is aluminum or boron, and R¹⁴'s are each independently hydrogen, a halogen group, a hydrocarbyl group having 1 to 20 carbon atoms, or a hydrocarbyl group having 1 to 20 carbon atoms, which is substituted with a halogen group.

[Chemical Formula 8] [L-H]⁺[G(Y)₄]⁻

[Chemical Formula 9] [L]⁺[G(Y)₄]⁻

In Chemical Formulae 8 and 9, L is a neutral or cationic Lewis acid, [LH]⁺ is Brønsted acid, G is a Group 13 element, and Y's are each independently a substituted or unsubstituted alkyl group having 1 to 20 carbon atoms or a substituted or unsubstituted aryl group having 6 to 20 carbon atoms, where in a case where the alkyl group or the aryl group is substituted, a substituent is a halogen group a hydrocarbyl group having 1 to 20 carbon atoms, an alkoxy group having 1 to 20 carbon atoms, or an aryloxy group having 6 to 20 carbon atoms.

According to an embodiment of the present invention, the catalyst composition can be produced by any of a plurality of methods. As a specific example, firstly, the catalyst composition can be produced by including a step of bringing the organic chromium compound into contact with the compound represented by Chemical Formula 6 or Chemical Formula 7. Secondly, the catalyst composition can be produced by including a step of bringing the organic chromium compound into contact with the compound represented by Chemical Formula 6 or Chemical Formula 7 to obtain a mixture; and a step of adding the compound represented by Chemical Formula 8 or Chemical Formula 9 to the mixture. Thirdly, the catalyst composition can be produced by including a step of bringing the organic chromium compound into contact with the compound represented by Chemical Formula 8 or Chemical Formula 9. Fourthly, the catalyst composition can be produced by including a step of bringing the organic chromium compound into contact with the compound represented by Chemical Formula 8 or Chemical Formula 9 to obtain a mixture; and a step of adding the compound represented by Chemical Formula 6 or Chemical Formula 7 to the mixture. Fifthly, the catalyst composition can be produced by including a step of bringing the chromium source into contact with the compound represented by Chemical Formula 8 or Chemical Formula 9 to obtain a mixture; and a step of bringing the ligand compound into contact with the reactant.

According to an embodiment of the present invention, in the case of the first or third method among the catalyst composition producing methods, the molar ratio of the compound represented by Chemical Formula 6 or Chemical Formula 7 to the organic chromium compound may be 1:2 to 1:5,000, may be 1:100 to 1:3,000 as a specific example, and may be 1:300 to 1:1,500 as a more specific example. Within this range, it is possible to allow the alkylation of the organic chromium compound to proceed completely, thereby improving the activity of the catalyst composition, and it is possible to prevent the deterioration of the activation of the alkylated organic chromium compound due to side reactions between the remaining alkylating agents, and at the same time improve the economic efficiency and the purity of the produced linear alpha-olefin.

According to an embodiment of the present invention, in the case of the second method among the catalyst composition producing methods, the molar ratio of the compound represented by Chemical Formula 8 or Chemical Formula 9 to the organic chromium compound may be 1:1 to 1:500, may be 1:1 to 1:50 as a specific example, and may be 1:1 to 1:25 as a more specific example. Within this range, the amount of activator is sufficient, and thus the metal compound is completely activated, whereby the activity of the catalyst composition is improved. In addition, the amount of the remaining activator is minimized, which makes it possible to improve the economic efficiency and the purity of the produced linear alpha-olefin.

According to an embodiment of the present invention, the compound represented by Chemical Formula 6 may be an alkyl aluminoxane, may be methyl aluminoxane, ethyl aluminoxane, isobutyl aluminoxane, or butyl aluminoxane as a specific example, and may be methyl aluminoxane as a more specific example.

According to an embodiment of the present invention, the compound represented by Chemical Formula 7 may be a trialkyl aluminum, a dialkyl aluminum halide, an alkyl aluminum dihalide, a dialkyl aluminum hydride, an alkyl aluminum dihydride, a trialkyl boron, or the like. As a specific example, the compound represented by Chemical Formula 6 may be a trialkyl aluminum such as trimethyl aluminum, triethyl aluminum, triisobutyl aluminum, tripropyl aluminum, tributyl aluminum, triisopropyl aluminum, tri-s-butyl aluminum, tricyclopentyl aluminum, tripentyl aluminum, triisopentyl aluminum, trihexyl aluminum, trioctyl aluminum, ethyldimethyl aluminum, methyldiethyl aluminum, triphenyl aluminum, or trip-tolyl aluminum; a dialkyl aluminum halide such as diethyl aluminum chloride; a dialkyl aluminum hydride such as diethyl aluminum hydride, di-n-propyl aluminum hydride, diisopropyl aluminum hydride, di-n-butyl aluminum hydride, dibutyl aluminum hydride, diisobutyl aluminum hydride (DIBAH), di-n-octyl aluminum hydride, diphenyl aluminum hydride, di-p-tolyl aluminum hydride, dibenzyl aluminum hydride, phenylethyl aluminum hydride, phenyl-n-propyl aluminum hydride, phenyl isopropyl aluminum hydride, phenyl -n-butyl aluminum hydride, phenyl isobutyl aluminum hydride, phenyl-n-octyl aluminum hydride, p-tolylethyl aluminum hydride, p-tolyl-n-propyl aluminum hydride, p-tolyl isopropyl aluminum hydride, p-tolyl-n-butyl aluminum hydride, p-tolylisobutyl aluminum hydride, p-tolyl-n-octyl aluminum hydride, benzylethyl aluminum hydride, benzyl-n-propyl aluminum hydride, benzylisopropyl aluminum hydride, benzyl-n-butyl aluminum hydride, benzylisobutyl aluminum hydride, or benzyl-n-octyl aluminum hydride; an alkyl aluminum dihydride such as n-propyl aluminum dihydride, isopropyl aluminum dihydride, n-butyl aluminum dihydride, isobutyl aluminum dihydride, or n-octyl aluminum dihydride; or a trialkyl boron such as trimethyl boron, triethyl boron, triisobutyl boron, tripropyl boron, or tributyl boron.

According to an embodiment of the present invention, the compound represented by Chemical Formula 8 or Chemical Formula 9 may be trimethylammonium tetraphenylborate, triethylammonium tetraphenylborate, tripropylammonium tetraphenylborate, tributylammonium tetraphenylborate, methyl dioctadecylammonium tetraphenylborate, N,N-dimethylanilinium tetraphenylborate, N,N-diethylanilinium tetraphenylborate, N,N-dioctadecylanilinium tetraphenylborate, trimethylammonium tetra (p-tolyl)borate, triethylammonium tetra (p-tolyl)borate, tripropylammonium tetra(p-tolyl)borate, tributylammonium tetra(p-tolyl)borate, methyl dioctadecylammonium tetra(p-tolyl)borate, N,N-dimethylanilinium tetra(p-tolyl)borate, N,N-diethylanilinium tetra(p-tolyl)borate, N,N-dioctadecylanilinium tetra(p-tolyl)borate, trimethylammonium tetra(o,p-dimethylphenyl)borate, triethylammonium tetra(o,p-dimethylphenyl) borate, tripropylammonium tetra(o,p-dimethylphenyl) borate, tributylammonium tetra(o,p-dimethylphenyl)borate, methyl dioctadecylammonium tetra(o,p-dimethylphenyl) borate, N,N-dimethylanilinium tetra(o,p-dimethylphenyl) borate, N,N-diethylanilinium tetra(o,p-dimethylphenyl) borate, N,N-dioctadecylanilinium tetra(o,p-dimethylphenyl) borate, trimethylammonium tetrakis(p-trifluoromethylphenyl) borate, triethylammonium tetrakis(p-trifluoromethylphenyl)borate, tripropylammonium tetrakis(p-trifluoromethylphenyl) borate, tributylammonium tetrakis( p-trifluoromethylphenyl)borate, methyl dioctadecylammonium tetrakis(p-trifluoromethylphenyl)borate, N,N-dimethylanilinium tetrakis(p-trifluoromethylphenyl)borate, N,N-diethylanilinium tetrakis(p-trifluoromethylphenyl)borate, N,N-dioctadecylanilinium tetrakis(p-trifluoromethylphenyl) borate, trimethylammonium tetrakis(pentafluorophenyl)borate, triethylammonium tetrakis(pentafluorophenyl)borate, tripropylammonium tetrakis(pentafluorophenyl)borate, tributylammonium tetrakis(pentafluorophenyl)borate, methyl dioctadecylammonium tetrakis(pentafluorophenyl)borate, N,N-dimethylanilinium tetrakis(pentafluorophenyl)borate, N,N-diethylanilinium tetrakis(pentafluorophenyl)borate, N,N-dioctadecylanilinium tetrakis(pentafluorophenyl)borate, trimethylphosphonium tetraphenylborate, triethylphosphonium tetraphenylborate, tripropylphosphonium tetraphenylborate, tributylphosphonium tetraphenylborate, trimethylcarbonium tetraphenylborate, triethylcarbonium tetraphenylborate, tripropylcarbonium tetraphenylborate, tributylcarbonium tetraphenylborate, trimethylammonium tetraphenylaluminate, triethylammonium tetraphenylaluminate, tripropylammonium tetraphenylaluminate, tributylammonium tetraphenylaluminate, trimethylammonium tetra(p-tolyl)aluminate, triethylammonium tetra(p-tolyl)aluminate, tripropylammonium tetra(p-tolyl)aluminate, tributylammonium tetra(p-tolyl)aluminate, or the like.

According to an embodiment of the present invention, the content ratio between the components that form the catalyst composition may be determined in consideration of the catalytic activity, the selectivity for the linear alpha-olefin, and the like. As a specific example, in a case where the catalyst composition is a three components-based catalyst composition, the molar ratio (diphosphino aminyl moiety:chromium source:co-catalyst) in the ligand compound can be adjusted to about 1:1:1 to about 10:1:10,000, or about 1:1:100 to 5:1:3,000. In addition, in a case where the catalyst composition is a two components-based catalyst composition, the molar ratio (diphosphino aminyl moiety:co-catalyst) in the organic chromium compound can be adjusted to about 1:1 to 1:10,000, 1:1 to 1:5,000, or 1:1 to 1:3,000.

According to an embodiment of the present invention, in the production of the catalyst composition, the reaction solvent to be used may be a hydrocarbon-based solvent such as pentane, hexane, or heptane; and an aromatic solvent such as benzene or toluene.

According to an embodiment of the present invention, the components that form the catalyst composition are added simultaneously or in any order in the presence or absence of an appropriate solvent and a monomer, whereby the catalyst composition can act as an active catalyst composition. In this case, the suitable solvent may be heptane, toluene, cyclohexane, methylcyclohexane, 1-hexene, 1-octene, diethyl ether, tetrahydrofuran, acetonitrile, dichloromethane, chloroform, chlorobenzene, methanol, acetone, or the like.

According to an embodiment of the present invention, the organic chromium compound and the co-catalyst can be used as a form in which the organic chromium compound and the co-catalyst are supported on a carrier, and in this case, the carrier may be silica or alumina.

According to an embodiment of the present invention, the catalyst composition may further contain a carrier. As a specific example, the ligand compound represented by Chemical Formula 1 can be applied to an ethylene oligomerization reaction as a form in which the ligand compound represented by Chemical Formula 1 is supported on a carrier. The carrier may be a metal, a metal salt, or a metal oxide, which is applied to a carried catalyst. As a specific example, the carrier may be silica, silica-alumina, or silica-magnesia, and it may include an oxide, a carbonate, sulfate, or nitrate component of a metal, such as Na₂O, K₂CO₃, BaSO₄, or Mg(NO₃)₂.

According to an embodiment of the present invention, the catalyst composition can be used for a trimerization or tetramerization reaction of ethylene, and 1-hexene or 1-octene can be produced with high selectivity as described above.

### Ethylene oligomerization method

The present invention provides a production method for linear alpha-olefin as an ethylene oligomerization method including a step (S10) of oligomerizing ethylene in the presence of the catalyst composition.

In the present invention, the "oligomerization" means that olefins are oligomerized. It is called trimerization or tetramerization according to the number of olefins to be polymerized, which is collectively called multimerization. In particular, in the present specification, it may mean selectively producing, from ethylene, 1-hexene and 1-octene, which are the main comonomers of LLDPE.

According to an embodiment of the present invention, the oligomerization reaction of ethylene may be a trimerization or tetramerization reaction of ethylene, and 1-hexene or 1-octene is formed as a result of the reaction, whereby the linear alpha-olefin may be 1-hexene, 1-octene, or a mixture thereof.

According to an embodiment of the present invention, the method for ethylene oligomerization may be carried out by using ethylene as a raw material and applying the previously described catalyst composition and conventional devices and contact technology. As a specific example, the oligomerization reaction of ethylene may be carried out as a homogeneous liquid phase reaction in the presence or absence of an inert solvent, alternatively a slurry reaction in which part or all of the catalyst composition is in an undissolved form, or a bulk phase reaction or gas phase reaction in which the alpha-olefin as a product serves as a main medium.

According to an embodiment of the present invention, the oligomerization reaction of ethylene may be carried out in an inert solvent. As a specific example, the inert solvent may be benzene, toluene, xylene, cumene, chlorobenzene, dichlorobenzene, heptane, cyclohexane, methylcyclohexane, methylcyclopentane, n-hexane, 1-hexene, 1-octene, or 2,2,4-trimethylpentane.

According to an embodiment of the present invention, the oligomerization reaction of ethylene may be carried out at a temperature of 0°C to 200°C, 0°C to 150°C, 30°C to 100°C, or 50°C to 100°C. In addition, the above reaction may be carried out at a pressure of 15 psig to 3,000 psig, 15 psig to 1,500 psig, or 15 psig to 1,000 psig.

Hereinafter, examples of the present invention will be described in detail so that a person skilled in the art can easily carry out the examples of the present invention. However, the present invention may be embodied in various forms different from each other and thus is not limited to the examples described herein.

### Synthesis Examples and Comparative Synthesis Examples

### Synthesis Example 1: Synthesis of ligand compound represented by Chemical Formula 2-1

0.34 g of a methylamine HCl salt, 1.77 g of triethylamine, and 10 ml of dichloromethane were charged into a well-dried flask in a nitrogen atmosphere, and then stirring was started with cooling to 0°C. 4.37 g of chloro(2-fluorophenyl) (4-(tributylsilyl)phenyl)phosphane) was diluted in 10 ml of dichloromethane and then slowly charged into the flask. After stirring the reactant for 22 hours, the conversion rate was checked through NMR. After the reaction was completed, the precipitate was removed by filtration, and the solvent was removed by reducing the pressure. The obtained organic material was purified using column chromatography to obtain a target product, 2.75 g (yield: 66%) of 1-(2-fluorophenyl)-N-((2-fluorophenyl)(4-(tributylsilyl)phenyl)phosphaneyl)-N-methyl-1-(4-(tributylsilyl)phenyl)phosphanamine.

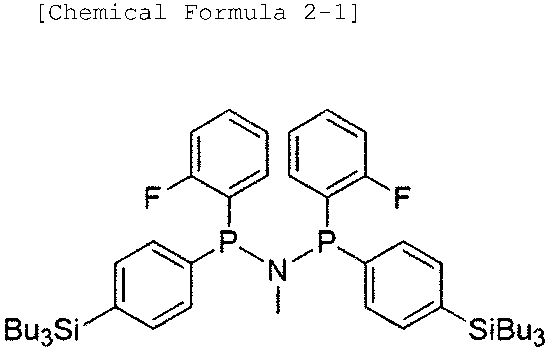

¹H NMR (500 MHz, CDCl₃): δ 7.71 (m, 2H), 7.48(m, 4H), 7.28(m, 2H), 7.27 (m, 2H), 7.20(m, 6H), 2.51(m, 3H), 1.50 (m, 12H), 1.31 (m, 12H), 1.27 (m, 12H), 0.93 (m, 18H)

### Synthesis Example 2: Synthesis of ligand compound represented by Chemical Formula 2-2

0.34 g of a methylamine HCl salt, 1.77 g of triethylamine, and 10 ml of dichloromethane were charged into a well-dried flask in a nitrogen atmosphere, and then stirring was started with cooling to 0°C. 4.87 g of chloro(4-(tributylsilyl)phenyl)(2-(trifluoromethyl)phenyl)phosphane was diluted in 10 ml of dichloromethane and then slowly charged into the flask. After stirring the reactant for 21 hour, the conversion rate was checked through NMR. After the reaction was completed, the precipitate was removed by filtration, and the solvent was removed by reducing the pressure. The obtained organic material was purified using column chromatography to obtain a target product, 3.64 g (yield: 78%) of N-methyl-1-(4-(tributylsilyl)phenyl)-N-((4-(tributylsilyl)phenyl)(2-(trifluoromethyl)phenyl)phosphaneyl)-1-(2-(trifluoromethyl)phenyl)phosphanamine.

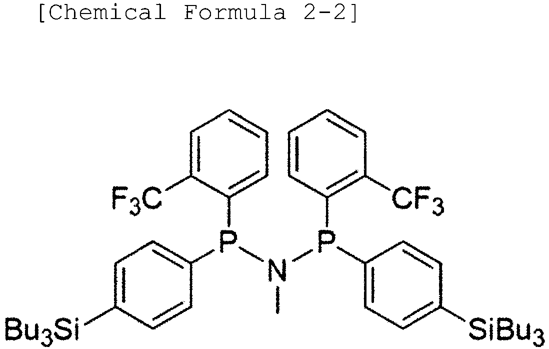

¹H NMR (500 MHz, CDCl₃) : δ 7.52(m, 2H), 7.46 (m, 2H), 7.42 (m, 4H), 7.29(m, 2H), 7.17(m, 4H), 7.10(m, 2H), 2.47 (m, 3H), 1.44(m, 12H), 1.40(m, 12H), 1.23(m, 12H), 0.91(m, 18H)

### Synthesis Example 3: Synthesis of ligand compound represented by Chemical Formula 2-3

0.34 g of a methylamine HCl salt, 1.77 g of triethylamine, and 10 ml of dichloromethane were charged into a well-dried flask in a nitrogen atmosphere, and then stirring was started with cooling to 0°C. 4.49 g of chloro(2-methoxyphenyl)(4-(tributylsilyl)phenyl)phosphane was diluted in 10 ml of dichloromethane and then slowly charged into the flask. After stirring the reactant for 17 hours, the conversion rate was checked through NMR. After the reaction was completed, the precipitate was removed by filtration, and the solvent was removed by reducing the pressure. The obtained organic material was purified using column chromatography to obtain a target product, 4.07 g (yield: 95%) of 1-(2-methoxyphenyl)-N-((2-methoxyphenyl)(4-(tributylsilyl)phenyl)phosphaneyl)-N-methyl-1-(4-(tributylsilyl)phenyl)phosphanamine.

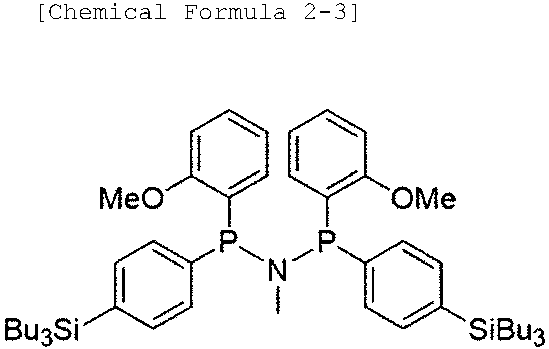

¹H NMR (500 MHz, CDCl₃) : δ 7.43(m, 2H), 7.40 (m, 4H), 7.19(m, 2H), 7.15(m, 2H), 7.14 (m, 4H), 7.01(m, 2H), 3.86(m, 6H), 2.48(m, 3H), 1.51(m, 12H), 1.36(m, 12H), 1.31(m, 12H), 0.91(m, 18H)

### Synthesis Example 4: Synthesis of ligand compound represented by Chemical Formula 2-4

0.34 g of a methylamine HCl salt, 1.77 g of triethylamine, and 10 ml of dichloromethane were charged into a well-dried flask in a nitrogen atmosphere, and then stirring was started with cooling to 0°C. 4.33 g of chloro(o-tolyl) (4-(tributylsilyl)phenyl)phosphane was diluted in 10 ml of dichloromethane and then slowly charged into the flask. After stirring the reactant for 20 hours, the conversion rate was checked through NMR. After the reaction was completed, the precipitate was removed by filtration, and the solvent was removed by reducing the pressure. The obtained organic material was purified using column chromatography to obtain a target product, 2.47 g (yield: 60%) of N-methyl-1-(o-tolyl)-N-(o-tolyl(4-(tributylsilyl)phenyl)phosphaneyl)-1-(4-(tributylsilyl)phenyl)phosphanamine.

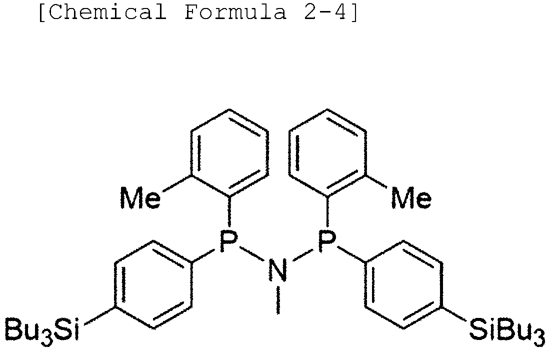

¹H NMR (500 MHz, CDCl₃): δ 7.41 (m, 4H), 7.35 (m, 2H), 7.30 (m, 2H), 7.22 (m, 2H), 7.16(m, 4H), 7.15 (m, 2H), 2.57 (m, 3H), 2.42(m, 6H), 1.51(m, 12H), 1.35(m, 12H), 1.25(m, 12H), 0.96(m, 18H)

### Synthesis Example 5: Synthesis of ligand compound represented by Chemical Formula 2-5

0.34 g of a methylamine HCl salt, 1.77 g of triethylamine, and 10 ml of dichloromethane were charged into a well-dried flask in a nitrogen atmosphere, and then stirring was started with cooling to 0°C. 5.03 g of chloro(4-(tributylsilyl)phenyl) (2-(trifluoromethoxy)phenyl)phosphane was diluted in 10 ml of dichloromethane and then slowly charged into the flask. After stirring the reactant for 16 hours, the conversion rate was checked through NMR. After the reaction was completed, the precipitate was removed by filtration, and the solvent was removed by reducing the pressure. The obtained organic material was purified using column chromatography to obtain a target product, 4.39 g (yield: 91%) of N-methyl-1-(4-(tributylsilyl)phenyl)-N-((4-(tributylsilyl)phenyl)(2-(trifluoromethoxy)phenyl)phosphaneyl)-1-(2-(trifluoromethoxy)phenyl)phosphanamine.

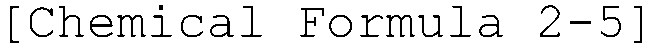

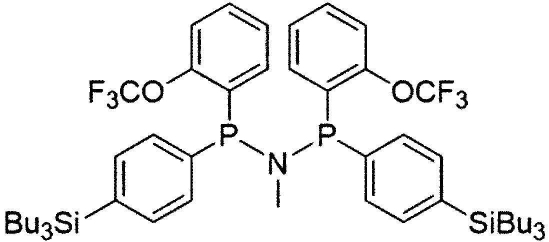

¹H NMR (500 MHz, CDCl₃) : δ 7.50(m, 4H), 7.49(m, 2H), 7.20(m, 2H), 7.16(m, 4H), 7.14(m, 2H), 7.03(m, 2H), 2.53(m, 3H), 1.49(m, 12H), 1.32(m, 12H), 1.30(m, 12H), 0.94 (m, 18H)

### Synthesis Example 6: Synthesis of ligand compound represented by Chemical Formula 2-6

0.34 g of a methylamine HCl salt, 1.77 g of triethylamine, and 10 ml of dichloromethane were charged into a well-dried flask in a nitrogen atmosphere, and then stirring was started with cooling to 0°C. 5.07 g of chloro(4-(tributylsilyl)phenyl)(2-(trifluoro-14-sulfaneyl)phenyl)phosphane was diluted in 10 ml of dichloromethane and then slowly charged into the flask. After stirring the reactant for 18 hours, the conversion rate was checked through NMR. After the reaction was completed, the precipitate was removed by filtration, and the solvent was removed by reducing the pressure. The obtained organic material was purified using column chromatography to obtain a target product, 3.74 g (yield: 77%) of N-methyl-1-(4-(tributylsilyl)phenyl)-N-((4-(tributylsilyl)phenyl)(2-(trifluoro-14-sulfaneyl)phenyl)phosphaneyl)-1-(2-(trifluoro-14-sulfaneyl)phenyl)phosphanamine.

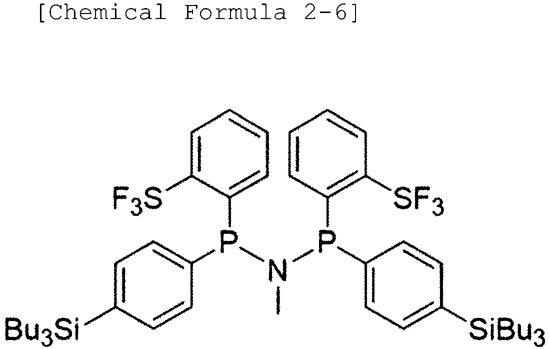

¹H NMR (500 MHz, CDCl₃) : δ 7.41 (m, 4H), 7.40(m, 2H), 7.36(m, 2H), 7.23(m, 2H), 7.14(m, 4H), 7.03(m, 2H), 2.56(m, 3H), 1.45(m, 12H), 1.38 (m, 12H), 1.30 (m, 12H), 0.93 (m, 18H)

### Synthesis Example 7: Synthesis of ligand compound represented by Chemical Formula 2-7

0.34 g of a methylamine HCl salt, 1.77 g of triethylamine, and 10 ml of dichloromethane were charged into a well-dried flask in a nitrogen atmosphere, and then stirring was started with cooling to 0°C. 4.65 g of chloro(2-(methylthio)phenyl)(4-(tributylsilyl)phenyl)phosphanewas diluted in 10 ml of dichloromethane and then slowly charged into the flask. After stirring the reactant for 13 hours, the conversion rate was checked through NMR. After the reaction was completed, the precipitate was removed by filtration, and the solvent was removed by reducing the pressure. The obtained organic material was purified using column chromatography to obtain a target product, 3.73 g (yield: 84%) of N-methyl-1-(2-(methylthio)phenyl)-N-((2-(methylthio) phenyl) (4-(tributylsilyl)phenyl)phosphaneyl)-1-(4-(tributylsilyl)phenyl)phosphanamine.

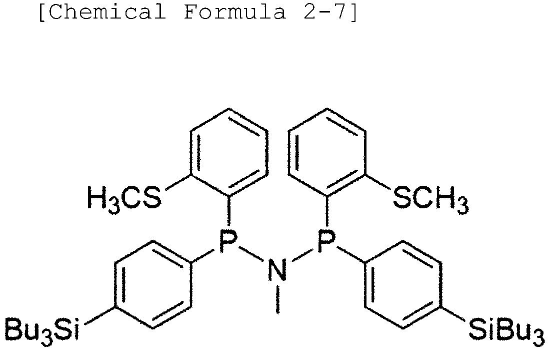

¹H NMR (500 MHz, CDCl₃) : δ 7.45(m, 4H), 7.40 (m, 2H), 7.35 (m, 2H), 7.21(m, 2H), 7.16(m, 4H), 7.08 (m, 2H), 2.56 (m, 3H), 2.52 (m, 6H), 1.46(m, 12H), 1.34 (m, 12H), 1.29 (m, 12H), 0.97 (m, 18H)

### Synthesis Example 8: Synthesis of ligand compound represented by Chemical Formula 2-8

0.34 g of a methylamine HCl salt, 1.77 g of triethylamine, and 10 ml of dichloromethane were charged into a well-dried flask in a nitrogen atmosphere, and then stirring was started with cooling to 0°C. 5.13 g of 2-(chloro(4-(tributylsilyl)phenyl)phosphaneyl)phenyl methanesulfonate was diluted in 10 ml of dichloromethane and then slowly charged into the flask. After stirring the reactant for 15 hours, the conversion rate was checked through NMR. After the reaction was completed, the precipitate was removed by filtration, and the solvent was removed by reducing the pressure. The obtained organic material was purified using column chromatography to obtain a target product, 3.94 g (yield: 80%) of ((methylazanediyl)bis((4-(tributylsilyl)phenyl)phosphanediyl))bis(2,1-phenylene) dimethanesulfonate.

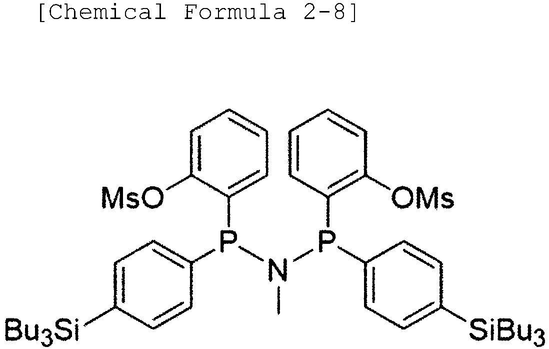

¹H NMR (500 MHz, CDCl₃) : δ 7.55(m, 2H), 7.50 (m, 2H), 7.46 (m, 4H), 7.32(m, 2H), 7.30(m, 2H), 7.17(m, 4H), 3.54(m, 6H), 2.47(m, 3H), 1.50(m, 12H), 1.37(m, 12H), 1.29(m, 12H), 0.89(m, 18H)

### Synthesis Example 9: Synthesis of ligand compound represented by Chemical Formula 2-9

0.34 g of a methylamine HCl salt, 1.77 g of triethylamine, and 10 ml of dichloromethane were charged into a well-dried flask in a nitrogen atmosphere, and then stirring was started with cooling to 0°C. 4.91 g of chloro(4-(tributylsilyl)phenyl)(2-(trimethylsilyl)phenyl)phosphane was diluted in 10 ml of dichloromethane and then slowly charged into the flask. After stirring the reactant for 11 hour, the conversion rate was checked through NMR. After the reaction was completed, the precipitate was removed by filtration, and the solvent was removed by reducing the pressure. The obtained organic material was purified using column chromatography to obtain a target product, 3.67 g (yield: 78%) of N-methyl-1-(4-(tributylsilyl)phenyl)-N-((4-(tributylsilyl)phenyl)(2-(trimethylsilyl)phenyl)phosphaneyl)-1-(2-(trimethylsilyl)phenyl)phosphanamine.

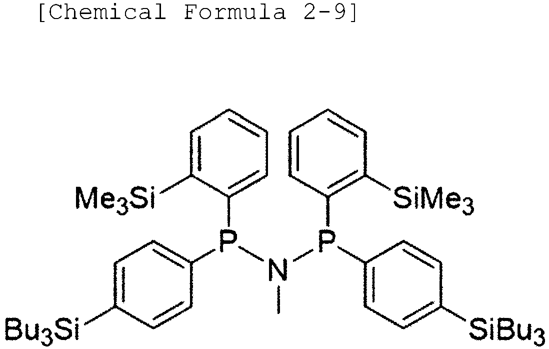

¹H NMR (500 MHz, CDCl₃) : δ 7.42(m, 8H), 7.32(m, 2H), 7.13(m, 6H), 2.49(m, 3H), 1.44(m, 12H), 1.39(m, 12H), 1.26(m, 12H), 0.90(m, 18H), 0.27(m, 18H)

### Synthesis Example 10: Synthesis of ligand compound represented by Chemical Formula 2-10

0.34 g of a methylamine HCl salt, 1.77 g of triethylamine, and 10 ml of dichloromethane were charged into a well-dried flask in a nitrogen atmosphere, and then stirring was started with cooling to 0°C. 4.59 g of benzofuran-7-ylchloro(4-(tributylsilyl)phenyl)phosphane was diluted in 10 ml of dichloromethane and then slowly charged into the flask. After stirring the reactant for 11 hour, the conversion rate was checked through NMR. After the reaction was completed, the precipitate was removed by filtration, and the solvent was removed by reducing the pressure. The obtained organic material was purified using column chromatography to obtain a target product, 3.29 g (yield: 75%) of 1-(benzofuran-7-yl)-N-(benzofuran-7-yl(4-(tributylsilyl)phenyl)phosphaneyl)-N-methyl-1-(4-(tributylsilyl)phenyl)phosphanamine.

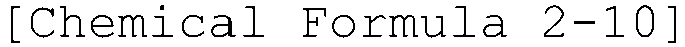

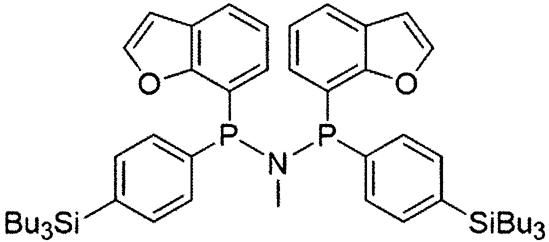

¹H NMR (500 MHz, CDCl₃): δ 7.69 (m, 2H), 7. 64 (m, 2H), 7.48(m, 6H), 7.41(m, 2H), 7.17(m, 4H), 6.78 (m, 2H), 2.55(m, 3H), 1.51(m, 12H), 1.38 (m, 12H), 1.29(m, 12H), 0.91(m, 18H)

### Synthesis Example 11: Synthesis of ligand compound represented by Chemical Formula 2-11

0.34 g of a methylamine HCl salt, 1.77 g of triethylamine, and 10 ml of dichloromethane were charged into a well-dried flask in a nitrogen atmosphere, and then stirring was started with cooling to 0°C. 5.09 g of chloro(dibenzo[b,d]furan-4-yl) (4-(tributylsilyl)phenyl)phosphane was diluted in 10 ml of dichloromethane and then slowly charged into the flask. After stirring the reactant for 18 hours, the conversion rate was checked through NMR. After the reaction was completed, the precipitate was removed by filtration, and the solvent was removed by reducing the pressure. The obtained organic material was purified using column chromatography to obtain a target product, 4.25 g (yield: 87%) of 1-(dibenzo[b,d]furan-4-yl)-N-(dibenzo[b,d]furan-4-yl(4-(tributylsilyl)phenyl)phosphaneyl)-N-methyl-1-(4-(tributylsilyl)phenyl)phosphanamine.

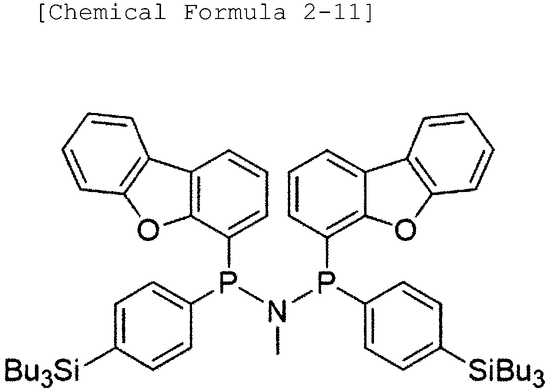

¹H NMR (500 MHz, CDCl₃) : δ 8.02(m, 4H), 7.54 (m, 2H), 7.50 (m, 2H), 7.47(m, 4H), 7.43(m, 2H), 7.41(m, 4H), 7.16(m, 4H), 2.50 (m, 3H), 1.47(m, 12H), 1.37 (m, 12H), 1.31(m, 12H), 0.93 (m, 18H)

### Synthesis Example 12: Synthesis of ligand compound represented by Chemical Formula 2-12

0.37 g of butylamine, 1.26 g of triethylamine, and 10 ml of dichloromethane were charged into a well-dried flask in a nitrogen atmosphere, and then stirring was started with cooling to 0°C. 4.37 g of chloro(2-fluorophenyl) (4-(tributylsilyl)phenyl)phosphane was diluted in 10 ml of dichloromethane and then slowly charged into the flask. After stirring the reactant for 21 hour, the conversion rate was checked through NMR. After the reaction was completed, the precipitate was removed by filtration, and the solvent was removed by reducing the pressure. The obtained organic material was purified using column chromatography to obtain a target product, 3.67 g (yield: 84%) of N-butyl-1-(2-fluorophenyl)-N-((2-fluorophenyl)(4-(tributylsilyl)phenyl)phosphaneyl)-1-(4-(tributylsilyl)phenyl)phosphanamine.

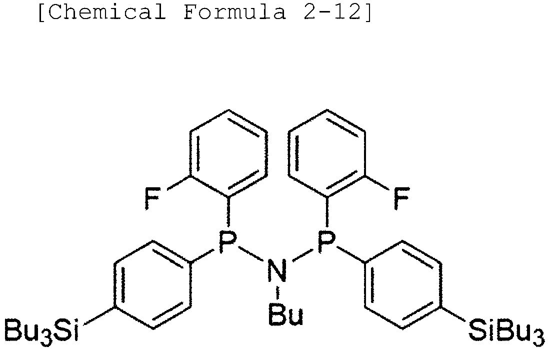

¹H NMR (500 MHz, CDCl₃) : δ 7.68(m, 2H), 7.44 (m, 4H), 7.25(m, 2H), 7.21(m, 2H), 7.20(m, 6H), 2.64(m, 2H), 1.54(m, 2H), 1.50(m, 12H), 1.32(m, 14H), 1.29(m, 12H), 0.98(m, 21H)

### Synthesis Example 13: Synthesis of ligand compound represented by Chemical Formula 2-13

0.37 g of butylamine, 1.26 g of triethylamine, and 10 ml of dichloromethane were charged into a well-dried flask in a nitrogen atmosphere, and then stirring was started with cooling to 0°C. 4.87 g of chloro(4-(tributylsilyl)phenyl) (2-(trifluoromethyl)phenyl)phosphane was diluted in 10 ml of dichloromethane and then slowly charged into the flask. After stirring the reactant for 11 hour, the conversion rate was checked through NMR. After the reaction was completed, the precipitate was removed by filtration, and the solvent was removed by reducing the pressure. The obtained organic material was purified using column chromatography to obtain a target product, 3.26 g (yield: 67%) of N-butyl-1-(4-(tributylsilyl)phenyl)-N-((4-(tributylsilyl)phenyl)(2-(trifluoromethyl)phenyl)phosphaneyl)-1-(2-(trifluoromethyl)phenyl)phosphanamine.

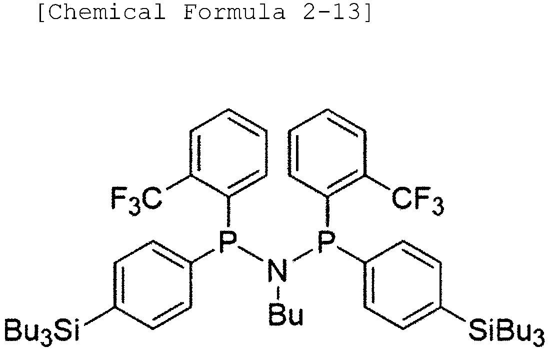

¹H NMR (500 MHz, CDCl₃) : δ 7.50(m, 4H), 7.49(m, 2H), 7.47(m, 2H), 7.28(m, 2H), 7.18(m, 4H), 7.15(m, 2H), 2.71(m, 2H), 1.61(m, 2H), 1.48(m, 12H), 1.36(m, 14H), 1.27(m, 12H), 0.98(m, 21H)

### Synthesis Example 14: Synthesis of ligand compound represented by Chemical Formula 2-14

0.37 g of butylamine, 1.26 g of triethylamine, and 10 ml of dichloromethane were charged into a well-dried flask in a nitrogen atmosphere, and then stirring was started with cooling to 0°C. 4.49 g of chloro(2-methoxyphenyl) (4-(tributylsilyl)phenyl)phosphane was diluted in 10 ml of dichloromethane and then slowly charged into the flask. After stirring the reactant for 24 hours, the conversion rate was checked through NMR. After the reaction was completed, the precipitate was removed by filtration, and the solvent was removed by reducing the pressure. The obtained organic material was purified using column chromatography to obtain a target product, 3.14 g (yield: 70%) of N-butyl-1-(2-methoxyphenyl)-N-((2-methoxyphenyl) (4-(tributylsilyl)phenyl)phosphaneyl)-1-(4-(tributylsilyl)phenyl)phosphanamine.

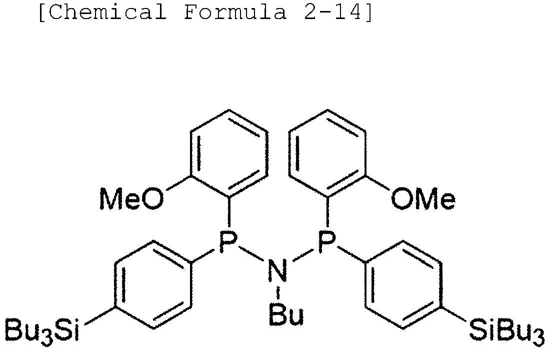

¹H NMR (500 MHz, CDCl₃) : δ 7.45(m, 2H), 7.40(m, 4H), 7.18(m, 6H), 7.14(m, 2H), 7.03(m, 2H), 3.89(m, 6H), 2.73 (m, 2H), 1.59(m, 2H), 1.47(m, 12H), 1.30(m, 14H), 1.28(m, 12H), 0.96(m, 21H)

### Synthesis Example 15: Synthesis of ligand compound represented by Chemical Formula 2-15

0.37 g of butylamine, 1.26 g of triethylamine, and 10 ml of dichloromethane were charged into a well-dried flask in a nitrogen atmosphere, and then stirring was started with cooling to 0°C. 4.33 g of chloro (o-tolyl) (4-(tributylsilyl)phenyl)phosphane was diluted in 10 ml of dichloromethane and then slowly charged into the flask. After stirring the reactant for 13 hours, the conversion rate was checked through NMR. After the reaction was completed, the precipitate was removed by filtration, and the solvent was removed by reducing the pressure. The obtained organic material was purified using column chromatography to obtain a target product, 2.82. g (yield: 65%) of N-butyl-1-(o-tolyl)-N-(o-tolyl(4-(tributylsilyl)phenyl)phosphaneyl)-1-(4-(tributylsilyl)phenyl)phosphanamine.

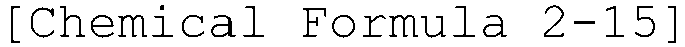

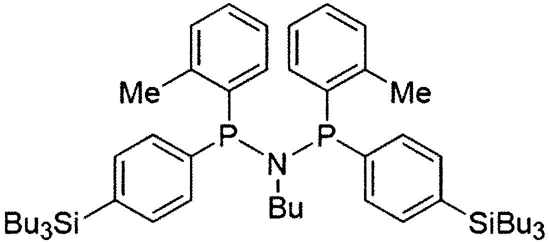

¹H NMR (500 MHz, CDCl₃) : δ 7.42(m, 4H), 7.37 (m, 2H), 7.34 (m, 2H), 7.19 (m, 2H), 7.17 (m, 4H), 7.13 (m, 2H), 2.73 (m, 2H), 2.37(m, 6H), 1.61(m, 2H), 1.44(m, 12H), 1.37 (m, 14H), 1.31(m, 12H), 0.91(m, 21H)

### Synthesis Example 16: Synthesis of ligand compound represented by Chemical Formula 2-16

0.37 g of butylamine, 1.26 g of triethylamine, and 10 ml of dichloromethane were charged into a well-dried flask in a nitrogen atmosphere, and then stirring was started with cooling to 0°C. 5.03 g of chloro(4-(tributylsilyl)phenyl) (2-(trifluoromethoxy)phenyl)phosphane was diluted in 10 ml of dichloromethane and then slowly charged into the flask. After stirring the reactant for 24 hours, the conversion rate was checked through NMR. After the reaction was completed, the precipitate was removed by filtration, and the solvent was removed by reducing the pressure. The obtained organic material was purified using column chromatography to obtain a target product, 3.37 g (yield: 67%) of N-butyl-1-(4-(tributylsilyl)phenyl)-N-((4-(tributylsilyl)phenyl)(2-(trifluoromethoxy)phenyl)phosphaneyl)-1-(2-(trifluoromethoxy)phenyl)phosphanamine.

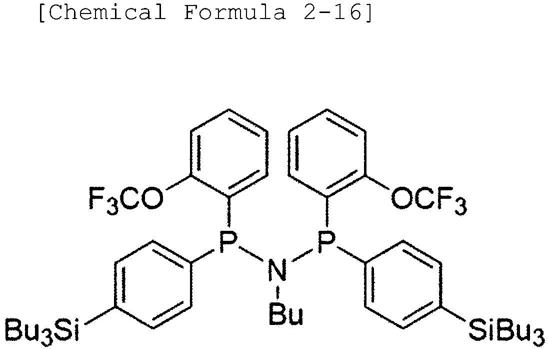

¹H NMR (500 MHz, CDCl₃) : δ 7.43 (m, 4H), 7.42 (m, 2H), 7.20(m, 4H), 7.19(m, 4H), 7.04(m, 2H), 2.65(m, 2H), 1.59 (m, 2H), 1.47(m, 12H), 1.34 (m, 14H), 1.29(m, 12H), 0.94 (m, 21H)

### Synthesis Example 17: Synthesis of ligand compound represented by Chemical Formula 2-17

0.37 g of butylamine, 1.26 g of triethylamine, and 10 ml of dichloromethane were charged into a well-dried flask in a nitrogen atmosphere, and then stirring was started with cooling to 0°C. 5.07 g of chloro(4-(tributylsilyl)phenyl) (2-(trifluoro-l4-sulfaneyl)phenyl)phosphane was diluted in 10 ml of dichloromethane and then slowly charged into the flask. After stirring the reactant for 20 hours, the conversion rate was checked through NMR. After the reaction was completed, the precipitate was removed by filtration, and the solvent was removed by reducing the pressure. The obtained organic material was purified using column chromatography to obtain a target product, 3.4 g (yield: 67%) of N-butyl-1-(4-(tributylsilyl)phenyl)-N-((4-(tributylsilyl)phenyl)(2-(trifluoro-14-sulfaneyl)phenyl)phosphaneyl)-1-(2-(trifluoro-14-sulfaneyl)phenyl)phosphanamine.

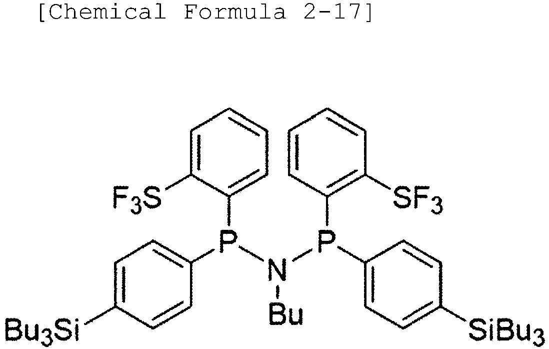

¹H NMR (500 MHz, CDCl₃) : δ 7.48 (m, 4H), 7.43 (m, 2H), 7.33(m, 2H), 7.27(m, 2H), 7.18(m, 4H), 7.00(m, 2H), 2.68(m, 2H), 1.54 (m, 2H), 1.47(m, 12H), 1.35(m, 14H), 1.27(m, 12H), 0.95(m, 21H)

### Synthesis Example 18: Synthesis of ligand compound represented by Chemical Formula 2-18

0.37 g of butylamine, 1.26 g of triethylamine, and 10 ml of dichloromethane were charged into a well-dried flask in a nitrogen atmosphere, and then stirring was started with cooling to 0°C. 4.65 g of chloro(2-(methylthio)phenyl)(4-(tributylsilyl)phenyl)phosphanewas diluted in 10 ml of dichloromethane and then slowly charged into the flask. After stirring the reactant for 12 hours, the conversion rate was checked through NMR. After the reaction was completed, the precipitate was removed by filtration, and the solvent was removed by reducing the pressure. The obtained organic material was purified using column chromatography to obtain a target product, 3.77 g (yield: 81%) of N-butyl-1-(2-(methylthio)phenyl)-N-((2-(methylthio) phenyl) (4-(tributylsilyl)phenyl)phosphaneyl)-1-(4-(tributylsilyl)phenyl)phosphanamine.

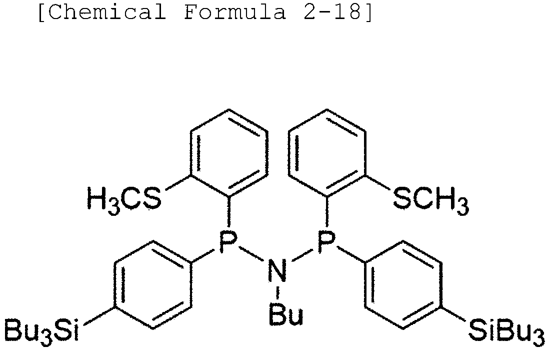

¹H NMR (500 MHz, CDCl₃) : δ 7.43 (m, 2H), 7.42(m, 4H), 7.41(m, 2H), 7.23(m, 2H), 7.13 (m, 4H), 7.06 (m, 2H), 2.73 (m, 2H), 2.51(m, 6H), 1.57(m, 2H), 1.51(m, 12H), 1.31(m, 14H), 1.28 (m, 12H), 0.92(m, 21H)

### Synthesis Example 19: Synthesis of ligand compound represented by Chemical Formula 2-19

0.37 g of butylamine, 1.26 g of triethylamine, and 10 ml of dichloromethane were charged into a well-dried flask in a nitrogen atmosphere, and then stirring was started with cooling to 0°C. 5.13 g of 2-(chloro(4-(tributylsilyl)phenyl)phosphaneyl)phenyl methanesulfonate was diluted in 10 ml of dichloromethane and then slowly charged into the flask. After stirring the reactant for 12 hours, the conversion rate was checked through NMR. After the reaction was completed, the precipitate was removed by filtration, and the solvent was removed by reducing the pressure. The obtained organic material was purified using column chromatography to obtain a target product, 4.11 g (yield: 80%) of ((butylazanediyl)bis((4-(tributylsilyl)phenyl)phosphanediyl))bis(2,1-phenylene)dimethanesulfonate.

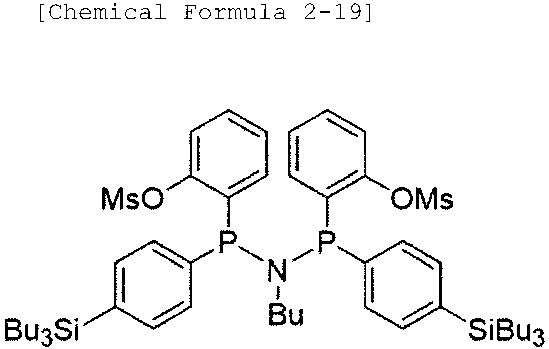

¹H NMR (500 MHz, CDCl₃) : δ 7.52(m, 2H), 7.49(m, 4H), 7.45(m, 2H), 7.32 (m, 2H), 7.28 (m, 2H), 7.21(m, 4H), 3.57 (m, 6H), 2.65(m, 2H), 1.56 (m, 2H), 1.50 (m, 12H), 1.38 (m, 14H), 1.26 (m, 12H), 0.93 (m, 21H)

### Synthesis Example 20: Synthesis of ligand compound represented by Chemical Formula 2-20

0.37 g of butylamine, 1.26 g of triethylamine, and 10 ml of dichloromethane were charged into a well-dried flask in a nitrogen atmosphere, and then stirring was started with cooling to 0°C. 4.91 g of chloro(4-(tributylsilyl)phenyl)(2-(trimethylsilyl)phenyl)phosphane was diluted in 10 ml of dichloromethane and then slowly charged into the flask. After stirring the reactant for 16 hours, the conversion rate was checked through NMR. After the reaction was completed, the precipitate was removed by filtration, and the solvent was removed by reducing the pressure. The obtained organic material was purified using column chromatography to obtain a target product, 3.98 g (yield: 81%) of N-butyl-1-(4-(tributylsilyl)phenyl)-N-((4-(tributylsilyl)phenyl)(2-(trimethylsilyl)phenyl)phosphaneyl)-1-(2-(trimethylsilyl)phenyl)phosphanamine.

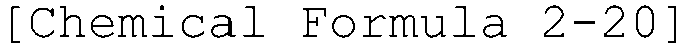

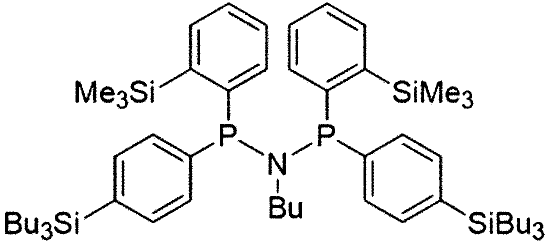

¹H NMR (500 MHz, CDCl₃) : δ 7.49(m, 8H), 7.32 (m, 2H), 7.20(m, 6H), 2.68(m, 2H), 1.55(m, 2H), 1.50 (m, 12H), 1.38 (m, 14H), 1.25 (m, 12H), 0.93 (m, 21H), 0.31(m, 18H)

### Synthesis Example 21: Synthesis of ligand compound represented by Chemical Formula 2-21

0.37 g of butylamine, 1.26 g of triethylamine, and 10 ml of dichloromethane were charged into a well-dried flask in a nitrogen atmosphere, and then stirring was started with cooling to 0°C. 4.59 g of benzofuran-7-ylchloro(4-(tributylsilyl)phenyl)phosphane was diluted in 10 ml of dichloromethane and then slowly charged into the flask. After stirring the reactant for 18 hours, the conversion rate was checked through NMR. After the reaction was completed, the precipitate was removed by filtration, and the solvent was removed by reducing the pressure. The obtained organic material was purified using column chromatography to obtain a target product, 4.41 g (yield: 96%) of 1-(benzofuran-7-yl)-N-(benzofuran-7-yl(4-(tributylsilyl)phenyl)phosphaneyl)-N-butyl-1-(4-(tributylsilyl)phenyl)phosphanamine.

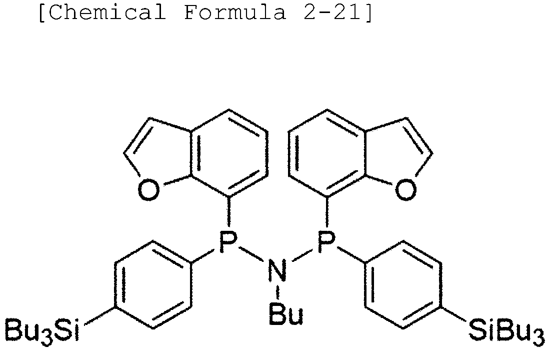

¹H NMR (500 MHz, CDCl₃): δ 7.69(m, 2H), 7. 64 (m, 2H), 7.50 (m, 2H), 7.46(m, 4H), 7.34(m, 2H), 7.14(m, 4H), 6.76(m, 2H), 2.72 (m, 2H), 1.57(m, 2H), 1.44(m, 12H), 1.33 (m, 14H), 1.32 (m, 12H), 0.97 (m, 21H)

### Synthesis Example 22: Synthesis of ligand compound represented by Chemical Formula 2-22

0.37 g of butylamine, 1.26 g of triethylamine, and 10 ml of dichloromethane were charged into a well-dried flask in a nitrogen atmosphere, and then stirring was started with cooling to 0°C. 5.09 g of chloro (dibenzo [b, d] furan-4-yl) (4-(tributylsilyl)phenyl)phosphane was diluted in 10 ml of dichloromethane and then slowly charged into the flask. After stirring the reactant for 15 hours, the conversion rate was checked through NMR. After the reaction was completed, the precipitate was removed by filtration, and the solvent was removed by reducing the pressure. The obtained organic material was purified using column chromatography to obtain a target product, 4.43 g (yield: 87%) of N-butyl-1-(dibenzo[b,d]furan-4-yl)-N-(dibenzo[b,d]furan-4-yl(4-(tributylsilyl)phenyl)phosphaneyl)-1-(4-(tributylsilyl)phenyl)phosphanamine.

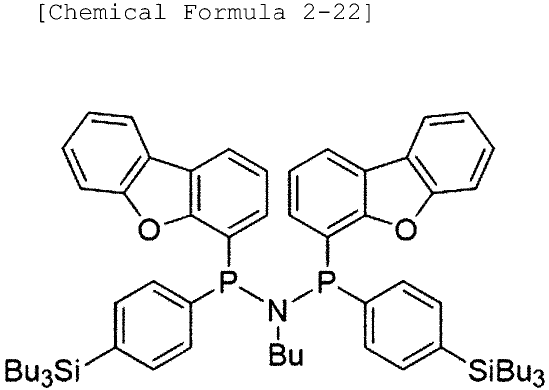

¹H NMR (500 MHz, CDCl₃) : δ 8.00(m, 4H), 7.57 (m, 2H), 7.42 (m, 6H), 7.39 (m, 2H), 7.38 (m, 2H), 7.37 (m, 2H), 7.15 (m, 4H), 2.69(m, 2H), 1.59 (m, 2H), 1.47(m, 12H), 1.31(m, 14H), 1.27(m, 12H), 0.99 (m, 21H)

### Synthesis Example 23: Synthesis of ligand compound represented by Chemical Formula 2-23

0.54 g of benzylamine, 1.26 g of triethylamine, and 10 ml of dichloromethane were charged into a well-dried flask in a nitrogen atmosphere, and then stirring was started with cooling to 0°C. 4.37 g of chloro(2-fluorophenyl) (4-(tributylsilyl)phenyl)phosphane was diluted in 10 ml of dichloromethane and then slowly charged into the flask. After stirring the reactant for 20 hours, the conversion rate was checked through NMR. After the reaction was completed, the precipitate was removed by filtration, and the solvent was removed by reducing the pressure. The obtained organic material was purified using column chromatography to obtain a target product, 4.06 g (yield: 88%) of N,N-bis((2-fluorophenyl) (4-(tributylsilyl)phenyl)phosphaneyl)benzamide.

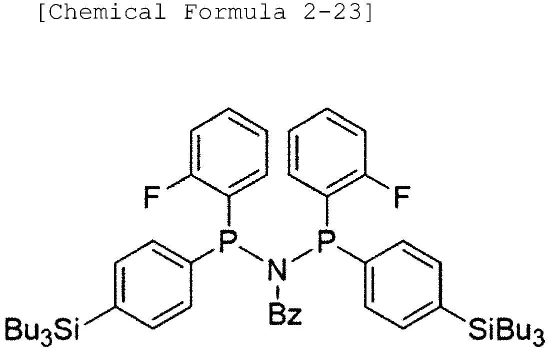

¹H NMR (500 MHz, CDCl₃): δ 8.02(m, 2H), 7.67(m, 1H), 7.61(m, 2H), 7.55 (m, 2H), 7.45(m, 4H), 7.28(m, 2H), 7.23 (m, 2H), 7.17 (m, 6H), 1.51(m, 12H), 1.36(m, 12H), 1.29(m, 12H), 0.95(m, 18H)

### Synthesis Example 24: Synthesis of ligand compound represented by Chemical Formula 2-24

0.54 g of benzylamine, 1.26 g of triethylamine, and 10 ml of dichloromethane were charged into a well-dried flask in a nitrogen atmosphere, and then stirring was started with cooling to 0°C. 4.87 g of chloro(4-(tributylsilyl)phenyl) (2-(trifluoromethyl)phenyl)phosphane was diluted in 10 ml of dichloromethane and then slowly charged into the flask. After stirring the reactant for 10 hours, the conversion rate was checked through NMR. After the reaction was completed, the precipitate was removed by filtration, and the solvent was removed by reducing the pressure. The obtained organic material was purified using column chromatography to obtain a target product, 3.99 g (yield: 78%) of N,N-bis((4-(tributylsilyl)phenyl) (2-(trifluoromethyl)phenyl)phosphaneyl)benzamide.

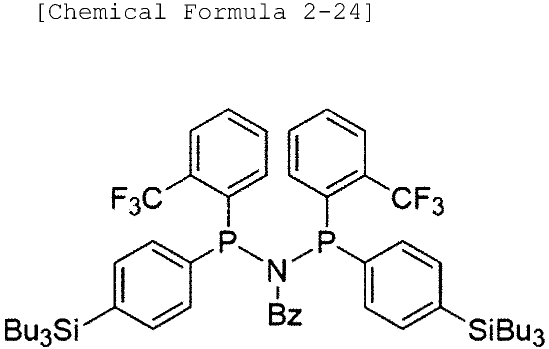

¹H NMR (500 MHz, CDCl₃): δ 8.00 (m, 2H), 7.70 (m, 1H), 7.59(m, 2H), 7.56(m, 2H), 7.50(m, 2H), 7.49(m, 4H), 7.28 (m, 2H), 7.21(m, 4H), 7.18 (m, 2H), 1.43(m, 12H), 1.39 (m, 12H), 1.32 (m, 12H), 0.97 (m, 18H)

### Synthesis Example 25: Synthesis of ligand compound represented by Chemical Formula 2-25

0.54 g of benzylamine, 1.26 g of triethylamine, and 10 ml of dichloromethane were charged into a well-dried flask in a nitrogen atmosphere, and then stirring was started with cooling to 0°C. 4.49 g of chloro(2-methoxyphenyl)(4-(tributylsilyl)phenyl)phosphane was diluted in 10 ml of dichloromethane and then slowly charged into the flask. After stirring the reactant for 13 hours, the conversion rate was checked through NMR. After the reaction was completed, the precipitate was removed by filtration, and the solvent was removed by reducing the pressure. The obtained organic material was purified using column chromatography to obtain a target product, 3.36 g (yield: 71%) of N,N-bis((2-methoxyphenyl)(4-(tributylsilyl)phenyl)phosphaneyl)benzamide.

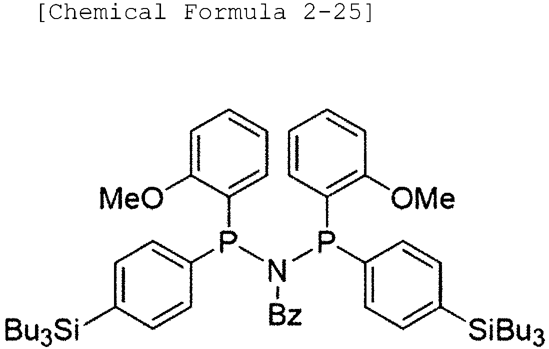

¹H NMR (500 MHz, CDCl₃): δ 7.99 (m, 2H), 7.68 (m, 1H), 7.58 (m, 2H), 7.49(m, 4H), 7.42(m, 2H), 7.21(m, 6H), 7.12 (m, 2H), 6.99(m, 2H), 3.87(m, 6H), 1.47(m, 12H), 1.33(m, 12H), 1.30(m, 12H), 0.95(m, 18H)

### Synthesis Example 26: Synthesis of ligand compound represented by Chemical Formula 2-26

0.54 g of benzylamine, 1.26 g of triethylamine, and 10 ml of dichloromethane were charged into a well-dried flask in a nitrogen atmosphere, and then stirring was started with cooling to 0°C. 4.33 g of chloro (o-tolyl) (4-(tributylsilyl)phenyl)phosphane was diluted in 10 ml of dichloromethane and then slowly charged into the flask. After stirring the reactant for 24 hours, the conversion rate was checked through NMR. After the reaction was completed, the precipitate was removed by filtration, and the solvent was removed by reducing the pressure. The obtained organic material was purified using column chromatography to obtain a target product, 3.2 g (yield: 70%) of N,N-bis(o-tolyl(4-(tributylsilyl)phenyl)phosphaneyl)benzamide.

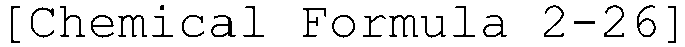

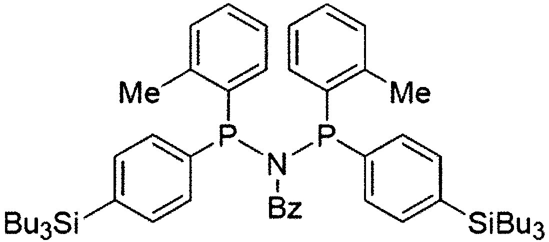

¹H NMR (500 MHz, CDCl₃): δ 7.99 (m, 2H), 7. 62 (m, 1H), 7.56 (m, 2H), 7.46(m, 4H), 7.41(m, 2H), 7.34 (m, 2H), 7.20 (m, 2H), 7.19 (m, 2H), 7.18(m, 4H), 2.36(m, 6H), 1.52(m, 12H), 1.32 (m, 12H), 1.24(m, 12H), 0.98 (m, 18H)

### Synthesis Example 27: Synthesis of ligand compound represented by Chemical Formula 2-27

0.54 g of benzylamine, 1.26 g of triethylamine, and 10 ml of dichloromethane were charged into a well-dried flask in a nitrogen atmosphere, and then stirring was started with cooling to 0°C. 5.03 g of chloro(4-(tributylsilyl)phenyl)(2-(trifluoromethoxy)phenyl)phosphane was diluted in 10 ml of dichloromethane and then slowly charged into the flask. After stirring the reactant for 14 hours, the conversion rate was checked through NMR. After the reaction was completed, the precipitate was removed by filtration, and the solvent was removed by reducing the pressure. The obtained organic material was purified using column chromatography to obtain a target product, 3.95 g (yield: 75%) of N,N-bis((4-(tributylsilyl)phenyl)(2-(trifluoromethoxy)phenyl)phosphaneyl)benzamide.

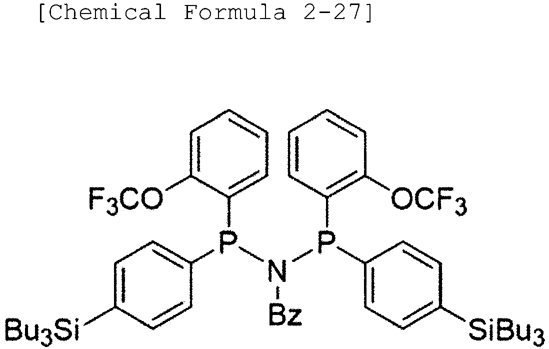

¹H NMR (500 MHz, CDCl₃): δ 8.00(m, 2H), 7.67 (m, 1H), 7.59(m, 2H), 7.47(m, 4H), 7.41(m, 2H), 7.21(m, 4H), 7.18 (m, 2H), 7.17 (m, 2H), 6.98(m, 2H), 1.47(m, 12H), 1.32 (m, 12H), 1.30 (m, 12H), 0.91(m, 18H)

### Synthesis Example 28: Synthesis of ligand compound represented by Chemical Formula 2-28

0.54 g of benzylamine, 1.26 g of triethylamine, and 10 ml of dichloromethane were charged into a well-dried flask in a nitrogen atmosphere, and then stirring was started with cooling to 0°C. 5.07 g of chloro(4-(tributylsilyl)phenyl) (2-(trifluoro-14-sulfaneyl)phenyl)phosphane was diluted in 10 ml of dichloromethane and then slowly charged into the flask. After stirring the reactant for 11 hour, the conversion rate was checked through NMR. After the reaction was completed, the precipitate was removed by filtration, and the solvent was removed by reducing the pressure. The obtained organic material was purified using column chromatography to obtain a target product, 3.29 g (yield: 62%) of N,N-bis((4-(tributylsilyl)phenyl)(2-(trifluoro-14-sulfaneyl)phenyl)phosphaneyl)benzamide.

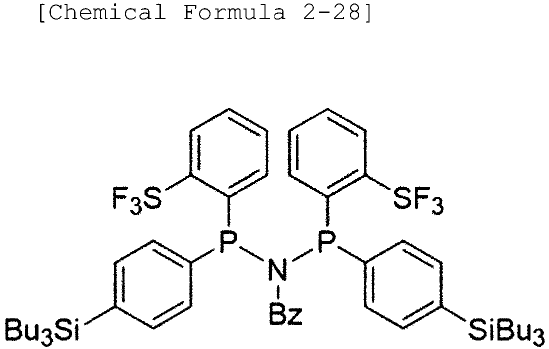

¹H NMR (500 MHz, CDCl₃) : δ 7.96(m, 2H), 7.72 (m, 1H), 7.59(m, 2H), 7.48(m, 4H), 7.37 (m, 2H), 7.32 (m, 2H), 7.22 (m, 6H), 7.03 (m, 2H), 1.48(m, 12H), 1.33 (m, 12H), 1.27 (m, 12H), 0.97 (m, 18H)

### Synthesis Example 29: Synthesis of ligand compound represented by Chemical Formula 2-29

0.54 g of benzylamine, 1.26 g of triethylamine, and 10 ml of dichloromethane were charged into a well-dried flask in a nitrogen atmosphere, and then stirring was started with cooling to 0°C. 4.65 g of chloro(2-(methylthio) phenyl)(4-(tributylsilyl)phenyl)phosphanewas diluted in 10 ml of dichloromethane and then slowly charged into the flask. After stirring the reactant for 15 hours, the conversion rate was checked through NMR. After the reaction was completed, the precipitate was removed by filtration, and the solvent was removed by reducing the pressure. The obtained organic material was purified using column chromatography to obtain a target product, 4.31 g (yield: 88%) of N,N-bis((2-(methylthio)phenyl) (4-(tributylsilyl)phenyl)phosphaneyl)benzamide.

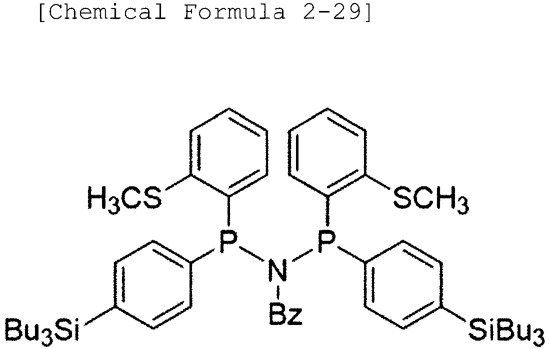

¹H NMR (500 MHz, CDCl₃): δ 8.01(m, 2H), 7.63(m, 3H), 7.47(m, 4H), 7.46(m, 2H), 7.38(m, 2H), 7.26 (m, 2H), 7.21(m, 4H), 7.14 (m, 2H), 2.56 (m, 6H), 1.45(m, 12H), 1.36(m, 12H), 1.23 (m, 12H), 0.91(m, 18H)

### Synthesis Example 30: Synthesis of ligand compound represented by Chemical Formula 2-30

0.54 g of benzylamine, 1.26 g of triethylamine, and 10 ml of dichloromethane were charged into a well-dried flask in a nitrogen atmosphere, and then stirring was started with cooling to 0°C. 5.13 g of 2-(chloro(4-(tributylsilyl)phenyl)phosphaneyl)phenyl methanesulfonate was diluted in 10 ml of dichloromethane and then slowly charged into the flask. After stirring the reactant for 14 hours, the conversion rate was checked through NMR. After the reaction was completed, the precipitate was removed by filtration, and the solvent was removed by reducing the pressure. The obtained organic material was purified using column chromatography to obtain a target product, 3.49 g (yield: 65%) of ((benzoylazanediyl)bis((4-(tributylsilyl)phenyl)phosphanediyl))bis(2,1-phenylene) dimethanesulfonate.

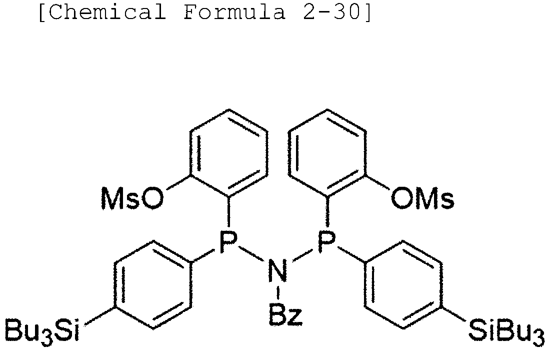

¹H NMR (500 MHz, CDCl₃): δ 8.01(m, 2H), 7.66(m, 1H), 7.60(m, 2H), 7.50 (m, 2H), 7.44(m, 2H), 7.42(m, 4H), 7.31(m, 2H), 7.24(m, 2H), 7.20 (m, 4H), 3.57 (m, 6H), 1.53(m, 12H), 1.34(m, 12H), 1.23 (m, 12H), 0.97(m, 18H)

### Synthesis Example 31: Synthesis of ligand compound represented by Chemical Formula 2-31

0.54 g of benzylamine, 1.26 g of triethylamine, and 10 ml of dichloromethane were charged into a well-dried flask in a nitrogen atmosphere, and then stirring was started with cooling to 0°C. 4.91 g of chloro(4-(tributylsilyl)phenyl) (2-(trimethylsilyl)phenyl)phosphane was diluted in 10 ml of dichloromethane and then slowly charged into the flask. After stirring the reactant for 15 hours, the conversion rate was checked through NMR. After the reaction was completed, the precipitate was removed by filtration, and the solvent was removed by reducing the pressure. The obtained organic material was purified using column chromatography to obtain a target product, 3.66 g (yield: 71%) of N,N-bis((4-(tributylsilyl)phenyl)(2-(trimethylsilyl)phenyl)phosphaneyl)benzamide.

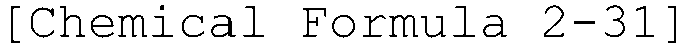

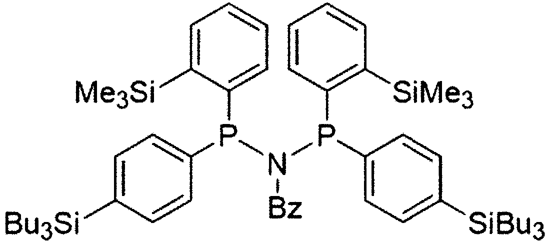

¹H NMR (500 MHz, CDCl₃) : δ 8.01 (m, 2H), 7.71(m, 1H), 7.61(m, 2H), 7.50(m, 8H), 7.31(m, 2H), 7.19(m, 6H), 1.52 (m, 12H), 1.38(m, 12H), 1.29 (m, 12H), 0.95 (m, 18H), 0.34 (m, 18H)

### Synthesis Example 32: Synthesis of ligand compound represented by Chemical Formula 2-32

0.54 g of benzylamine, 1.26 g of triethylamine, and 10 ml of dichloromethane were charged into a well-dried flask in a nitrogen atmosphere, and then stirring was started with cooling to 0°C. 4.59 g of benzofuran-7-ylchloro(4-(tributylsilyl)phenyl)phosphane was diluted in 10 ml of dichloromethane and then slowly charged into the flask. After stirring the reactant for 23 hours, the conversion rate was checked through NMR. After the reaction was completed, the precipitate was removed by filtration, and the solvent was removed by reducing the pressure. The obtained organic material was purified using column chromatography to obtain a target product, 3.53 g (yield: 73%) of N,N-bis(benzofuran-7-yl(4-(tributylsilyl)phenyl)phosphaneyl)benzamide.

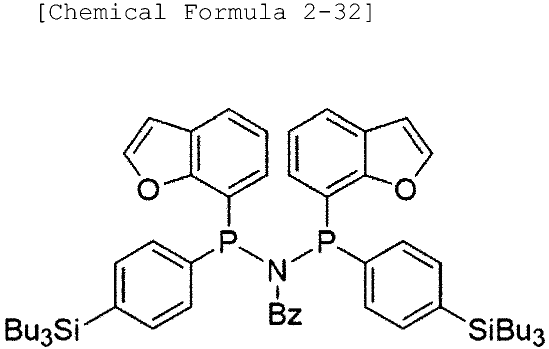

¹H NMR (500 MHz, CDCl₃) : δ 7.97 (m, 2H), 7.70 (m, 1H), 7. 67 (m, 2H), 7.60(m, 2H), 7.56(m, 2H), 7.48(m, 6H), 7.36 (m, 2H), 7.16 (m, 4H), 6.80 (m, 2H), 1.50 (m, 12H), 1.33(m, 12H), 1.26(m, 12H), 0.91(m, 18H)

### Synthesis Example 33: Synthesis of ligand compound represented by Chemical Formula 2-33

0.54 g of benzylamine, 1.26 g of triethylamine, and 10 ml of dichloromethane were charged into a well-dried flask in a nitrogen atmosphere, and then stirring was started with cooling to 0°C. 5.09 g of chloro (dibenzo [b, d] furan-4-yl) (4-(tributylsilyl)phenyl)phosphane was diluted in 10 ml of dichloromethane and then slowly charged into the flask. After stirring the reactant for 14 hours, the conversion rate was checked through NMR. After the reaction was completed, the precipitate was removed by filtration, and the solvent was removed by reducing the pressure. The obtained organic material was purified using column chromatography to obtain a target product, 3.79 g (yield: 71%) of N,N-bis(dibenzo[b,d]furan-4-yl(4-(tributylsilyl)phenyl)phosphaneyl)benzamide.

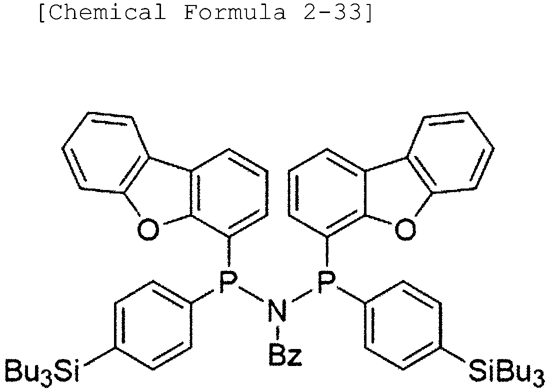

¹H NMR (500 MHz, CDCl₃): δ 8.04(m, 4H), 7.96(m, 2H), 7.62(m, 1H), 7.57(m, 4H), 7.47(m, 2H), 7.42(m, 2H), 7.41(m, 6H), 7.35 (m, 2H), 7.14(m, 4H), 1.49(m, 12H), 1.31(m, 12H), 1.27(m, 12H), 0.96 (m, 18H)

### Synthesis Example 34: Synthesis of ligand compound represented by Chemical Formula 2-34

0.54 g of isopropyl amine, 1.26 g of triethylamine, and 10 ml of dichloromethane were charged into a well-dried flask in a nitrogen atmosphere, and then stirring was started with cooling to 0°C. 4.37 g of chloro(2-fluorophenyl) (4-(tributylsilyl)phenyl)phosphane was diluted in 10 ml of dichloromethane and then slowly charged into the flask. After stirring the reactant for 15 hours, the conversion rate was checked through NMR. After the reaction was completed, the precipitate was removed by filtration, and the solvent was removed by reducing the pressure. The obtained organic material was purified using column chromatography to obtain a target product, 2.67 g (yield: 62%) of 1-(2-fluorophenyl)-N-((2-fluorophenyl) (4-(tributylsilyl)phenyl)phosphaneyl)-N-isopropyl-1-(4-(tributylsilyl)phenyl)phosphanamine.

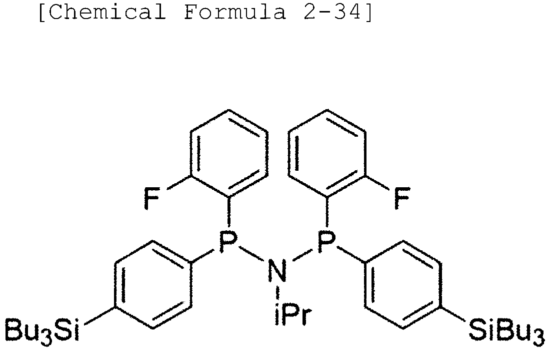

¹H NMR (500 MHz, CDCl₃) : δ 7.67(m, 2H), 7.48 (m, 4H), 7.26(m, 2H), 7.20(m, 2H), 7.17(m, 6H), 2.93(m, 1H), 1.51(m, 12H), 1.33 (m, 12H), 1.30 (m, 12H), 1.13(m, 6H), 0.97 (m, 18H)

### Synthesis Example 35: Synthesis of ligand compound represented by Chemical Formula 2-35

0.54 g of isopropyl amine, 1.26 g of triethylamine, and 10 ml of dichloromethane were charged into a well-dried flask in a nitrogen atmosphere, and then stirring was started with cooling to 0°C. 4.87 g of chloro(4-(tributylsilyl)phenyl)(2-(trifluoromethyl)phenyl)phosphane was diluted in 10 ml of dichloromethane and then slowly charged into the flask. After stirring the reactant for 16 hours, the conversion rate was checked through NMR. After the reaction was completed, the precipitate was removed by filtration, and the solvent was removed by reducing the pressure. The obtained organic material was purified using column chromatography to obtain a target product, 3.22 g (yield: 67%) of N-isopropyl-1-(4-(tributylsilyl)phenyl)-N-((4-(tributylsilyl)phenyl)(2-(trifluoromethyl)phenyl)phosphaneyl)-1-(2-(trifluoromethyl)phenyl)phosphanamine.

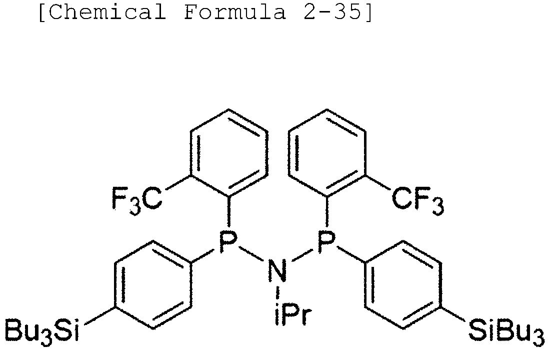

¹H NMR (500 MHz, CDCl₃) : δ 7.48(m, 6H), 7.44(m, 2H), 7.28(m, 2H), 7.23(m, 4H), 7.11(m, 2H), 2.92 (m, 1H), 1.45(m, 12H), 1.31(m, 24H), 1.07(m, 6H), 0.91(m, 18H)

### Synthesis Example 36: Synthesis of ligand compound represented by Chemical Formula 2-36

0.54 g of isopropyl amine, 1.26 g of triethylamine, and 10 ml of dichloromethane were charged into a well-dried flask in a nitrogen atmosphere, and then stirring was started with cooling to 0°C. 4.49 g of chloro(2-methoxyphenyl) (4-(tributylsilyl)phenyl)phosphane was diluted in 10 ml of dichloromethane and then slowly charged into the flask. After stirring the reactant for 23 hours, the conversion rate was checked through NMR. After the reaction was completed, the precipitate was removed by filtration, and the solvent was removed by reducing the pressure. The obtained organic material was purified using column chromatography to obtain a target product, 4.38 g (yield: 99%) of N-isopropyl-1-(2-methoxyphenyl)-N-((2-methoxyphenyl)(4-(tributylsilyl)phenyl)phosphaneyl)-1-(4-(tributylsilyl)phenyl)phosphanamine.

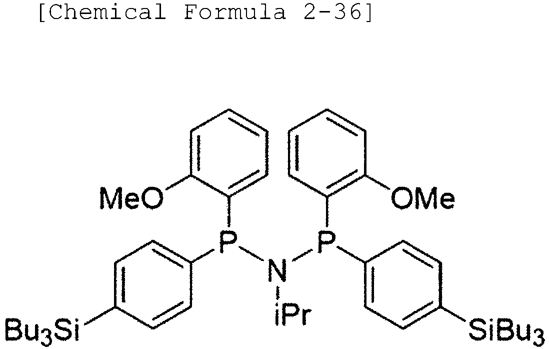

¹H NMR (500 MHz, CDCl₃) : δ 7.46(m, 2H), 7.41 (m, 4H), 7.25 (m, 2H), 7.15(m, 4H), 7.12 (m, 2H), 6.96 (m, 2H), 3.89(m, 6H), 2.93 (m, 1H), 1.52 (m, 12H), 1.37(m, 12H), 1.32 (m, 12H), 1.08 (m, 6H), 0.93 (m, 18H)

### Synthesis Example 37: Synthesis of ligand compound represented by Chemical Formula 2-37

0.54 g of isopropyl amine, 1.26 g of triethylamine, and 10 ml of dichloromethane were charged into a well-dried flask in a nitrogen atmosphere, and then stirring was started with cooling to 0°C. 4.33 g of chloro (o-tolyl) (4-(tributylsilyl)phenyl)phosphane was diluted in 10 ml of dichloromethane and then slowly charged into the flask. After stirring the reactant for 11 hour, the conversion rate was checked through NMR. After the reaction was completed, the precipitate was removed by filtration, and the solvent was removed by reducing the pressure. The obtained organic material was purified using column chromatography to obtain a target product, 3.2 g (yield: 75%) of N-isopropyl-1-(o-tolyl)-N-(o-tolyl(4-(tributylsilyl)phenyl)phosphaneyl)-1-(4-(tributylsilyl)phenyl)phosphanamine.

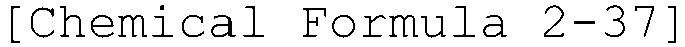

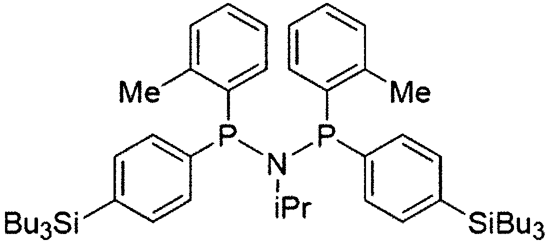

¹H NMR (500 MHz, CDCl₃): δ 7.44(m, 4H), 7.37(m, 2H), 7.34(m, 2H), 7.24(m, 2H), 7.22(m, 4H), 7.12(m, 2H), 2.93(m, 1H), 2.37 (m, 6H), 1.48(m, 12H), 1.37 (m, 12H), 1.29(m, 12H), 1.13(m, 6H), 0.99(m, 18H)

### Synthesis Example 38: Synthesis of ligand compound represented by Chemical Formula 2-38

0.54 g of isopropyl amine, 1.26 g of triethylamine, and 10 ml of dichloromethane were charged into a well-dried flask in a nitrogen atmosphere, and then stirring was started with cooling to 0°C. 5.03 g of chloro(4-(tributylsilyl)phenyl)(2-(trifluoromethoxy)phenyl)phosphane was diluted in 10 ml of dichloromethane and then slowly charged into the flask. After stirring the reactant for 21 hour, the conversion rate was checked through NMR. After the reaction was completed, the precipitate was removed by filtration, and the solvent was removed by reducing the pressure. The obtained organic material was purified using column chromatography to obtain a target product, 3.77 g (yield: 76%) of N-isopropyl-1-(4-(tributylsilyl)phenyl)-N-((4-(tributylsilyl)phenyl)(2-(trifluoromethoxy)phenyl)phosphaneyl)-1-(2-(trifluoromethoxy)phenyl)phosphanamine.

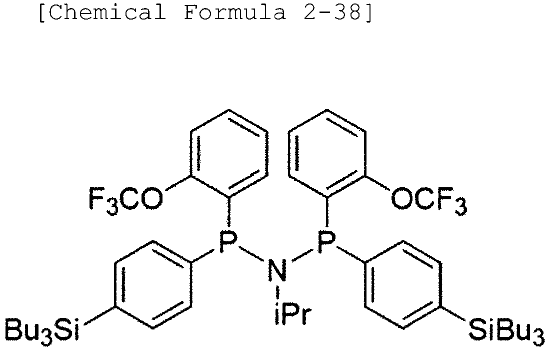

¹H NMR (500 MHz, CDCl₃) : δ 7.47 (m, 4H), 7.44 (m, 2H), 7.21(m, 4H), 7.15(m, 2H), 7.12(m, 2H), 7.05 (m, 2H), 2.91(m, 1H), 1.48(m, 12H), 1.34(m, 12H), 1.27(m, 12H), 1.07(m, 6H), 0.97 (m, 18H)

### Synthesis Example 39: Synthesis of ligand compound represented by Chemical Formula 2-39

0.54 g of isopropyl amine, 1.26 g of triethylamine, and 10 ml of dichloromethane were charged into a well-dried flask in a nitrogen atmosphere, and then stirring was started with cooling to 0°C. 5.07 g of chloro(4-(tributylsilyl)phenyl)(2-(trifluoro-14-sulfaneyl)phenyl)phosphane was diluted in 10 ml of dichloromethane and then slowly charged into the flask. After stirring the reactant for 19 hours, the conversion rate was checked through NMR. After the reaction was completed, the precipitate was removed by filtration, and the solvent was removed by reducing the pressure. The obtained organic material was purified using column chromatography to obtain a target product, 3.3 g (yield: 66%) of N-isopropyl-1-(4-(tributylsilyl)phenyl)-N-((4-(tributylsilyl)phenyl)(2-(trifluoro-14-sulfaneyl)phenyl)phosphaneyl)-1-(2-(trifluoro-14-sulfaneyl)phenyl)phosphanamine.

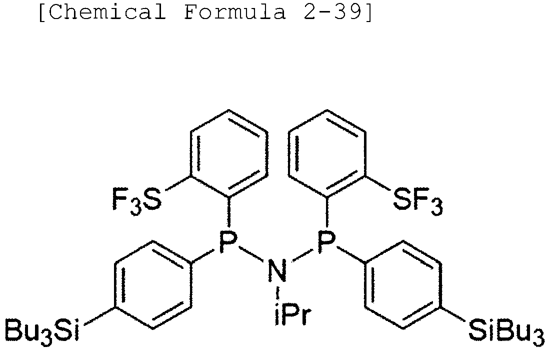

¹H NMR (500 MHz, CDCl₃) : δ 7.43(m, 6H), 7.28(m, 2H), 7.27(m, 2H), 7.19(m, 4H), 6.99(m, 2H), 2.88(m, 1H), 1.46(m, 12H), 1.39 (m, 12H), 1.25 (m, 12H), 1.12 (m, 6H), 0.95(m, 18H)

### Synthesis Example 40: Synthesis of ligand compound represented by Chemical Formula 2-40

0.54 g of isopropyl amine, 1.26 g of triethylamine, and 10 ml of dichloromethane were charged into a well-dried flask in a nitrogen atmosphere, and then stirring was started with cooling to 0°C. 4.65 g of chloro(2-(methylthio)phenyl)(4-(tributylsilyl)phenyl)phosphanewas diluted in 10 ml of dichloromethane and then slowly charged into the flask. After stirring the reactant for 21 hour, the conversion rate was checked through NMR. After the reaction was completed, the precipitate was removed by filtration, and the solvent was removed by reducing the pressure. The obtained organic material was purified using column chromatography to obtain a target product, 3.35 g (yield: 73%) of N-isopropyl-1-(2-(methylthio)phenyl)-N-((2-(methylthio)phenyl)(4-(tributylsilyl)phenyl)phosphaneyl)-1-(4-(tributylsilyl)phenyl)phosphanamine.

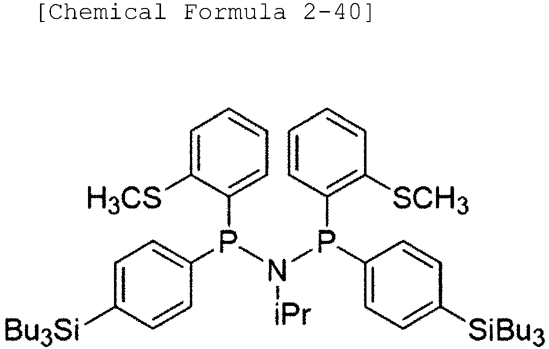

¹H NMR (500 MHz, CDCl₃) : δ 7.50 (m, 4H), 7.40(m, 2H), 7.37 (m, 2H), 7.21(m, 2H), 7.14 (m, 4H), 7.10 (m, 2H), 2.92 (m, 1H), 2.51(m, 6H), 1.48(m, 12H), 1.37 (m, 12H), 1.32 (m, 12H), 1.15(m, 6H), 0.92(m, 18H)

### Synthesis Example 41: Synthesis of ligand compound represented by Chemical Formula 2-41

0.54 g of isopropyl amine, 1.26 g of triethylamine, and 10 ml of dichloromethane were charged into a well-dried flask in a nitrogen atmosphere, and then stirring was started with cooling to 0°C. 5.13 g of 2-(chloro(4-(tributylsilyl)phenyl)phosphaneyl)phenyl methanesulfonate was diluted in 10 ml of dichloromethane and then slowly charged into the flask. After stirring the reactant for 22 hours, the conversion rate was checked through NMR. After the reaction was completed, the precipitate was removed by filtration, and the solvent was removed by reducing the pressure. The obtained organic material was purified using column chromatography to obtain a target product, 3.64 g (yield: 72%) of ((isopropylazanediyl)bis((4-(tributylsilyl)phenyl)phosphanediyl))bis(2,1-phenylene) dimethanesulfonate.

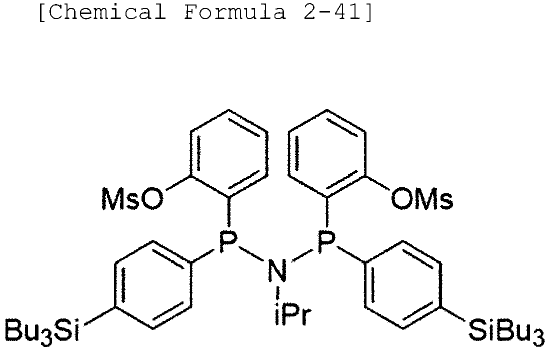

¹H NMR (500 MHz, CDCl₃) : δ 7.54(m, 2H), 7.42(m, 2H), 7.41(m, 4H), 7.32 (m, 2H), 7.23(m, 2H), 7.17(m, 4H), 3.61(m, 6H), 2.91(m, 1H), 1.49(m, 12H), 1.39(m, 12H), 1.30(m, 12H), 1.11(m, 6H), 0.90(m, 18H)

### Synthesis Example 42: Synthesis of ligand compound represented by Chemical Formula 2-42

0.54 g of isopropyl amine, 1.26 g of triethylamine, and 10 ml of dichloromethane were charged into a well-dried flask in a nitrogen atmosphere, and then stirring was started with cooling to 0°C. 4.91 g of chloro(4-(tributylsilyl)phenyl)(2-(trimethylsilyl)phenyl)phosphane was diluted in 10 ml of dichloromethane and then slowly charged into the flask. After stirring the reactant for 12 hours, the conversion rate was checked through NMR. After the reaction was completed, the precipitate was removed by filtration, and the solvent was removed by reducing the pressure. The obtained organic material was purified using column chromatography to obtain a target product, 3.39 g (yield: 70%) of N-isopropyl-1-(4-(tributylsilyl)phenyl)-N-((4-(tributylsilyl)phenyl)(2-(trimethylsilyl)phenyl)phosphaneyl)-1-(2-(trimethylsilyl)phenyl)phosphanamine.

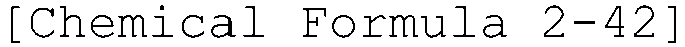

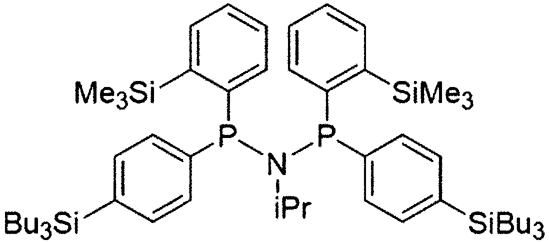

¹H NMR (500 MHz, CDCl₃) : δ 7.43(m, 8H), 7.32(m, 2H), 7.20(m, 6H), 2.88(m, 1H), 1.44(m, 12H), 1.35(m, 12H), 1.24 (m, 12H), 1.12 (m, 6H), 0.93(m, 18H), 0.27 (m, 18H)

### Synthesis Example 43: Synthesis of ligand compound represented by Chemical Formula 2-43

0.54 g of isopropyl amine, 1.26 g of triethylamine, and 10 ml of dichloromethane were charged into a well-dried flask in a nitrogen atmosphere, and then stirring was started with cooling to 0°C. 4.59 g of benzofuran-7-ylchloro(4-(tributylsilyl)phenyl)phosphane was diluted in 10 ml of dichloromethane and then slowly charged into the flask. After stirring the reactant for 20 hours, the conversion rate was checked through NMR. After the reaction was completed, the precipitate was removed by filtration, and the solvent was removed by reducing the pressure. The obtained organic material was purified using column chromatography to obtain a target product, 3.35 g (yield: 74%) of 1-(benzofuran-7-yl)-N-(benzofuran-7-yl(4-(tributylsilyl)phenyl)phosphaneyl)-N-isopropyl-1-(4-(tributylsilyl)phenyl)phosphanamine.

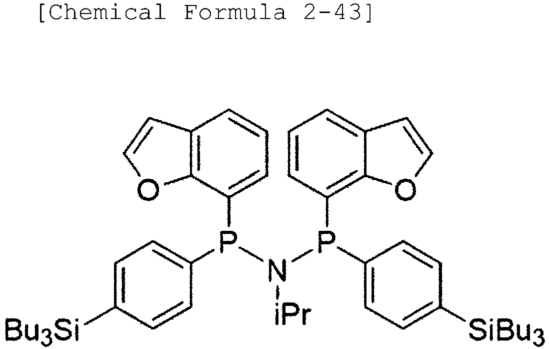

¹H NMR (500 MHz, CDCl₃): δ 7.61(m, 2H), 7.60(m, 2H), 7.50 (m, 2H), 7.42(m, 4H), 7.40(m, 2H), 7.20 (m, 4H), 6.81(m, 2H), 2.88 (m, 1H), 1.46(m, 12H), 1.30 (m, 24H), 1.10 (m, 6H), 0.97 (m, 18H)

### Synthesis Example 44: Synthesis of ligand compound represented by Chemical Formula 2-44

0.54 g of isopropyl amine, 1.26 g of triethylamine, and 10 ml of dichloromethane were charged into a well-dried flask in a nitrogen atmosphere, and then stirring was started with cooling to 0°C. 5.09 g of chloro(dibenzo[b,d]furan-4-yl) (4-(tributylsilyl)phenyl)phosphane was diluted in 10 ml of dichloromethane and then slowly charged into the flask. After stirring the reactant for 21 hour, the conversion rate was checked through NMR. After the reaction was completed, the precipitate was removed by filtration, and the solvent was removed by reducing the pressure. The obtained organic material was purified using column chromatography to obtain a target product, 3.16 g (yield: 63%) of 1-(dibenzo[b,d]furan-4-yl)-N-(dibenzo[b,d]furan-4-yl(4-(tributylsilyl)phenyl)phosphaneyl)-N-isopropyl-1-(4-(tributylsilyl)phenyl)phosphanamine.

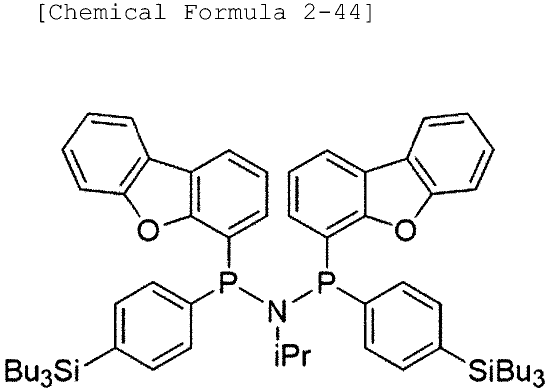

¹H NMR (500 MHz, CDCl₃): δ 8. 02 (m, 4H), 7.60(m, 2H), 7.52(m, 2H), 7.42(m, 4H), 7.41(m, 2H), 7.37(m, 2H), 7.32(m, 2H), 7.18(m, 4H), 2.89(m, 1H), 1.49(m, 12H), 1.34 (m, 12H), 1.32 (m, 12H), 1.11(m, 6H), 0.93(m, 18H)

### Synthesis Example 45: Synthesis of ligand compound represented by Chemical Formula 2-45

0.5 g of cyclohexylamine, 1.26 g of triethylamine, and 10 ml of dichloromethane were charged into a well-dried flask in a nitrogen atmosphere, and then stirring was started with cooling to 0°C. 4.37 g of chloro(2-fluorophenyl) (4-(tributylsilyl)phenyl)phosphane was diluted in 10 ml of dichloromethane and then slowly charged into the flask. After stirring the reactant for 18 hours, the conversion rate was checked through NMR. After the reaction was completed, the precipitate was removed by filtration, and the solvent was removed by reducing the pressure. The obtained organic material was purified using column chromatography to obtain a target product, 3.42 g (yield: 76%) of N-cyclohexyl-1-(2-fluorophenyl)-N-((2-fluorophenyl)(4-(tributylsilyl)phenyl)phosphaneyl)-1-(4-(tributylsilyl)phenyl)phosphanamine.

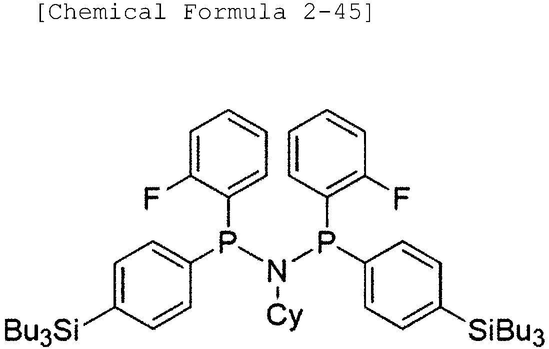

¹H NMR (500 MHz, CDCl₃): δ 7.65(m, 2H), 7.47(m, 4H), 7.29(m, 2H), 7.22(m, 2H), 7.19(m, 6H), 2.61(m, 1H), 1.78 (m, 2H), 1.53(m, 2H), 1.52 (m, 1H), 1.45(m, 13H), 1.38 (m, 12H), 1.29(m, 12H), 1.28(m, 2H), 1.15(m, 2H), 0.96(m, 18H)

### Synthesis Example 46: Synthesis of ligand compound represented by Chemical Formula 2-46

0.5 g of cyclohexylamine, 1.26 g of triethylamine, and 10 ml of dichloromethane were charged into a well-dried flask in a nitrogen atmosphere, and then stirring was started with cooling to 0°C. 4.87 g of chloro(4-(tributylsilyl)phenyl) (2-(trifluoromethyl)phenyl)phosphane was diluted in 10 ml of dichloromethane and then slowly charged into the flask. After stirring the reactant for 11 hour, the conversion rate was checked through NMR. After the reaction was completed, the precipitate was removed by filtration, and the solvent was removed by reducing the pressure. The obtained organic material was purified using column chromatography to obtain a target product, 4.7 g (yield: 94%) of N-cyclohexyl-1-(4-(tributylsilyl) phenyl) -N- ((4- (tributylsilyl)phenyl) (2-(trifluoromethyl)phenyl)phosphaneyl)-1-(2-(trifluoromethyl)phenyl)phosphanamine.

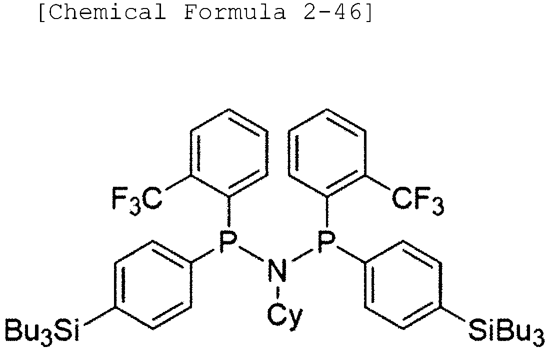

¹H NMR (500 MHz, CDCl₃): δ 7.51(m, 2H), 7.50(m, 2H), 7.44 (m, 4H), 7.32 (m, 2H), 7.14 (m, 4H), 7.08 (m, 2H), 2.62 (m, 1H), 1.84(m, 2H), 1.53(m, 1H), 1.50(m, 14H), 1.46(m, 1H), 1.38(m, 12H), 1.26(m, 2H), 1.24(m, 12H), 1.14(m, 2H), 0.90(m, 18H)

### Synthesis Example 47: Synthesis of ligand compound represented by Chemical Formula 2-47

0.5 g of cyclohexylamine, 1.26 g of triethylamine, and 10 ml of dichloromethane were charged into a well-dried flask in a nitrogen atmosphere, and then stirring was started with cooling to 0°C. 4.49 g of chloro(2-methoxyphenyl) (4-(tributylsilyl)phenyl)phosphane was diluted in 10 ml of dichloromethane and then slowly charged into the flask. After stirring the reactant for 15 hours, the conversion rate was checked through NMR. After the reaction was completed, the precipitate was removed by filtration, and the solvent was removed by reducing the pressure. The obtained organic material was purified using column chromatography to obtain a target product, 3.7 g (yield: 80%) of N-cyclohexyl-1-(2-methoxyphenyl)-N-((2-methoxyphenyl)(4-(tributylsilyl)phenyl)phosphaneyl)-1-(4-(tributylsilyl)phenyl)phosphanamine.

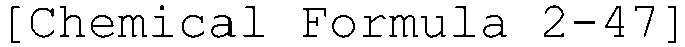

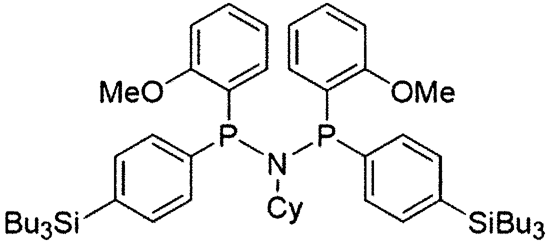

¹H NMR (500 MHz, CDCl₃): δ 7.50(m, 4H), 7.48(m, 2H), 7.23(m, 4H), 7.16(m, 2H), 7.11(m, 2H), 7.04(m, 2H), 3.90(m, 6H), 2.65(m, 1H), 1.79(m, 2H), 1.52 (m, 15H), 1.51(m, 1H), 1.31(m, 12H), 1.27(m, 12H), 1.23(m, 2H), 1.19(m, 2H), 0.92(m, 18H)

### Synthesis Example 48: Synthesis of ligand compound represented by Chemical Formula 2-48

0.5 g of cyclohexylamine, 1.26 g of triethylamine, and 10 ml of dichloromethane were charged into a well-dried flask in a nitrogen atmosphere, and then stirring was started with cooling to 0°C. 4.33 g of chloro (o-tolyl) (4-(tributylsilyl)phenyl)phosphane was diluted in 10 ml of dichloromethane and then slowly charged into the flask. After stirring the reactant for 13 hours, the conversion rate was checked through NMR. After the reaction was completed, the precipitate was removed by filtration, and the solvent was removed by reducing the pressure. The obtained organic material was purified using column chromatography to obtain a target product, 3.57 g (yield: 80%) of N-cyclohexyl-1-(o-tolyl)-N-(o-tolyl(4-(tributylsilyl)phenyl)phosphaneyl)-1-(4-(tributylsilyl)phenyl)phosphanamine.

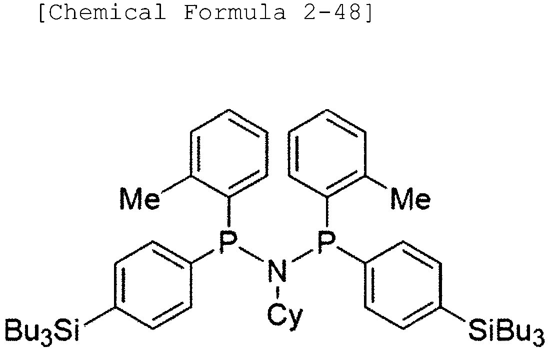

¹H NMR (500 MHz, CDCl₃): δ 7.45 (m, 4H), 7.39 (m, 2H), 7.33 (m, 2H), 7.18 (m, 4H), 7.16 (m, 2H), 7.12 (m, 2H), 2.58 (m, 1H), 2.36 (m, 6H), 1.79(m, 2H), 1.57(m, 2H), 1.54(m, 1H), 1.48(m, 12H), 1.44(m, 1H), 1.34(m, 12H), 1.29(m, 12H), 1.24(m, 2H), 1.17 (m, 2H), 0.90(m, 18H)

### Synthesis Example 49: Synthesis of ligand compound represented by Chemical Formula 2-49

0.5 g of cyclohexylamine, 1.26 g of triethylamine, and 10 ml of dichloromethane were charged into a well-dried flask in a nitrogen atmosphere, and then stirring was started with cooling to 0°C. 5.03 g of chloro(4-(tributylsilyl)phenyl)(2-(trifluoromethoxy)phenyl)phosphane was diluted in 10 ml of dichloromethane and then slowly charged into the flask. After stirring the reactant for 15 hours, the conversion rate was checked through NMR. After the reaction was completed, the precipitate was removed by filtration, and the solvent was removed by reducing the pressure. The obtained organic material was purified using column chromatography to obtain a target product, 3.92 g (yield: 76%) of N-cyclohexyl-1-(4-(tributylsilyl)phenyl)-N-((4-(tributylsilyl)phenyl)(2-(trifluoromethoxy)phenyl)phosphaneyl)-1-(2-(trifluoromethoxy)phenyl)phosphanamine.

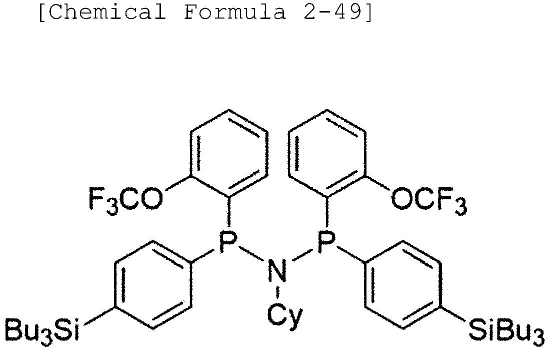

¹H NMR (500 MHz, CDCl₃): δ 7.49(m, 4H), 7.48 (m, 2H), 7.18 (m, 2H), 7.16 (m, 2H), 7.13 (m, 4H), 6.97(m, 2H), 2.60(m, 1H), 1.82(m, 2H), 1.57 (m, 2H), 1.47(m, 1H), 1.45(m, 1H), 1.44(m, 12H), 1.38 (m, 12H), 1.30 (m, 14H), 1.16 (m, 2H), 0.94 (m, 18H)

### Synthesis Example 50: Synthesis of ligand compound represented by Chemical Formula 2-50

0.5 g of cyclohexylamine, 1.26 g of triethylamine, and 10 ml of dichloromethane were charged into a well-dried flask in a nitrogen atmosphere, and then stirring was started with cooling to 0°C. 5.07 g of chloro(4-(tributylsilyl)phenyl)(2-(trifluoro-l4-sulfaneyl)phenyl)phosphane was diluted in 10 ml of dichloromethane and then slowly charged into the flask. After stirring the reactant for 17 hours, the conversion rate was checked through NMR. After the reaction was completed, the precipitate was removed by filtration, and the solvent was removed by reducing the pressure. The obtained organic material was purified using column chromatography to obtain a target product, 4.94 g (yield: 95%) of N-cyclohexyl-1-(4-(tributylsilyl)phenyl)-N-((4-(tributylsilyl)phenyl) (2-(trifluoro-l4-sulfaneyl)phenyl)phosphaneyl)-1-(2-(trifluoro-l4-sulfaneyl)phenyl)phosphanamine.

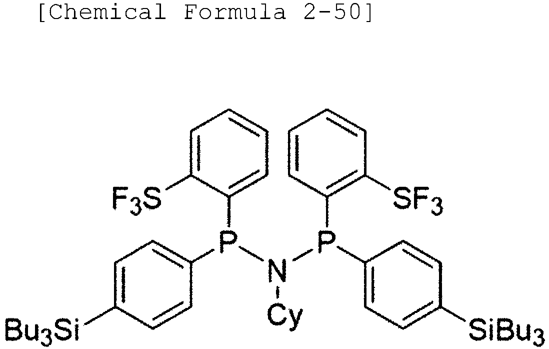

¹H NMR (500 MHz, CDCl₃): δ 7.45 (m, 4H), 7.42 (m, 2H), 7.31(m, 2H), 7.27(m, 2H), 7.17 (m, 4H), 6.99(m, 2H), 2.65(m, 1H), 1.77(m, 2H), 1.56(m, 2H), 1.49(m, 13H), 1.48(m, 1H), 1.31(m, 12H), 1.30(m, 2H), 1.25(m, 12H), 1.12(m, 2H), 0.96 (m, 18H)

### Synthesis Example 51: Synthesis of ligand compound represented by Chemical Formula 2-51

0.5 g of cyclohexylamine, 1.26 g of triethylamine, and 10 ml of dichloromethane were charged into a well-dried flask in a nitrogen atmosphere, and then stirring was started with cooling to 0°C. 4.65 g of chloro(2-(methylthio)phenyl)(4-(tributylsilyl)phenyl)phosphanewas diluted in 10 ml of dichloromethane and then slowly charged into the flask. After stirring the reactant for 14 hours, the conversion rate was checked through NMR. After the reaction was completed, the precipitate was removed by filtration, and the solvent was removed by reducing the pressure. The obtained organic material was purified using column chromatography to obtain a target product, 3.78 g (yield: 79%) of N-cyclohexyl-1-(2-(methylthio)phenyl)-N-((2-(methylthio)phenyl)(4-(tributylsilyl)phenyl)phosphaneyl)-1-(4-(tributylsilyl)phenyl)phosphanamine.

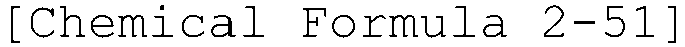

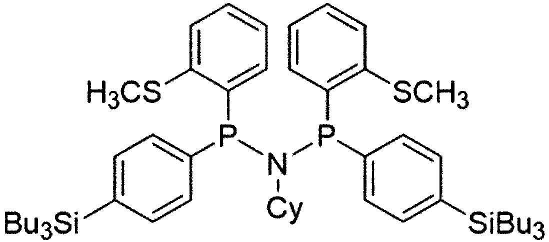

¹H NMR (500 MHz, CDCl₃): δ 7.42(m, 2H), 7. 41(m, 4H), 7.39(m, 2H), 7.25(m, 2H), 7.14(m, 2H), 7.13(m, 4H), 2.64(m, 1H), 2.53 (m, 6H), 1.74 (m, 2H), 1.53 (m, 2H), 1.50 (m, 1H), 1.47 (m, 12H), 1.46 (m, 1H), 1.35(m, 12H), 1.28 (m, 12H), 1.24 (m, 2H), 1.11(m, 2H), 0.96(m, 18H)

### Synthesis Example 52: Synthesis of ligand compound represented by Chemical Formula 2-52

0.5 g of cyclohexylamine, 1.26 g of triethylamine, and 10 ml of dichloromethane were charged into a well-dried flask in a nitrogen atmosphere, and then stirring was started with cooling to 0°C. 5.13 g of 2-(chloro(4-(tributylsilyl)phenyl)phosphaneyl)phenyl methanesulfonate was diluted in 10 ml of dichloromethane and then slowly charged into the flask. After stirring the reactant for 16 hours, the conversion rate was checked through NMR. After the reaction was completed, the precipitate was removed by filtration, and the solvent was removed by reducing the pressure. The obtained organic material was purified using column chromatography to obtain a target product, 3.95 g (yield: 75%) of ((cyclohexylazanediyl)bis((4-(tributylsilyl)phenyl)phosphanediyl))bis(2,1-phenylene) dimethanesulfonate.

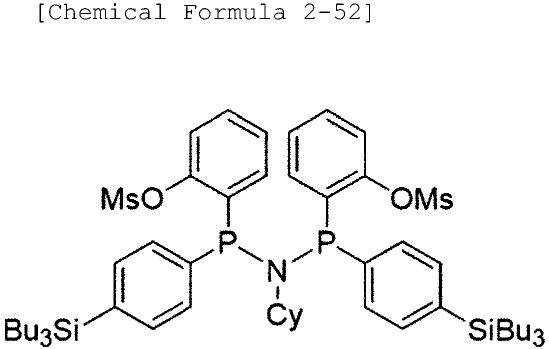

¹H NMR (500 MHz, CDCl₃): δ 7.51(m, 2H), 7.43(m, 6H), 7.39(m, 2H), 7.28(m, 2H), 7.17(m, 4H), 3.58(m, 6H), 2.66(m, 1H), 1.83(m, 2H), 1.56(m, 1H), 1.52(m, 2H), 1.47(m, 12H), 1.44(m, 1H), 1.35(m, 12H), 1.27(m, 12H), 1.23(m, 2H), 1.21(m, 2H), 0.89(m, 18H)

### Synthesis Example 53: Synthesis of ligand compound represented by Chemical Formula 2-53

0.5 g of cyclohexylamine, 1.26 g of triethylamine, and 10 ml of dichloromethane were charged into a well-dried flask in a nitrogen atmosphere, and then stirring was started with cooling to 0°C. 4.91 g of chloro(4-(tributylsilyl)phenyl) (2-(trimethylsilyl)phenyl)phosphane was diluted in 10 ml of dichloromethane and then slowly charged into the flask. After stirring the reactant for 23 hours, the conversion rate was checked through NMR. After the reaction was completed, the precipitate was removed by filtration, and the solvent was removed by reducing the pressure. The obtained organic material was purified using column chromatography to obtain a target product, 4.34 g (yield: 86%) of N-cyclohexyl-1-(4-(tributylsilyl)phenyl)-N-((4-(tributylsilyl)phenyl)(2-(trimethylsilyl)phenyl)phosphaneyl)-1-(2-(trimethylsilyl)phenyl)phosphanamine.

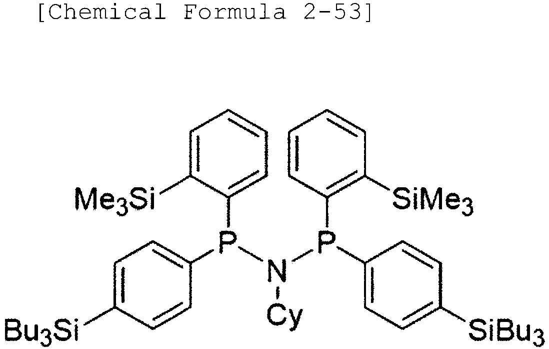

¹H NMR (500 MHz, CDCl₃): δ 7.42(m, 8H), 7.33 (m, 2H), 7.15(m, 6H), 2.66(m, 1H), 1.74(m, 2H), 1.53(m, 14H), 1.52(m, 1H), 1.48(m, 1H), 1.31(m, 12H), 1.30 (m, 2H), 1.25(m, 12H), 1.18 (m, 2H), 0.97(m, 18H), 0.33 (m, 18H)

### Synthesis Example 54: Synthesis of ligand compound represented by Chemical Formula 2-54

0.5 g of cyclohexylamine, 1.26 g of triethylamine, and 10 ml of dichloromethane were charged into a well-dried flask in a nitrogen atmosphere, and then stirring was started with cooling to 0°C. 4.59 g of benzofuran-7-ylchloro(4-(tributylsilyl)phenyl)phosphane was diluted in 10 ml of dichloromethane and then slowly charged into the flask. After stirring the reactant for 17 hours, the conversion rate was checked through NMR. After the reaction was completed, the precipitate was removed by filtration, and the solvent was removed by reducing the pressure. The obtained organic material was purified using column chromatography to obtain a target product, 4.3 g (yield: 91%) of 1-(benzofuran-7-yl)-N-(benzofuran-7-yl(4-(tributylsilyl)phenyl)phosphaneyl)-N-cyclohexyl-1-(4-(tributylsilyl)phenyl)phosphanamine.

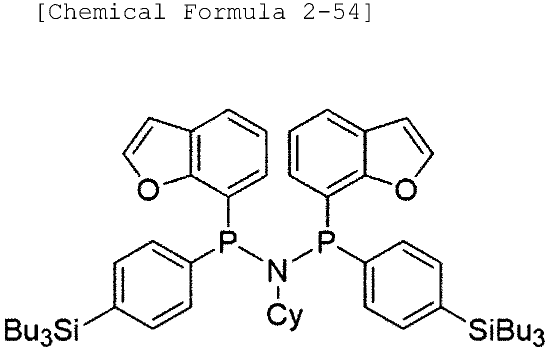

¹H NMR (500 MHz, CDCl₃): δ 7.68(m, 2H), 7.63(m, 2H), 7.50(m, 2H), 7.48(m, 4H), 7.38(m, 2H), 7.15(m, 4H), 6.85 (m, 2H), 2.62(m, 1H), 1.80(m, 2H), 1.57(m, 2H), 1.55(m, 1H), 1.52(m, 1H), 1.46(m, 12H), 1.35(m, 12H), 1.32(m, 12H), 1.23(m, 2H), 1.12 (m, 2H), 0.94 (m, 18H)

### Synthesis Example 55: Synthesis of ligand compound represented by Chemical Formula 2-55

0.5 g of cyclohexylamine, 1.26 g of triethylamine, and 10 ml of dichloromethane were charged into a well-dried flask in a nitrogen atmosphere, and then stirring was started with cooling to 0°C. 5.09 g of chloro(dibenzo[b,d]furan-4-yl)(4-(tributylsilyl)phenyl)phosphane was diluted in 10 ml of dichloromethane and then slowly charged into the flask. After stirring the reactant for 19 hours, the conversion rate was checked through NMR. After the reaction was completed, the precipitate was removed by filtration, and the solvent was removed by reducing the pressure. The obtained organic material was purified using column chromatography to obtain a target product, 4.75 g (yield: 91%) of N-cyclohexyl-1-(dibenzo[b,d]furan-4-yl)-N-(dibenzo[b,d]furan-4-yl(4-(tributylsilyl)phenyl)phosphaneyl)-1-(4-(tributylsilyl)phenyl)phosphanamine.

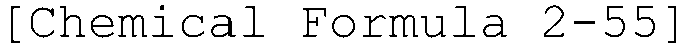

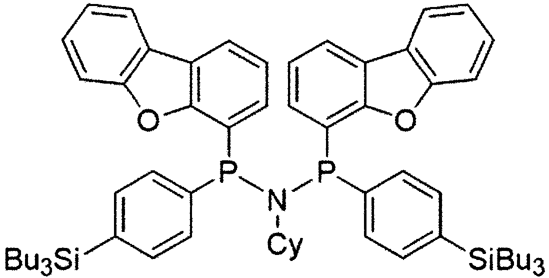

¹H NMR (500 MHz, CDCl₃): δ 8.05(m, 4H), 7.55(m, 2H), 7.47 (m, 2H), 7.46(m, 2H), 7.44(m, 4H), 7.35 (m, 2H), 7.34 (m, 2H), 7.20 (m, 4H), 2.58 (m, 1H), 1.79 (m, 2H), 1.50(m, 3H), 1.49(m, 1H), 1.47(m, 12H), 1.36 (m, 12H), 1.29 (m, 12H), 1.24 (m, 2H), 1.12 (m, 2H), 0.93 (m, 18H)

### Synthesis Example 56: Synthesis of ligand compound represented by Chemical Formula 2-56

0.47 g of aniline, 1.26 g of triethylamine, and 10 ml of dichloromethane were charged into a well-dried flask in a nitrogen atmosphere, and then stirring was started with cooling to 0°C. 4.37 g of chloro(2-fluorophenyl) (4-(tributylsilyl)phenyl)phosphane was diluted in 10 ml of dichloromethane and then slowly charged into the flask. After stirring the reactant for 15 hours, the conversion rate was checked through NMR. After the reaction was completed, the precipitate was removed by filtration, and the solvent was removed by reducing the pressure. The obtained organic material was purified using column chromatography to obtain a target product, 3.53 g (yield: 79%) of 1-(2-fluorophenyl)-N-((2-fluorophenyl) (4-(tributylsilyl)phenyl)phosphaneyl)-N-phenyl-1-(4-(tributylsilyl)phenyl)phosphanamine.

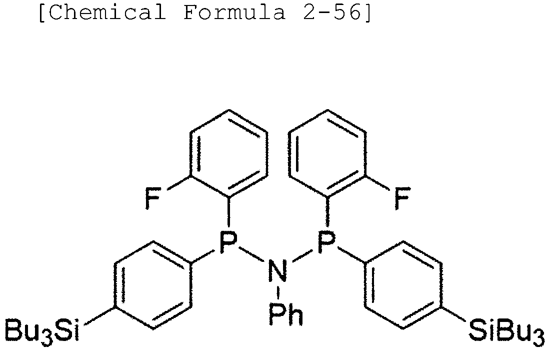

¹H NMR (500 MHz, CDCl₃): δ 7.69(m, 2H), 7.43(m, 4H), 7.23 (m, 10H), 7.18(m, 2H), 6.88(m, 2H), 6.83(m, 1H), 1.49(m, 12H), 1.33 (m, 12H), 1.24 (m, 12H), 0.98(m, 18H)

### Synthesis Example 57: Synthesis of ligand compound represented by Chemical Formula 2-57

0.47 g of aniline, 1.26 g of triethylamine, and 10 ml of dichloromethane were charged into a well-dried flask in a nitrogen atmosphere, and then stirring was started with cooling to 0°C. 4.87 g of chloro(4-(tributylsilyl)phenyl) (2-(trifluoromethyl)phenyl)phosphane was diluted in 10 ml of dichloromethane and then slowly charged into the flask. After stirring the reactant for 23 hours, the conversion rate was checked through NMR. After the reaction was completed, the precipitate was removed by filtration, and the solvent was removed by reducing the pressure. The obtained organic material was purified using column chromatography to obtain a target product, 3.88 g (yield: 78%) of N-phenyl-1-(4-(tributylsilyl)phenyl)-N-((4-(tributylsilyl)phenyl)(2-(trifluoromethyl)phenyl)phosphaneyl)-1-(2-(trifluoromethyl)phenyl)phosphanamine.

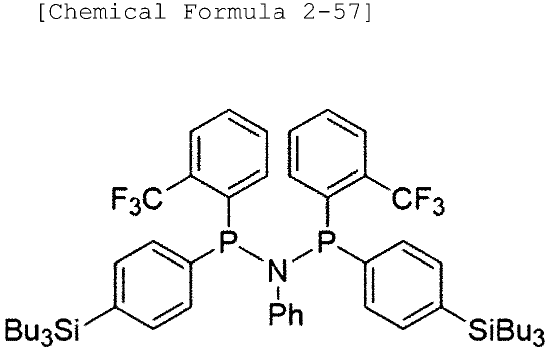

¹H NMR (500 MHz, CDCl₃): δ 7.48(m, 2H), 7.47(m, 6H), 7.28 (m, 2H), 7.22 (m, 4H), 7.18 (m, 2H), 7.08 (m, 2H), 6.85 (m, 3H), 1.48 (m, 12H), 1.30 (m, 12H), 1.24 (m, 12H), 0.90(m, 18H)

### Synthesis Example 58: Synthesis of ligand compound represented by Chemical Formula 2-58

0.47 g of aniline, 1.26 g of triethylamine, and 10 ml of dichloromethane were charged into a well-dried flask in a nitrogen atmosphere, and then stirring was started with cooling to 0°C. 4.49 g of chloro(2-methoxyphenyl)(4-(tributylsilyl)phenyl)phosphane was diluted in 10 ml of dichloromethane and then slowly charged into the flask. After stirring the reactant for 15 hours, the conversion rate was checked through NMR. After the reaction was completed, the precipitate was removed by filtration, and the solvent was removed by reducing the pressure. The obtained organic material was purified using column chromatography to obtain a target product, 3.54 g (yield: 77%) of 1-(2-methoxyphenyl)-N-((2-methoxyphenyl) (4-(tributylsilyl)phenyl)phosphaneyl)-N-phenyl-1-(4-(tributylsilyl)phenyl)phosphanamine.

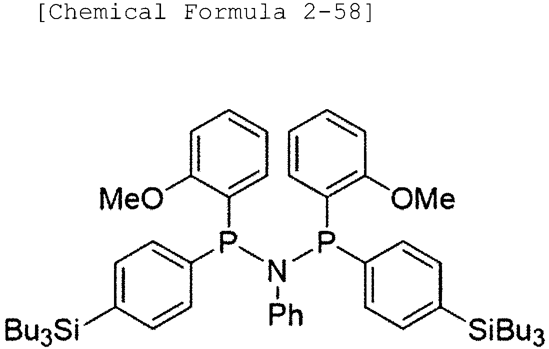

¹H NMR (500 MHz, CDCl₃): δ 7.46(m, 4H), 7.43 (m, 2H), 7.28 (m, 2H), 7.19 (m, 2H), 7.18 (m, 2H), 7.13 (m, 4H), 6.97(m, 2H), 6.86(m, 1H), 6.81(m, 2H), 3.86(m, 6H), 1.45(m, 12H), 1.32 (m, 12H), 1.29 (m, 12H), 0.91(m, 18H)

### Synthesis Example 59: Synthesis of ligand compound represented by Chemical Formula 2-59

0.47 g of aniline, 1.26 g of triethylamine, and 10 ml of dichloromethane were charged into a well-dried flask in a nitrogen atmosphere, and then stirring was started with cooling to 0°C. 4.33 g of chloro(o-tolyl) (4-(tributylsilyl)phenyl)phosphane was diluted in 10 ml of dichloromethane and then slowly charged into the flask. After stirring the reactant for 22 hours, the conversion rate was checked through NMR. After the reaction was completed, the precipitate was removed by filtration, and the solvent was removed by reducing the pressure. The obtained organic material was purified using column chromatography to obtain a target product, 3.24 g (yield: 73%) of (N-phenyl-1-(o-tolyl)-N-(o-tolyl(4-(tributylsilyl)phenyl)phosphaneyl)-1-(4-(tributylsilyl)phenyl)phosphanamine.

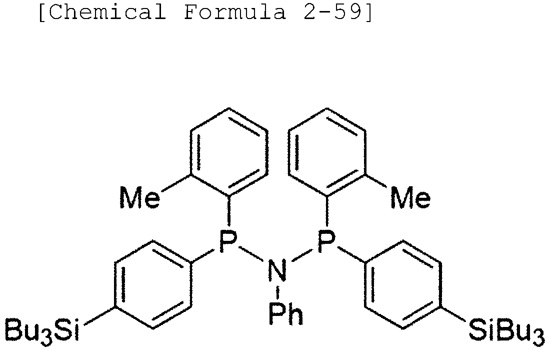

¹H NMR (500 MHz, CDCl₃): δ 7.48 (m, 4H), 7.41(m, 2H), 7.29(m, 2H), 7.26(m, 2H), 7.23(m, 4H), 7.20(m, 4H), 6.91(m, 1H), 6.85 (m, 2H), 2.43(m, 6H), 1.52 (m, 12H), 1.32 (m, 12H), 1.27(m, 12H), 0.93(m, 18H)

### Synthesis Example 60: Synthesis of ligand compound represented by Chemical Formula 2-60

0.47 g of aniline, 1.26 g of triethylamine, and 10 ml of dichloromethane were charged into a well-dried flask in a nitrogen atmosphere, and then stirring was started with cooling to 0°C. 5.03 g of chloro(4-(tributylsilyl)phenyl) (2-(trifluoromethoxy)phenyl)phosphane was diluted in 10 ml of dichloromethane and then slowly charged into the flask. After stirring the reactant for 18 hours, the conversion rate was checked through NMR. After the reaction was completed, the precipitate was removed by filtration, and the solvent was removed by reducing the pressure. The obtained organic material was purified using column chromatography to obtain a target product, 3.34 g (yield: 65%) of N-phenyl-1-(4-(tributylsilyl)phenyl)-N-((4-(tributylsilyl)phenyl)(2-(trifluoromethoxy)phenyl)phosphaneyl)-1-(2-(trifluoromethoxy)phenyl)phosphanamine.

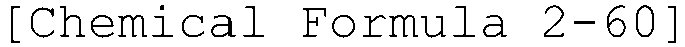

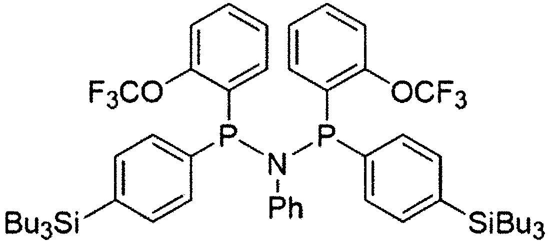

¹H NMR (500 MHz, CDCl₃): δ 7.44(m, 2H), 7.43(m, 4H), 7.25 (m, 2H), 7.23 (m, 2H), 7.17 (m, 4H), 7.15 (m, 2H), 7.05 (m, 2H), 6.83(m, 1H), 6.81(m, 2H), 1.49(m, 12H), 1.38 (m, 12H), 1.30(m, 12H), 0.93(m, 18H)

### Synthesis Example 61: Synthesis of ligand compound represented by Chemical Formula 2-61

0.47 g of aniline, 1.26 g of triethylamine, and 10 ml of dichloromethane were charged into a well-dried flask in a nitrogen atmosphere, and then stirring was started with cooling to 0°C. 5.07 g of chloro(4-(tributylsilyl)phenyl)(2-(trifluoro-l4-sulfaneyl)phenyl)phosphane was diluted in 10 ml of dichloromethane and then slowly charged into the flask. After stirring the reactant for 11 hour, the conversion rate was checked through NMR. After the reaction was completed, the precipitate was removed by filtration, and the solvent was removed by reducing the pressure. The obtained organic material was purified using column chromatography to obtain a target product, 3.83 g (yield: 74%) of N-phenyl-1-(4-(tributylsilyl)phenyl)-N-((4-(tributylsilyl)phenyl)(2-(trifluoro-l4-sulfaneyl)phenyl)phosphaneyl)-1-(2-(trifluoro-l4-sulfaneyl)phenyl)phosphanamine.

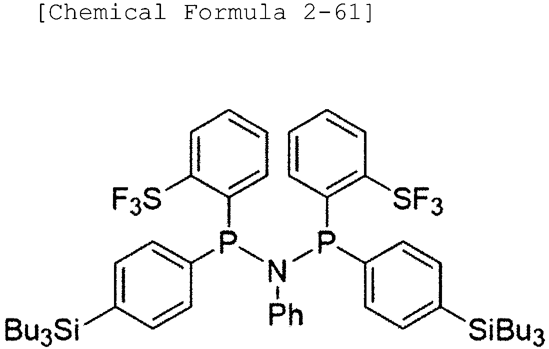

¹H NMR (500 MHz, CDCl₃): δ 7.49(m, 4H), 7.42(m, 2H), 7.36(m, 2H), 7.28(m, 2H), 7.20(m, 2H), 7.14 (m, 4H), 7.04 (m, 2H), 6.87 (m, 3H), 1.53(m, 12H), 1.32(m, 12H), 1.29(m, 12H), 0.93(m, 18H)

### Synthesis Example 62: Synthesis of ligand compound represented by Chemical Formula 2-62

0.47 g of aniline, 1.26 g of triethylamine, and 10 ml of dichloromethane were charged into a well-dried flask in a nitrogen atmosphere, and then stirring was started with cooling to 0°C. 4.65 g of chloro(2-(methylthio)phenyl) (4-(tributylsilyl)phenyl)phosphanewas diluted in 10 ml of dichloromethane and then slowly charged into the flask. After stirring the reactant for 10 hours, the conversion rate was checked through NMR. After the reaction was completed, the precipitate was removed by filtration, and the solvent was removed by reducing the pressure. The obtained organic material was purified using column chromatography to obtain a target product, 3.66 g (yield: 77%) of (1-(2-(methylthio)phenyl)-N-((2-(methylthio)phenyl)(4-(tributylsilyl)phenyl)phosphaneyl)-N-phenyl-1-(4-(tributylsilyl)phenyl)phosphanamine.

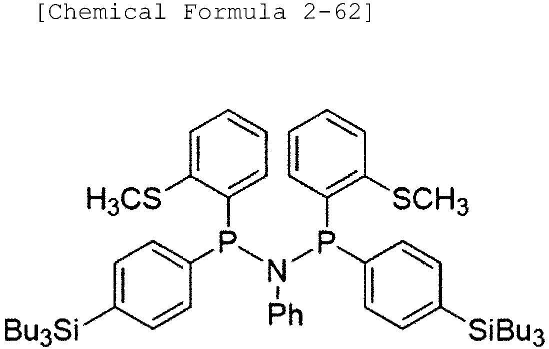

¹H NMR (500 MHz, CDCl₃): δ 7.45(m, 4H), 7.42 (m, 2H), 7.37 (m, 2H), 7.25 (m, 4H), 7.14 (m, 4H), 7.08 (m, 2H), 6.91(m, 1H), 6.81(m, 2H), 2.54(m, 6H), 1.51(m, 12H), 1.32 (m, 12H), 1.28(m, 12H), 0.90(m, 18H)

### Synthesis Example 63: Synthesis of ligand compound represented by Chemical Formula 2-63

0.47 g of aniline, 1.26 g of triethylamine, and 10 ml of dichloromethane were charged into a well-dried flask in a nitrogen atmosphere, and then stirring was started with cooling to 0°C. 5.13 g of 2-(chloro(4-(tributylsilyl)phenyl)phosphaneyl)phenyl methanesulfonate was diluted in 10 ml of dichloromethane and then slowly charged into the flask. After stirring the reactant for 18 hours, the conversion rate was checked through NMR. After the reaction was completed, the precipitate was removed by filtration, and the solvent was removed by reducing the pressure. The obtained organic material was purified using column chromatography to obtain a target product, 3.77 g (yield: 72%) of ((phenylazanediyl)bis((4-(tributylsilyl)phenyl)phosphanediyl))bis(2,1-phenylene) dimethanesulfonate.

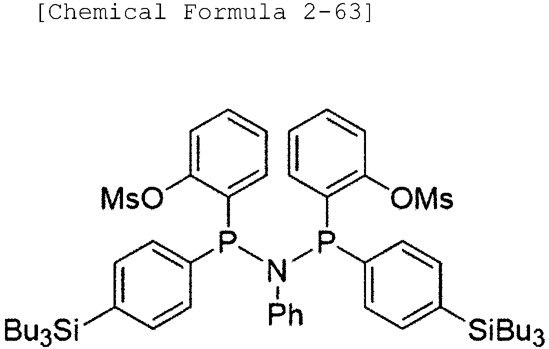

¹H NMR (500 MHz, CDCl₃): δ 7.49 (m, 2H), 7.47(m, 2H), 7.45(m, 4H), 7.35 (m, 2H), 7.31(m, 2H), 7.22 (m, 2H), 7.21(m, 4H), 6.90 (m, 2H), 6.83(m, 1H), 3.60(m, 6H), 1.53 (m, 12H), 1.37 (m, 12H), 1.31(m, 12H), 0.93(m, 18H)

### Synthesis Example 64: Synthesis of ligand compound represented by Chemical Formula 2-64

0.47 g of aniline, 1.26 g of triethylamine, and 10 ml of dichloromethane were charged into a well-dried flask in a nitrogen atmosphere, and then stirring was started with cooling to 0°C. 4.91 g of chloro(4-(tributylsilyl)phenyl) (2-(trimethylsilyl)phenyl)phosphane was diluted in 10 ml of dichloromethane and then slowly charged into the flask. After stirring the reactant for 24 hours, the conversion rate was checked through NMR. After the reaction was completed, the precipitate was removed by filtration, and the solvent was removed by reducing the pressure. The obtained organic material was purified using column chromatography to obtain a target product, 4.31 g (yield: 86%) of N-phenyl-1-(4-(tributylsilyl)phenyl)-N-((4-(tributylsilyl)phenyl)(2-(trimethylsilyl)phenyl)phosphaneyl)-1-(2-(trimethylsilyl)phenyl)phosphanamine.

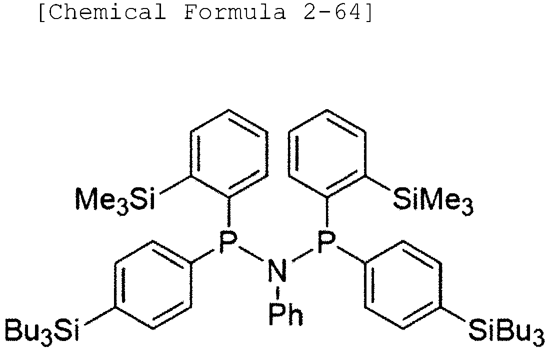

¹H NMR (500 MHz, CDCl₃): δ 7.45(m, 8H), 7.33(m, 2H), 7.27 (m, 2H), 7.23 (m, 6H), 6.85 (m, 1H), 6.82 (m, 2H), 1.50(m, 12H), 1.37 (m, 12H), 1.28 (m, 12H), 0.98(m, 18H), 0.28(m, 18H)

### Synthesis Example 65: Synthesis of ligand compound represented by Chemical Formula 2-65

0.47 g of aniline, 1.26 g of triethylamine, and 10 ml of dichloromethane were charged into a well-dried flask in a nitrogen atmosphere, and then stirring was started with cooling to 0°C. 4.59 g of benzofuran-7-ylchloro(4-(tributylsilyl)phenyl)phosphane was diluted in 10 ml of dichloromethane and then slowly charged into the flask. After stirring the reactant for 23 hours, the conversion rate was checked through NMR. After the reaction was completed, the precipitate was removed by filtration, and the solvent was removed by reducing the pressure. The obtained organic material was purified using column chromatography to obtain a target product, 3.57 g (yield: 76%) of 1-(benzofuran-7-yl)-N-(benzofuran-7-yl(4-(tributylsilyl)phenyl)phosphaneyl)-N-phenyl-1-(4-(tributylsilyl)phenyl)phosphanamine.

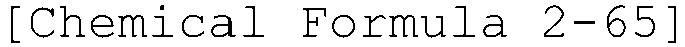

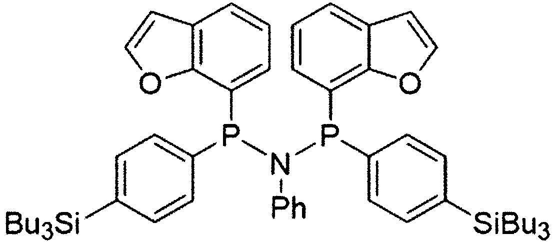

¹H NMR (500 MHz, CDCl₃): δ 7. 63 (m, 2H), 7.61(m, 2H), 7.46(m, 2H), 7.45(m, 4H), 7.42(m, 2H), 7.27(m, 2H), 7.16(m, 4H), 6.87(m, 1H), 6.86(m, 2H), 6.79(m, 2H), 1.51(m, 12H), 1.34 (m, 12H), 1.23(m, 12H), 0.97(m, 18H)

### Synthesis Example 66: Synthesis of ligand compound represented by Chemical Formula 2-66

0.47 g of aniline, 1.26 g of triethylamine, and 10 ml of dichloromethane were charged into a well-dried flask in a nitrogen atmosphere, and then stirring was started with cooling to 0°C. 5.09 g of chloro(dibenzo[b,d]furan-4-yl)(4-(tributylsilyl)phenyl)phosphane was diluted in 10 ml of dichloromethane and then slowly charged into the flask. After stirring the reactant for 20 hours, the conversion rate was checked through NMR. After the reaction was completed, the precipitate was removed by filtration, and the solvent was removed by reducing the pressure. The obtained organic material was purified using column chromatography to obtain a target product, 4.26 g (yield: 82%) of 1-(dibenzo[b,d]furan-4-yl)-N-(dibenzo[b,d]furan-4-yl(4-(tributylsilyl)phenyl)phosphaneyl)-N-phenyl-1-(4-(tributylsilyl)phenyl)phosphanamine.

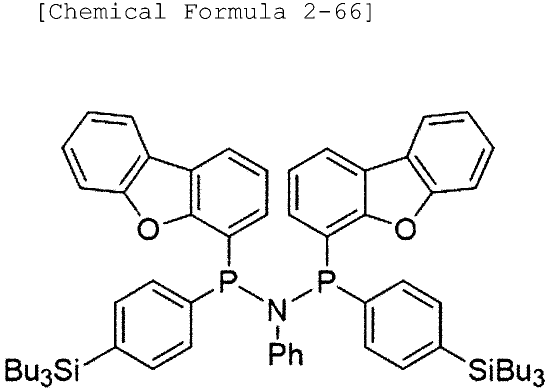

¹H NMR (500 MHz, CDCl₃): δ 8.06(m, 4H), 7.56(m, 2H), 7.44 (m, 4H), 7.41(m, 4H), 7.40(m, 2H), 7.36(m, 2H), 7.25 (m, 2H), 7.18 (m, 4H), 6.89 (m, 1H), 6.84 (m, 2H), 1.50 (m, 12H), 1.33(m, 12H), 1.29(m, 12H), 0.90(m, 18H)

### Synthesis Example 67: Synthesis of ligand compound represented by Chemical Formula 5-1

0.34 g of a methylamine HCl salt, 1.77 g of triethylamine, and 10 ml of dichloromethane were charged into a well-dried flask in a nitrogen atmosphere, and then stirring was started with cooling to 0°C. 4.37 g of chloro(2-fluorophenyl) (4-(tributylsilyl)phenyl)phosphane was diluted in 10 ml of dichloromethane and then slowly charged into the flask. After stirring the reactant for 14 hours, the conversion rate was checked through NMR. After the reaction was completed, the precipitate was removed by filtration, and the solvent was removed by reducing the pressure. The obtained organic material was purified using column chromatography to obtain a target product, 2.83 g (yield: 68%) of 1-(2-fluorophenyl)-N-((2-fluorophenyl)(3-(tributylsilyl)phenyl)phosphaneyl)-N-methyl-1-(3-(tributylsilyl)phenyl)phosphanamine.

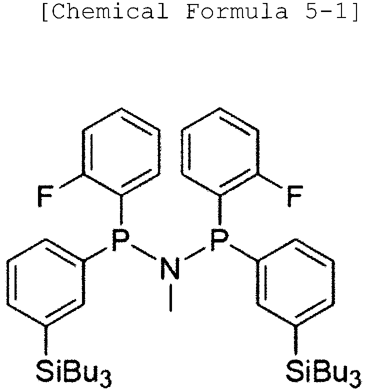

¹H NMR (500 MHz, CDCl₃): δ 7.65(m, 2H), 7. 64 (m, 2H), 7.44(m, 2H), 7.34 (m, 2H), 7.25(m, 2H), 7.24(m, 2H), 7.19(m, 4H), 2.57 (m, 3H), 1.53(m, 12H), 1.33 (m, 12H), 1.32(m, 12H), 0.98(m, 18H)

### Synthesis Example 68: Synthesis of ligand compound represented by Chemical Formula 5-2

0.34 g of a methylamine HCl salt, 1.77 g of triethylamine, and 10 ml of dichloromethane were charged into a well-dried flask in a nitrogen atmosphere, and then stirring was started with cooling to 0°C. 4.87 g of chloro(4-(tributylsilyl)phenyl) (2-(trifluoromethyl)phenyl)phosphane was diluted in 10 ml of dichloromethane and then slowly charged into the flask. After stirring the reactant for 22 hours, the conversion rate was checked through NMR. After the reaction was completed, the precipitate was removed by filtration, and the solvent was removed by reducing the pressure. The obtained organic material was purified using column chromatography to obtain a target product, 3.17 g (yield: 68%) of N-methyl-1-(3-(tributylsilyl)phenyl)-N-((3-(tributylsilyl)phenyl) (2-(trifluoromethyl)phenyl)phosphaneyl)-1-(2-(trifluoromethyl)phenyl)phosphanamine.

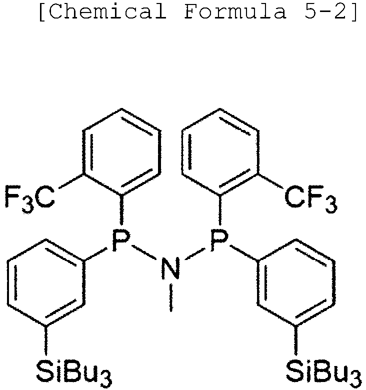

¹H NMR (500 MHz, CDCl₃): δ 7. 65 (m, 2H), 7.57(m, 2H), 7.49(m, 2H), 7.45(m, 2H), 7.37 (m, 2H), 7.29(m, 2H), 7.20 (m, 2H), 7.13 (m, 2H), 2.57 (m, 3H), 1.47(m, 12H), 1.35 (m, 12H), 1.33 (m, 12H), 0.91(m, 18H)

### Synthesis Example 69: Synthesis of ligand compound represented by Chemical Formula 5-3

0.34 g of a methylamine HCl salt, 1.77 g of triethylamine, and 10 ml of dichloromethane were charged into a well-dried flask in a nitrogen atmosphere, and then stirring was started with cooling to 0°C. 4.49 g of chloro(2-methoxyphenyl) (4-(tributylsilyl)phenyl)phosphane was diluted in 10 ml of dichloromethane and then slowly charged into the flask. After stirring the reactant for 16 hours, the conversion rate was checked through NMR. After the reaction was completed, the precipitate was removed by filtration, and the solvent was removed by reducing the pressure. The obtained organic material was purified using column chromatography to obtain a target product, 2.57 g (yield: 60%) of 1-(2-methoxyphenyl)-N-((2-methoxyphenyl)(3-(tributylsilyl)phenyl)phosphaneyl)-N-methyl-1-(3-(tributylsilyl)phenyl)phosphanamine.

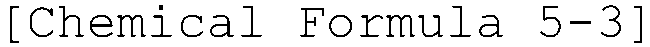

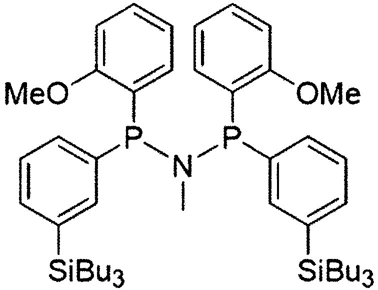

¹H NMR (500 MHz, CDCl₃): δ 7. 63 (m, 2H), 7.49(m, 4H), 7.41(m, 2H), 7.17 (m, 2H), 7.14(m, 2H), 7.13 (m, 2H), 6.98(m, 2H), 3.85(m, 6H), 2.48(m, 3H), 1.51(m, 12H), 1.33 (m, 12H), 1.29(m, 12H), 0.96(m, 18H)

### Synthesis Example 70: Synthesis of ligand compound represented by Chemical Formula 5-4

0.34 g of a methylamine HCl salt, 1.77 g of triethylamine, and 10 ml of dichloromethane were charged into a well-dried flask in a nitrogen atmosphere, and then stirring was started with cooling to 0°C. 4.33 g of chloro(o-tolyl) (4-(tributylsilyl)phenyl)phosphane was diluted in 10 ml of dichloromethane and then slowly charged into the flask. After stirring the reactant for 23 hours, the conversion rate was checked through NMR. After the reaction was completed, the precipitate was removed by filtration, and the solvent was removed by reducing the pressure. The obtained organic material was purified using column chromatography to obtain a target product, 3.96 g (yield: 96%) of N-methyl-1-(o-tolyl)-N-(o-tolyl(3-(tributylsilyl)phenyl)phosphaneyl)-1-(3-(tributylsilyl)phenyl)phosphanamine.

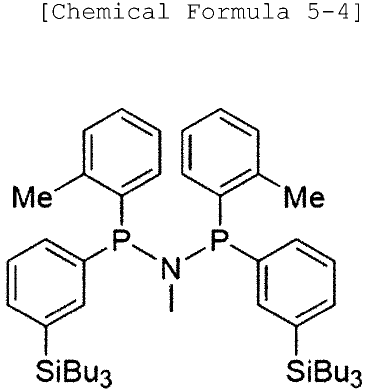

¹H NMR (500 MHz, CDCl₃): δ 7. 63 (m, 2H), 7.50(m, 2H), 7.40(m, 2H), 7.32 (m, 2H), 7.28(m, 2H), 7.19(m, 2H), 7.18(m, 2H), 7.14 (m, 2H), 2.51(m, 3H), 2.36 (m, 6H), 1.48(m, 12H), 1.35(m, 12H), 1.23(m, 12H), 0.99 (m, 18H)

### Synthesis Example 71: Synthesis of ligand compound represented by Chemical Formula 5-5

0.34 g of a methylamine HCl salt, 1.77 g of triethylamine, and 10 ml of dichloromethane were charged into a well-dried flask in a nitrogen atmosphere, and then stirring was started with cooling to 0°C. 5.03 g of chloro(4-(tributylsilyl)phenyl) (2-(trifluoromethoxy)phenyl)phosphane was diluted in 10 ml of dichloromethane and then slowly charged into the flask. After stirring the reactant for 17 hours, the conversion rate was checked through NMR. After the reaction was completed, the precipitate was removed by filtration, and the solvent was removed by reducing the pressure. The obtained organic material was purified using column chromatography to obtain a target product, 2.94 g (yield: 61%) of N-methyl-1-(3-(tributylsilyl)phenyl)-N-((3-(tributylsilyl)phenyl)(2-(trifluoromethoxy)phenyl)phosphaneyl)-1-(2-(trifluoromethoxy)phenyl)phosphanamine.

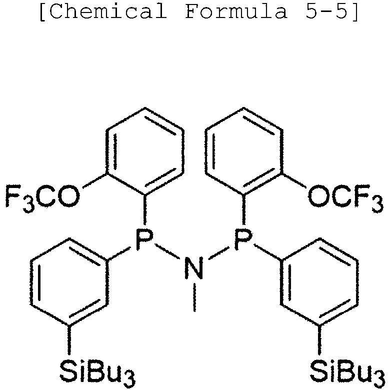

¹H NMR (500 MHz, CDCl₃): δ 7.57 (m, 2H), 7.43(m, 2H), 7.42 (m, 2H), 7.35 (m, 2H), 7.19 (m, 2H), 7.17 (m, 2H), 7.14 (m, 2H), 6.98(m, 2H), 2.57 (m, 3H), 1.50 (m, 12H), 1.37 (m, 12H), 1.24 (m, 12H), 0.90(m, 18H)

### Synthesis Example 72: Synthesis of ligand compound represented by Chemical Formula 5-6

0.37 g of butylamine, 1.26 g of triethylamine, and 10 ml of dichloromethane were charged into a well-dried flask in a nitrogen atmosphere, and then stirring was started with cooling to 0°C. 4.37 g of chloro(2-fluorophenyl) (4-(tributylsilyl)phenyl)phosphane was diluted in 10 ml of dichloromethane and then slowly charged into the flask. After stirring the reactant for 16 hours, the conversion rate was checked through NMR. After the reaction was completed, the precipitate was removed by filtration, and the solvent was removed by reducing the pressure. The obtained organic material was purified using column chromatography to obtain a target product, 3.02 g (yield: 69%) of N-butyl-1-(2-fluorophenyl)-N-((2-fluorophenyl)(3-(tributylsilyl)phenyl)phosphaneyl)-1-(3-(tributylsilyl)phenyl)phosphanamine.

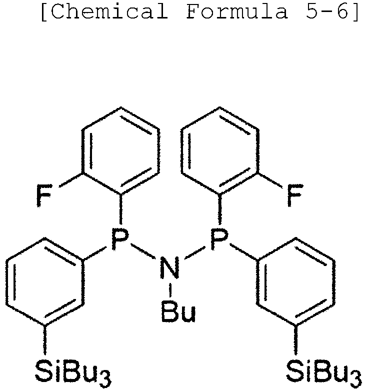

¹H NMR (500 MHz, CDCl₃): δ 7.71(m, 2H), 7.62(m, 2H), 7.44 (m, 2H), 7.34 (m, 2H), 7.29(m, 2H), 7.24(m, 2H), 7.17 (m, 4H), 2.65(m, 2H), 1.60(m, 2H), 1.44(m, 12H), 1.39 (m, 14H), 1.32 (m, 12H), 0.91(m, 21H)

### Synthesis Example 73: Synthesis of ligand compound represented by Chemical Formula 5-7

0.37 g of butylamine, 1.26 g of triethylamine, and 10 ml of dichloromethane were charged into a well-dried flask in a nitrogen atmosphere, and then stirring was started with cooling to 0°C. 4.87 g of chloro(4-(tributylsilyl)phenyl) (2-(trifluoromethyl)phenyl)phosphane was diluted in 10 ml of dichloromethane and then slowly charged into the flask. After stirring the reactant for 17 hours, the conversion rate was checked through NMR. After the reaction was completed, the precipitate was removed by filtration, and the solvent was removed by reducing the pressure. The obtained organic material was purified using column chromatography to obtain a target product, 4.09 g (yield: 84%) of N-butyl-1-(3-(tributylsilyl) phenyl) -N-((3-(tributylsilyl)phenyl) (2-(trifluoromethyl)phenyl)phosphaneyl)-1-(2-(trifluoromethyl)phenyl)phosphanamine.

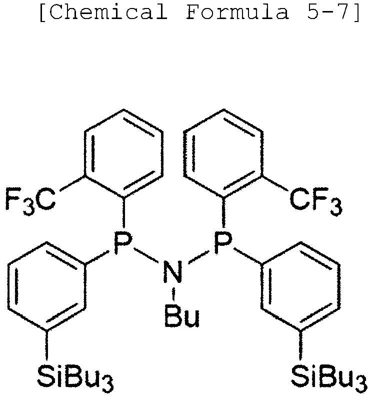

¹H NMR (500 MHz, CDCl₃): δ 7. 64 (m, 2H), 7.49(m, 2H), 7.47 (m, 2H), 7.43(m, 2H), 7.39 (m, 2H), 7.32 (m, 2H), 7.18 (m, 2H), 7.11(m, 2H), 2.65(m, 2H), 1.53(m, 2H), 1.45(m, 12H), 1.33 (m, 14H), 1.25 (m, 12H), 0.91(m, 21H)

### Synthesis Example 74: Synthesis of ligand compound represented by Chemical Formula 5-8

0.37 g of butylamine, 1.26 g of triethylamine, and 10 ml of dichloromethane were charged into a well-dried flask in a nitrogen atmosphere, and then stirring was started with cooling to 0°C. 4.49 g of chloro(2-methoxyphenyl) (4-(tributylsilyl)phenyl)phosphane was diluted in 10 ml of dichloromethane and then slowly charged into the flask. After stirring the reactant for 21 hour, the conversion rate was checked through NMR. After the reaction was completed, the precipitate was removed by filtration, and the solvent was removed by reducing the pressure. The obtained organic material was purified using column chromatography to obtain a target product, 3.68 g (yield: 82%) of N-butyl-1-(2-methoxyphenyl)-N-((2-methoxyphenyl)(3-(tributylsilyl)phenyl)phosphaneyl)-1-(3-(tributylsilyl)phenyl)phosphanamine.

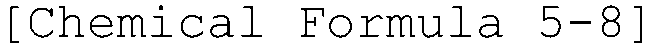

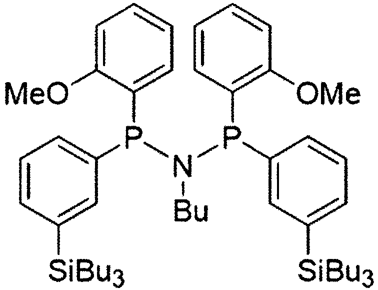

¹H NMR (500 MHz, CDCl₃): δ 7. 62 (m, 2H), 7.47(m, 2H), 7.43 (m, 4H), 7.20 (m, 2H), 7.16 (m, 2H), 7.13 (m, 2H), 6.98(m, 2H), 3.87 (m, 6H), 2.66(m, 2H), 1.54(m, 2H), 1.49(m, 12H), 1.31(m, 14H), 1.26(m, 12H), 0.89 (m, 21H)

### Synthesis Example 75: Synthesis of ligand compound represented by Chemical Formula 5-9

0.37 g of butylamine, 1.26 g of triethylamine, and 10 ml of dichloromethane were charged into a well-dried flask in a nitrogen atmosphere, and then stirring was started with cooling to 0°C. 4.33 g of chloro (o-tolyl) (4-(tributylsilyl)phenyl)phosphane was diluted in 10 ml of dichloromethane and then slowly charged into the flask. After stirring the reactant for 24 hours, the conversion rate was checked through NMR. After the reaction was completed, the precipitate was removed by filtration, and the solvent was removed by reducing the pressure. The obtained organic material was purified using column chromatography to obtain a target product, 2.69 g (yield: 62%) of N-butyl-1-(o-tolyl)-N-(o-tolyl(3-(tributylsilyl)phenyl)phosphaneyl)-1-(3-(tributylsilyl)phenyl)phosphanamine.

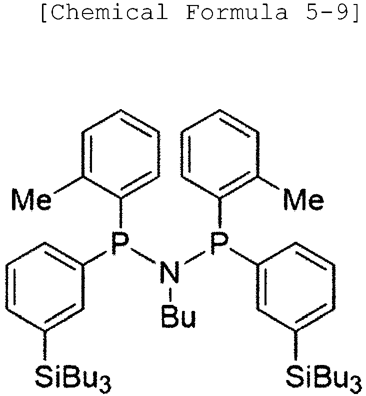

¹H NMR (500 MHz, CDCl₃): δ 7. 64 (m, 2H), 7.49(m, 2H), 7.42 (m, 2H), 7.37 (m, 2H), 7.28 (m, 2H), 7.23 (m, 2H), 7.15 (m, 2H), 7.13(m, 2H), 2.67(m, 2H), 2.37(m, 6H), 1.62 (m, 2H), 1.44 (m, 12H), 1.34 (m, 14H), 1.27 (m, 12H), 0.94(m, 21H)

### Synthesis Example 76: Synthesis of ligand compound represented by Chemical Formula 5-10

0.37 g of butylamine, 1.26 g of triethylamine, and 10 ml of dichloromethane were charged into a well-dried flask in a nitrogen atmosphere, and then stirring was started with cooling to 0°C. 5.03 g of chloro(4-(tributylsilyl)phenyl) (2-(trifluoromethoxy)phenyl)phosphane was diluted in 10 ml of dichloromethane and then slowly charged into the flask. After stirring the reactant for 23 hours, the conversion rate was checked through NMR. After the reaction was completed, the precipitate was removed by filtration, and the solvent was removed by reducing the pressure. The obtained organic material was purified using column chromatography to obtain a target product, 4.83 g (yield: 96%) of N-butyl-1-(3-(tributylsilyl)phenyl)-N-((3-(tributylsilyl)phenyl)(2-(trifluoromethoxy)phenyl)phosphaneyl)-1-(2-(trifluoromethoxy)phenyl)phosphanamine.

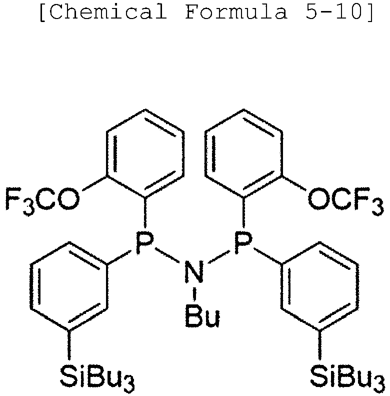

¹H NMR (500 MHz, CDCl₃): δ 7.60(m, 2H), 7.43(m, 2H), 7.41(m, 2H), 7.39(m, 2H), 7.21(m, 2H), 7.18 (m, 2H), 7.13 (m, 2H), 6.98(m, 2H), 2.68(m, 2H), 1.58 (m, 2H), 1.47(m, 12H), 1.31(m, 12H), 1.30 (m, 14H), 0.97(m, 21H)

### Synthesis Example 77: Synthesis of ligand compound represented by Chemical Formula 5-11

0.3 g of isopropyl amine, 1.26 g of triethylamine, and 10 ml of dichloromethane were charged into a well-dried flask in a nitrogen atmosphere, and then stirring was started with cooling to 0°C. 4.37 g of chloro(2-fluorophenyl) (4-(tributylsilyl)phenyl)phosphane was diluted in 10 ml of dichloromethane and then slowly charged into the flask. After stirring the reactant for 20 hours, the conversion rate was checked through NMR. After the reaction was completed, the precipitate was removed by filtration, and the solvent was removed by reducing the pressure. The obtained organic material was purified using column chromatography to obtain a target product, 3.23 g (yield: 75%) of 1-(2-fluorophenyl)-N-((2-fluorophenyl)(3-(tributylsilyl)phenyl)phosphaneyl)-N-isopropyl-1-(3-(tributylsilyl)phenyl)phosphanamine.

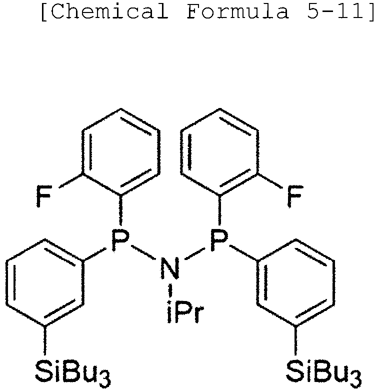

¹H NMR (500 MHz, CDCl₃): δ 7.67 (m, 2H), 7. 61(m, 2H), 7.45(m, 2H), 7.37 (m, 2H), 7.25 (m, 2H), 7.21(m, 6H), 2.84 (m, 1H), 1.50 (m, 12H), 1.38 (m, 12H), 1.27 (m, 12H), 1.13 (m, 6H), 0.90(m, 18H)

### Synthesis Example 78: Synthesis of ligand compound represented by Chemical Formula 5-12

0.3 g of isopropyl amine, 1.26 g of triethylamine, and 10 ml of dichloromethane were charged into a well-dried flask in a nitrogen atmosphere, and then stirring was started with cooling to 0°C. 4.87 g of chloro(4-(tributylsilyl)phenyl) (2-(trifluoromethyl)phenyl)phosphane was diluted in 10 ml of dichloromethane and then slowly charged into the flask. After stirring the reactant for 12 hours, the conversion rate was checked through NMR. After the reaction was completed, the precipitate was removed by filtration, and the solvent was removed by reducing the pressure. The obtained organic material was purified using column chromatography to obtain a target product, 4.51 g (yield: 94%) of N-isopropyl-1-(3-(tributylsilyl) phenyl) -N-((3-(tributylsilyl)phenyl) (2-(trifluoromethyl)phenyl)phosphaneyl)-1-(2-(trifluoromethyl)phenyl)phosphanamine.

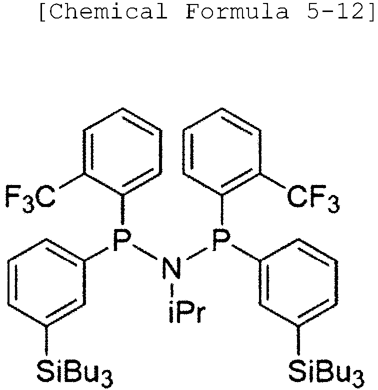

¹H NMR (500 MHz, CDCl₃): δ 7.57 (m, 2H), 7.51(m, 4H), 7.46(m, 2H), 7.39(m, 2H), 7.32(m, 2H), 7.18(m, 2H), 7.08(m, 2H), 2.92 (m, 1H), 1.49 (m, 12H), 1.32(m, 12H), 1.30(m, 12H), 1.08 (m, 6H), 0.92 (m, 18H)

### Synthesis Example 79: Synthesis of ligand compound represented by Chemical Formula 5-13

0.3 g of isopropyl amine, 1.26 g of triethylamine, and 10 ml of dichloromethane were charged into a well-dried flask in a nitrogen atmosphere, and then stirring was started with cooling to 0°C. 4.49 g of chloro(2-methoxyphenyl) (4-(tributylsilyl)phenyl)phosphane was diluted in 10 ml of dichloromethane and then slowly charged into the flask. After stirring the reactant for 19 hours, the conversion rate was checked through NMR. After the reaction was completed, the precipitate was removed by filtration, and the solvent was removed by reducing the pressure. The obtained organic material was purified using column chromatography to obtain a target product, 3.45 g (yield: 78%) of N-isopropyl-1-(2-methoxyphenyl)-N-((2-methoxyphenyl)(3-(tributylsilyl)phenyl)phosphaneyl)-1-(3-(tributylsilyl)phenyl)phosphanamine.

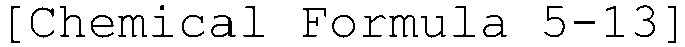

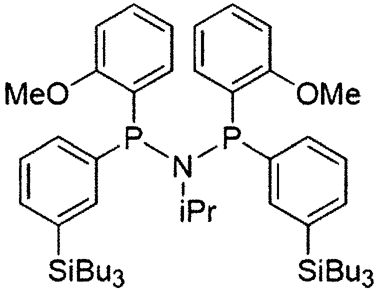

¹H NMR (500 MHz, CDCl₃): δ 7.56(m, 2H), 7.48(m, 2H), 7.43 (m, 2H), 7.38(m, 2H), 7.21(m, 2H), 7.19 (m, 2H), 7.16 (m, 2H), 7.02 (m, 2H), 3.83 (m, 6H), 2.92(m, 1H), 1.52(m, 12H), 1.34(m, 12H), 1.29 (m, 12H), 1.14(m, 6H), 0.94 (m, 18H)

### Synthesis Example 80: Synthesis of ligand compound represented by Chemical Formula 5-14

0.3 g of isopropyl amine, 1.26 g of triethylamine, and 10 ml of dichloromethane were charged into a well-dried flask in a nitrogen atmosphere, and then stirring was started with cooling to 0°C. 4.33 g of chloro (o-tolyl) (4-(tributylsilyl)phenyl)phosphane was diluted in 10 ml of dichloromethane and then slowly charged into the flask. After stirring the reactant for 13 hours, the conversion rate was checked through NMR. After the reaction was completed, the precipitate was removed by filtration, and the solvent was removed by reducing the pressure. The obtained organic material was purified using column chromatography to obtain a target product, 4.13 g (yield: 97%) of N-isopropyl-1-(o-tolyl)-N-(o-tolyl(3-(tributylsilyl)phenyl)phosphaneyl)-1-(3-(tributylsilyl)phenyl)phosphanamine.

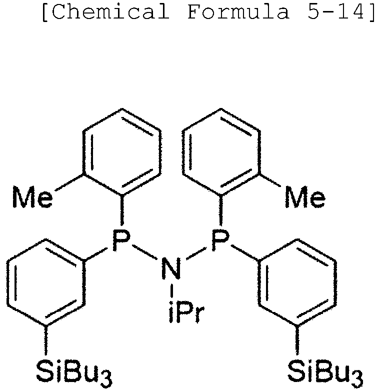

¹H NMR (500 MHz, CDCl₃): δ 7.57(m, 2H), 7.47(m, 2H), 7.41(m, 2H), 7.35 (m, 2H), 7.34 (m, 2H), 7.23 (m, 2H), 7.20 (m, 2H), 7.14 (m, 2H), 2.83(m, 1H), 2.40(m, 6H), 1.50 (m, 12H), 1.39(m, 12H), 1.27 (m, 12H), 1.14(m, 6H), 0.93 (m, 18H)

### Synthesis Example 81: Synthesis of ligand compound represented by Chemical Formula 5-15

0.3 g of isopropyl amine, 1.26 g of triethylamine, and 10 ml of dichloromethane were charged into a well-dried flask in a nitrogen atmosphere, and then stirring was started with cooling to 0°C. 5.03 g of chloro(4-(tributylsilyl)phenyl) (2-(trifluoromethoxy)phenyl)phosphane was diluted in 10 ml of dichloromethane and then slowly charged into the flask. After stirring the reactant for 23 hours, the conversion rate was checked through NMR. After the reaction was completed, the precipitate was removed by filtration, and the solvent was removed by reducing the pressure. The obtained organic material was purified using column chromatography to obtain a target product, 3.72 g (yield: 75%) of N-isopropyl-1-(3-(tributylsilyl)phenyl)-N-((3-(tributylsilyl)phenyl)(2-(trifluoromethoxy)phenyl)phosphaneyl)-1-(2-(trifluoromethoxy)phenyl)phosphanamine.

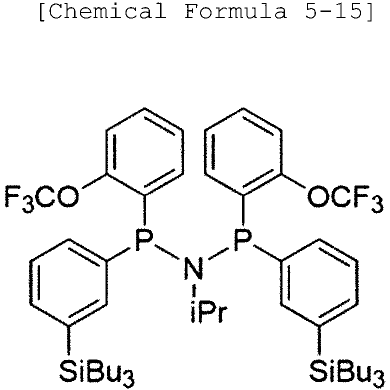

¹H NMR (500 MHz, CDCl₃): δ 7.64(m, 2H), 7. 43 (m, 2H), 7.42 (m, 2H), 7.40(m, 2H), 7.22(m, 4H), 7.14(m, 2H), 7.01(m, 2H), 2.85 (m, 1H), 1.44(m, 12H), 1.33 (m, 12H), 1.31(m, 12H), 1.13 (m, 6H), 0.98 (m, 18H)

### Synthesis Example 82: Synthesis of ligand compound represented by Chemical Formula 5-16

0.5 g of cyclohexylamine, 1.26 g of triethylamine, and 10 ml of dichloromethane were charged into a well-dried flask in a nitrogen atmosphere, and then stirring was started with cooling to 0°C. 4.37 g of chloro(2-fluorophenyl) (4-(tributylsilyl)phenyl)phosphane was diluted in 10 ml of dichloromethane and then slowly charged into the flask. After stirring the reactant for 18 hours, the conversion rate was checked through NMR. After the reaction was completed, the precipitate was removed by filtration, and the solvent was removed by reducing the pressure. The obtained organic material was purified using column chromatography to obtain a target product, 3.91 g (yield: 91%) of N-cyclohexyl-1-(2-fluorophenyl)-N-((2-fluorophenyl)(3-(tributylsilyl)phenyl)phosphaneyl)-1-(3-(tributylsilyl)phenyl)phosphanamine.

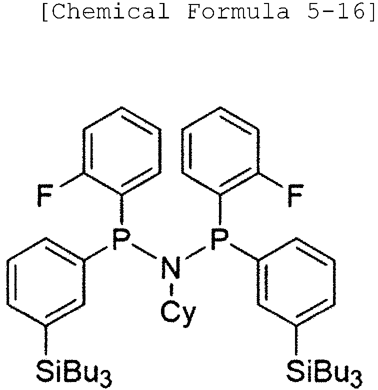

¹H NMR (500 MHz, CDCl₃): δ 7.63(m, 2H), 7.59(m, 2H), 7.42 (m, 2H), 7.39 (m, 2H), 7.25 (m, 2H), 7.22 (m, 4H), 7.21(m, 2H), 2.59(m, 1H), 1.82 (m, 2H), 1.57 (m, 2H), 1.53 (m, 1H), 1.47(m, 13H), 1.36(m, 12H), 1.30(m, 2H), 1.27(m, 12H), 1.18(m, 2H), 0.90(m, 18H)

### Synthesis Example 83: Synthesis of ligand compound represented by Chemical Formula 5-17

0.5 g of cyclohexylamine, 1.26 g of triethylamine, and 10 ml of dichloromethane were charged into a well-dried flask in a nitrogen atmosphere, and then stirring was started with cooling to 0°C. 4.87 g of chloro(4-(tributylsilyl)phenyl)(2-(trifluoromethyl)phenyl)phosphane was diluted in 10 ml of dichloromethane and then slowly charged into the flask. After stirring the reactant for 13 hours, the conversion rate was checked through NMR. After the reaction was completed, the precipitate was removed by filtration, and the solvent was removed by reducing the pressure. The obtained organic material was purified using column chromatography to obtain a target product, 4.75 g (yield: 99%) of N-cyclohexyl-1-(3-(tributylsilyl)phenyl)-N-((3-(tributylsilyl)phenyl)(2-(trifluoromethyl)phenyl)phosphaneyl)-1-(2-(trifluoromethyl)phenyl)phosphanamine.

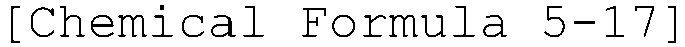

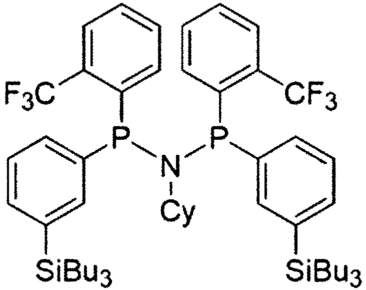

¹H NMR (500 MHz, CDCl₃): δ 7.57 (m, 2H), 7.49(m, 4H), 7.47 (m, 2H), 7.34 (m, 2H), 7.33 (m, 2H), 7.22 (m, 2H), 7.16 (m, 2H), 2.60(m, 1H), 1.80(m, 2H), 1.56(m, 1H), 1.54(m, 2H), 1.51(m, 12H), 1.47(m, 1H), 1.40 (m, 12H), 1.27 (m, 12H), 1.23 (m, 2H), 1.17(m, 2H), 0.94(m, 18H)

### Synthesis Example 84: Synthesis of ligand compound represented by Chemical Formula 5-18

0.5 g of cyclohexylamine, 1.26 g of triethylamine, and 10 ml of dichloromethane were charged into a well-dried flask in a nitrogen atmosphere, and then stirring was started with cooling to 0°C. 4.49 g of chloro(2-methoxyphenyl) (4-(tributylsilyl)phenyl)phosphane was diluted in 10 ml of dichloromethane and then slowly charged into the flask. After stirring the reactant for 11 hour, the conversion rate was checked through NMR. After the reaction was completed, the precipitate was removed by filtration, and the solvent was removed by reducing the pressure. The obtained organic material was purified using column chromatography to obtain a target product, 3.05 g (yield: 69%) of N-cyclohexyl-1-(2-methoxyphenyl)-N-((2-methoxyphenyl)(3-(tributylsilyl)phenyl)phosphaneyl)-1-(3-(tributylsilyl) phenyl) phosphanamine.

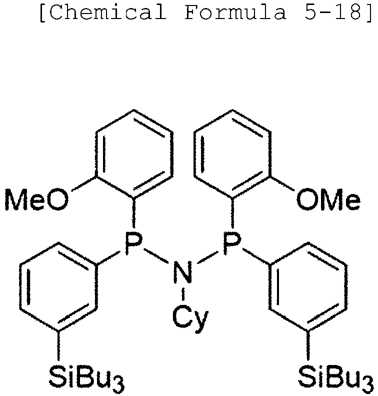

¹H NMR (500 MHz, CDCl₃) : δ 7. 63 (m, 2H), 7.43 (m, 2H), 7.42 (m, 2H), 7.37 (m, 2H), 7.24 (m, 2H), 7.18 (m, 2H), 7.12 (m, 2H), 6.98(m, 2H), 3.87 (m, 6H), 2.61 (m, 1H), 1.77(m, 2H), 1.55 (m, 2H), 1.54(m, 1H), 1.51 (m, 12H), 1.49(m, 1H), 1.40(m, 12H), 1.24(m, 2H), 1.23(m, 12H), 1.17(m, 2H), 0.99(m, 18H)

### Synthesis Example 85: Synthesis of ligand compound represented by Chemical Formula 5-19

0.5 g of cyclohexylamine, 1.26 g of triethylamine, and 10 ml of dichloromethane were charged into a well-dried flask in a nitrogen atmosphere, and then stirring was started with cooling to 0°C. 4.33 g of chloro (o-tolyl)(4-(tributylsilyl)phenyl)phosphane was diluted in 10 ml of dichloromethane and then slowly charged into the flask. After stirring the reactant for 20 hours, the conversion rate was checked through NMR. After the reaction was completed, the precipitate was removed by filtration, and the solvent was removed by reducing the pressure. The obtained organic material was purified using column chromatography to obtain a target product, 3.03 g (yield: 71%) of N-cyclohexyl-1-(o-tolyl)-N-(o-tolyl(3-(tributylsilyl)phenyl)phosphaneyl)-1-(3-(tributylsilyl)phenyl)phosphanamine.

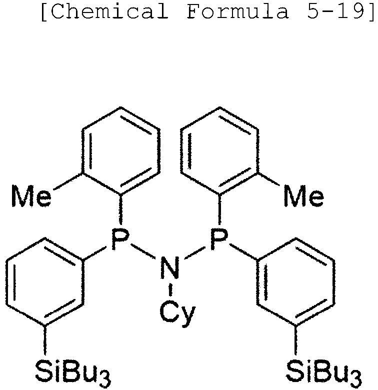

¹H NMR (500 MHz, CDCl₃) : δ 7.56(m, 2H), 7.43(m, 2H), 7.35 (m, 2H), 7.33 (m, 2H), 7.31 (m, 2H), 7.19 (m, 2H), 7.18 (m, 2H), 7.15 (m, 2H), 2.66(m, 1H), 2.38(m, 6H), 1.79 (m, 2H), 1.54 (m, 1H), 1.52 (m, 1H), 1.49(m, 14H), 1.32 (m, 12H), 1.30 (m, 12H), 1.29(m, 2H), 1.19(m, 2H), 0.98 (m, 18H)

### Synthesis Example 86: Synthesis of ligand compound represented by Chemical Formula 5-20

0.5 g of cyclohexylamine, 1.26 g of triethylamine, and 10 ml of dichloromethane were charged into a well-dried flask in a nitrogen atmosphere, and then stirring was started with cooling to 0°C. 5.03 g of chloro(4-(tributylsilyl)phenyl) (2-(trifluoromethoxy)phenyl)phosphane was diluted in 10 ml of dichloromethane and then slowly charged into the flask. After stirring the reactant for 15 hours, the conversion rate was checked through NMR. After the reaction was completed, the precipitate was removed by filtration, and the solvent was removed by reducing the pressure. The obtained organic material was purified using column chromatography to obtain a target product, 3.92 g (yield: 79%) of N-cyclohexyl-1-(3-(tributylsilyl)phenyl)-N-((3-(tributylsilyl)phenyl)(2-(trifluoromethoxy)phenyl)phosphaneyl)-1-(2-(trifluoromethoxy)phenyl)phosphanamine.

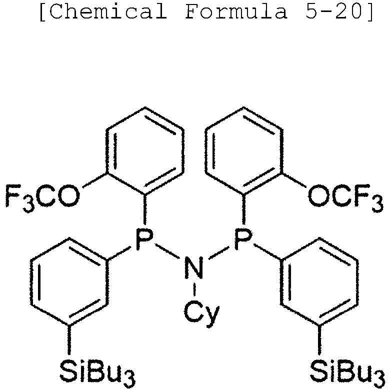

¹H NMR (500 MHz, CDCl₃) : δ 7.61(m, 2H), 7.49 (m, 2H), 7.43 (m, 2H), 7.35 (m, 2H), 7.18 (m, 2H), 7.17 (m, 2H), 7.16 (m, 2H), 6.98 (m, 2H), 2.64(m, 1H), 1.80(m, 2H), 1.52(m, 2H), 1.49(m, 1H), 1.47 (m, 12H), 1.45(m, 1H), 1.39(m, 12H), 1.23 (m, 14H), 1.14(m, 2H), 0.93(m, 18H)

### Synthesis Example 87: Synthesis of intermediate compound represented by Chemical Formula 10 and ligand compound represented by Chemical Formula 2-67

2.03 g of a methylamine HCl salt, 10.62 g of triethylamine, and 10 ml of dichloromethane were charged into a well-dried flask in a nitrogen atmosphere, and then stirring was started with cooling to 0°C. 13.11 g of chloro(2-fluorophenyl) (4-(tributylsilyl)phenyl)phosphane was diluted in 10 ml of dichloromethane and then slowly charged into the flask. After stirring the reactant for 22 hours, the conversion rate was checked through NMR. After the reaction was completed, the precipitate was removed by filtration, and the solvent was removed by reducing the pressure. The obtained organic material was purified using column chromatography to obtain a target intermediate product, 8.55 g (yield: 66%) of 1-(2-fluorophenyl)-N-methyl-1-(4-(tributylsilyl)phenyl)phosphanamine.

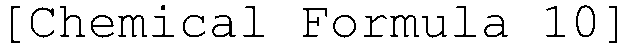

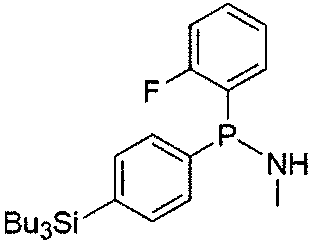

¹H NMR (500 MHz, CDCl₃): δ 7.66(m, 1H), 7.40(m, 2H), 7.29(m, 1H), 7.25(m, 1H), 7.20(m, 3H), 2.47(m, 3H), 1.59(m, 1H), 1.47(m, 6H), 1.35(m, 6H), 1.26(m, 6H), 0.98 (m, 9H)

Subsequently, 2.16 g of 1-(2-fluorophenyl)-N-methyl-1-(4-(tributylsilyl)phenyl)phosphanamine, 10.62 g of triethylamine, and 10 ml of dichloromethane were charged into a well-dried flask in a nitrogen atmosphere, and then stirring was started with cooling to 0°C. 2.44 g of chloro(4-(tributylsilyl)phenyl)(2-(trifluoromethyl)phenyl)phosphane was diluted in 10 ml of dichloromethane and then slowly charged into the flask. After stirring the reactant for 20 hours, the conversion rate was checked through NMR. After the reaction was completed, the precipitate was removed by filtration, and the solvent was removed by reducing the pressure. The obtained organic material was purified using column chromatography to obtain a target product, 4.15 g (yield: 94%) of 1-(2-fluorophenyl)-N-methyl-1-(4-(tributylsilyl)phenyl)-N-((4-(tributylsilyl)phenyl)(2-(trifluoromethyl)phenyl)phosphaneyl)phosphanamine.

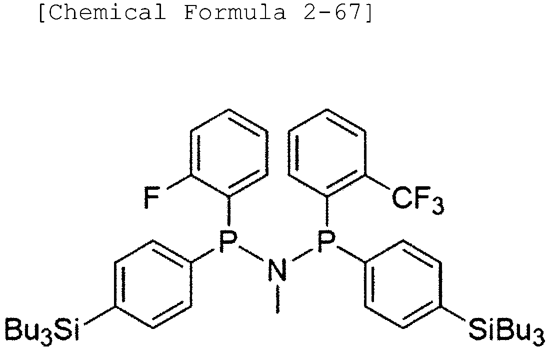

¹H NMR (500 MHz, CDCl₃): δ 7.66(m, 1H), 7.49(m, 1H), 7.48(m, 5H), 7.28(m, 2H), 7.25(m, 1H), 7.21(m, 5H), 7.10 (m, 1H), 2.56 (m, 3H), 1.50(m, 12H), 1.36(m, 12H), 1.28(m, 12H), 0.95 (m, 18H)

### Synthesis Example 88: Synthesis of ligand compound represented by Chemical Formula 2-68

2.16 g of 1-(2-fluorophenyl)-N-methyl-1-(4-(tributylsilyl)phenyl)phosphanamine, 10.62 g of triethylamine, and 10 ml of dichloromethane were charged into a well-dried flask in a nitrogen atmosphere, and then stirring was started with cooling to 0°C. 2.25 g of chloro(2-methoxyphenyl) (4-(tributylsilyl)phenyl)phosphane was diluted in 10 ml of dichloromethane and then slowly charged into the flask. After stirring the reactant for 15 hours, the conversion rate was checked through NMR. After the reaction was completed, the precipitate was removed by filtration, and the solvent was removed by reducing the pressure. The obtained organic material was purified using column chromatography to obtain a target product, 3.46 g (yield: 82%) of 1-(2-fluorophenyl)-N-((2-methoxyphenyl) (4-(tributylsilyl)phenyl)phosphaneyl)-N-methyl-1-(4-(tributylsilyl)phenyl)phosphanamine.

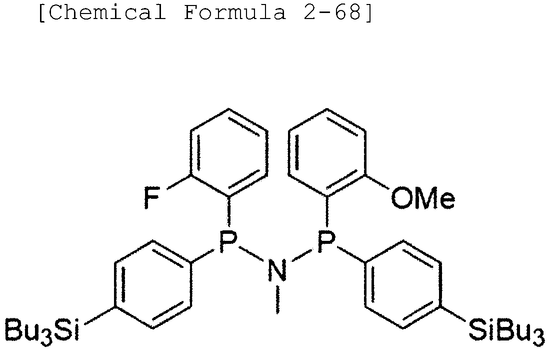

¹H NMR (500 MHz, CDCl₃) : δ 7.61(m, 1H), 7.47(m, 1H), 7.43(m, 4H), 7.25(m, 1H), 7.23(m, 1H), 7.21 (m, 1H), 7.20(m, 5H), 7.10(m, 1H), 7.01(m, 1H), 3.85(m, 3H), 2.48 (m, 3H), 1.44 (m, 12H), 1.37 (m, 12H), 1.32 (m, 12H), 0.95(m, 18H)

### Synthesis Example 89: Synthesis of ligand compound represented by Chemical Formula 2-69

2.16 g of 1-(2-fluorophenyl)-N-methyl-1-(4-(tributylsilyl)phenyl)phosphanamine, 10.62 g of triethylamine, and 10 ml of dichloromethane were charged into a well-dried flask in a nitrogen atmosphere, and then stirring was started with cooling to 0°C. 2.17 g of chloro(o-tolyl)(4-(tributylsilyl)phenyl)phosphane was diluted in 10 ml of dichloromethane and then slowly charged into the flask. After stirring the reactant for 19 hours, the conversion rate was checked through NMR. After the reaction was completed, the precipitate was removed by filtration, and the solvent was removed by reducing the pressure. The obtained organic material was purified using column chromatography to obtain a target product, 3.11 g (yield: 75%) of 1-(2-fluorophenyl)-N-methyl-N-(o-tolyl(4-(tributylsilyl)phenyl)phosphaneyl)-1-(4-(tributylsilyl)phenyl)phosphanamine.

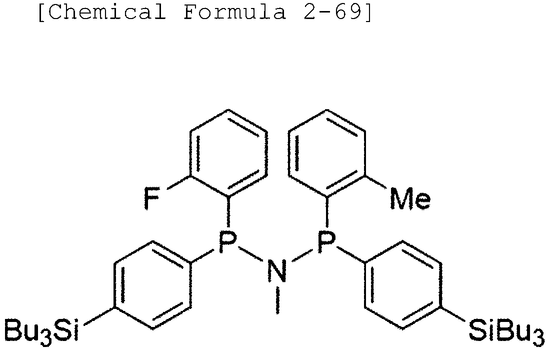

¹H NMR (500 MHz, CDCl₃) : δ 7.68(m, 1H), 7.44 (m, 4H), 7.37 (m, 1H), 7.29(m, 1H), 7.23(m, 1H), 7.21(m, 6H), 7.16(m, 1H), 7.12 (m, 1H), 2.51 (m, 3H), 2.33(m, 3H), 1.52 (m, 12H), 1.32 (m, 12H), 1.29(m, 12H), 0.92 (m, 18H)

### Synthesis Example 90: Synthesis of ligand compound represented by Chemical Formula 2-70

2.16 g of 1-(2-fluorophenyl)-N-methyl-1-(4-(tributylsilyl)phenyl)phosphanamine, 10.62 g of triethylamine, and 10 ml of dichloromethane were charged into a well-dried flask in a nitrogen atmosphere, and then stirring was started with cooling to 0°C. 2.52 g of (chloro(4-(tributylsilyl)phenyl) (2-(trifluoromethoxy)phenyl)phosphane was diluted in 10 ml of dichloromethane and then slowly charged into the flask. After stirring the reactant for 19 hours, the conversion rate was checked through NMR. After the reaction was completed, the precipitate was removed by filtration, and the solvent was removed by reducing the pressure. The obtained organic material was purified using column chromatography to obtain a target product, 2.74 g (yield: 61%) of 1-(2-fluorophenyl)-N-methyl-1-(4-(tributylsilyl)phenyl)-N-((4-(tributylsilyl)phenyl)(2-(trifluoromethoxy)phenyl)phosphaneyl)phosphanamine.

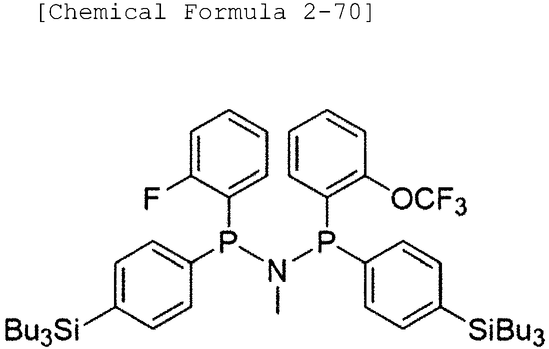

¹H NMR (500 MHz, CDCl₃) : δ 7. 68(m, 1H), 7.48(m, 1H), 7.46(m, 4H), 7.29(m, 1H), 7.22(m, 1H), 7.21(m, 1H), 7.18(m, 6H), 6.98(m, 1H), 2.54 (m, 3H), 1.44(m, 12H), 1.35(m, 12H), 1.25 (m, 12H), 0.91(m, 18H)

### Synthesis Example 91: Synthesis of ligand compound represented by Chemical Formula 2-71

2.16 g of 1-(2-fluorophenyl)-N-methyl-1-(4-(tributylsilyl)phenyl)phosphanamine, 10.62 g of triethylamine, and 10 ml of dichloromethane were charged into a well-dried flask in a nitrogen atmosphere, and then stirring was started with cooling to 0°C. 2.54 g of chloro(4-(tributylsilyl)phenyl)(2-(trifluoro-14-sulfaneyl)phenyl)phosphane was diluted in 10 ml of dichloromethane and then slowly charged into the flask. After stirring the reactant for 11 hour, the conversion rate was checked through NMR. After the reaction was completed, the precipitate was removed by filtration, and the solvent was removed by reducing the pressure. The obtained organic material was purified using column chromatography to obtain a target product, 3.74 g (yield: 83%) of 1-(2-fluorophenyl)-N-methyl-1-(4-(tributylsilyl)phenyl)-N-((4-(tributylsilyl)phenyl)(2-(trifluoro-14-sulfaneyl)phenyl)phosphaneyl)phosphanamine.

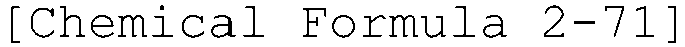

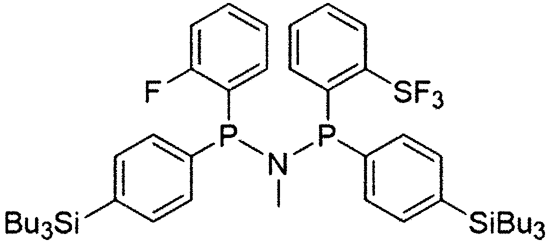

¹H NMR (500 MHz, CDCl₃) : δ 7.70(m, 1H), 7.47(m, 4H), 7.41(m, 1H), 7.28 (m, 1H), 7.22 (m, 3H), 7.19 (m, 5H), 7.01 (m, 1H), 2.50 (m, 3H), 1.47 (m, 12H), 1.31(m, 12H), 1.25 (m, 12H), 0.98(m, 18H)

### Synthesis Example 92: Synthesis of ligand compound represented by Chemical Formula 2-72

2.16 g of 1-(2-fluorophenyl)-N-methyl-1-(4-(tributylsilyl)phenyl)phosphanamine, 10.62 g of triethylamine, and 10 ml of dichloromethane were charged into a well-dried flask in a nitrogen atmosphere, and then stirring was started with cooling to 0°C. 2.33 g of chloro(2-(methylthio)phenyl)(4-(tributylsilyl)phenyl)phosphane was diluted in 10 ml of dichloromethane and then slowly charged into the flask. After stirring the reactant for 17 hours, the conversion rate was checked through NMR. After the reaction was completed, the precipitate was removed by filtration, and the solvent was removed by reducing the pressure. The obtained organic material was purified using column chromatography to obtain a target product, 2.88 g (yield: 67%) of 1-(2-fluorophenyl)-N-methyl-N-((2-(methylthio)phenyl)(4-(tributylsilyl)phenyl)phosphaneyl)-1-(4-(tributylsilyl)phenyl)phosphanamine.

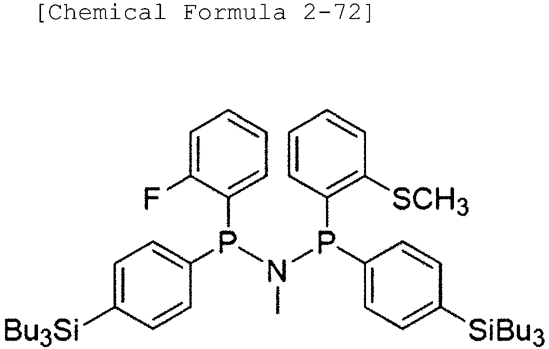

¹H NMR (500 MHz, CDCl₃) : δ 7.64(m, 1H), 7.44 (m, 4H), 7.37 (m, 2H), 7.28 (m, 2H), 7.24 (m, 1H), 7.20 (m, 5H), 7.12 (m, 1H), 2.56 (m, 3H), 2.54 (m, 3H), 1.47(m, 12H), 1.32 (m, 12H), 1.25 (m, 12H), 0.91 (m, 18H)

### Synthesis Example 93: Synthesis of ligand compound represented by Chemical Formula 2-73

2.16 g of 1-(2-fluorophenyl)-N-methyl-1-(4-(tributylsilyl)phenyl)phosphanamine, 10.62 g of triethylamine, and 10 ml of dichloromethane were charged into a well-dried flask in a nitrogen atmosphere, and then stirring was started with cooling to 0°C. 2.57 g of 2-(chloro(4-(tributylsilyl)phenyl)phosphaneyl)phenyl methanesulfonate was diluted in 10 ml of dichloromethane and then slowly charged into the flask. After stirring the reactant for 21 hour, the conversion rate was checked through NMR. After the reaction was completed, the precipitate was removed by filtration, and the solvent was removed by reducing the pressure. The obtained organic material was purified using column chromatography to obtain a target product, 3.68 g (yield: 81%) of 2-((((2-fluorophenyl) (4-(tributylsilyl)phenyl)phosphaneyl)(methyl)amino)(4-(tributylsilyl)phenyl)phosphaneyl)phenyl methanesulfonate.

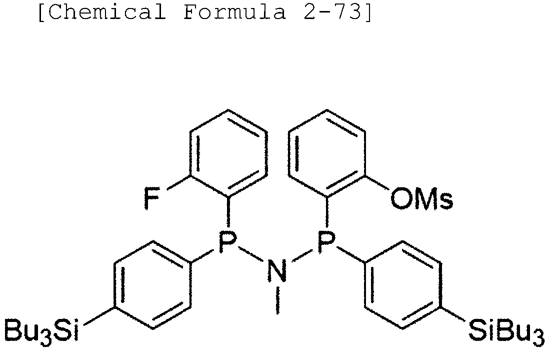

¹H NMR (500 MHz, CDCl₃) : δ 7.65(m, 1H), 7.53 (m, 1H), 7.47(m, 1H), 7.40(m, 4H), 7.30 (m, 1H), 7.25 (m, 2H), 7.23 (m, 1H), 7.15 (m, 5H), 3.62(m, 3H), 2.54 (m, 3H), 1.52 (m, 12H), 1.36(m, 12H), 1.31(m, 12H), 0.89(m, 18H)

### Synthesis Example 94: Synthesis of ligand compound represented by Chemical Formula 2-74

2.16 g of 1-(2-fluorophenyl)-N-methyl-1-(4-(tributylsilyl)phenyl)phosphanamine, 10.62 g of triethylamine, and 10 ml of dichloromethane were charged into a well-dried flask in a nitrogen atmosphere, and then stirring was started with cooling to 0°C. 2.46 g of chloro(4-(tributylsilyl)phenyl) (2-(trimethylsilyl)phenyl)phosphane was diluted in 10 ml of dichloromethane and then slowly charged into the flask. After stirring the reactant for 15 hours, the conversion rate was checked through NMR. After the reaction was completed, the precipitate was removed by filtration, and the solvent was removed by reducing the pressure. The obtained organic material was purified using column chromatography to obtain a target product, 4.3 g (yield: 97%) of 1-(2-fluorophenyl)-N-methyl-1-(4-(tributylsilyl)phenyl)-N-((4-(tributylsilyl)phenyl)(2-(trimethylsilyl)phenyl)phosphaneyl)phosphanamine.

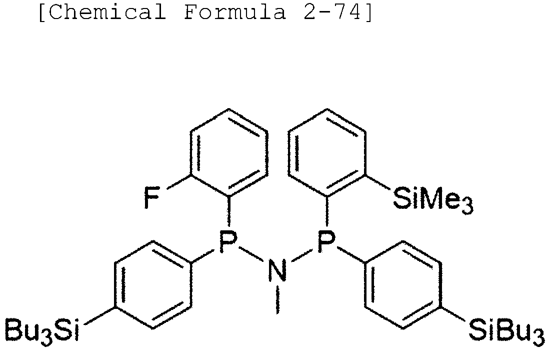

¹H NMR (500 MHz, CDCl₃) : δ 7. 67 (m, 1H), 7.49(m, 6H), 7.33 (m, 1H), 7.27 (m, 2H), 7.17 (m, 6H), 2.56 (m, 3H), 1.51 (m, 12H), 1.32(m, 12H), 1.31(m, 12H), 0.89(m, 18H), 0.27 (m, 9H)

### Synthesis Example 95: Synthesis of ligand compound represented by Chemical Formula 2-75

2.16 g of 1-(2-fluorophenyl)-N-methyl-1-(4-(tributylsilyl)phenyl)phosphanamine, 10.62 g of triethylamine, and 10 ml of dichloromethane were charged into a well-dried flask in a nitrogen atmosphere, and then stirring was started with cooling to 0°C. 2.3 g of benzofuran-7-ylchloro(4-(tributylsilyl)phenyl)phosphane was diluted in 10 ml of dichloromethane and then slowly charged into the flask. After stirring the reactant for 11 hour, the conversion rate was checked through NMR. After the reaction was completed, the precipitate was removed by filtration, and the solvent was removed by reducing the pressure. The obtained organic material was purified using column chromatography to obtain a target product, 4.23 g (yield: 99%) of 1-(benzofuran-7-yl)-N-((2-fluorophenyl) (4-(tributylsilyl)phenyl)phosphaneyl)-N-methyl-1-(4-(tributylsilyl)phenyl)phosphanamine.

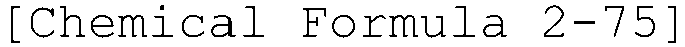

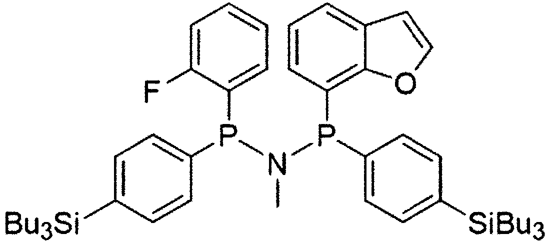

¹H NMR (500 MHz, CDCl₃) : δ 7. 62 (m, 3H), 7.50(m, 4H), 7.48(m, 1H), 7.33 (m, 1H), 7.25 (m, 1H), 7.21 (m, 6H), 6.83(m, 1H), 2.54 (m, 3H), 1.47(m, 12H), 1.31 (m, 12H), 1.29(m, 12H), 0.96 (m, 18H)

### Synthesis Example 96: Synthesis of ligand compound represented by Chemical Formula 2-76

2.16 g of 1-(2-fluorophenyl)-N-methyl-1-(4-(tributylsilyl)phenyl)phosphanamine, 10.62 g of triethylamine, and 10 ml of dichloromethane were charged into a well-dried flask in a nitrogen atmosphere, and then stirring was started with cooling to 0°C. 2.55 g of chloro (dibenzo [b, d] furan-4-yl) (4-(tributylsilyl)phenyl)phosphane was diluted in 10 ml of dichloromethane and then slowly charged into the flask. After stirring the reactant for 20 hours, the conversion rate was checked through NMR. After the reaction was completed, the precipitate was removed by filtration, and the solvent was removed by reducing the pressure. The obtained organic material was purified using column chromatography to obtain a target product, 2.94 g (yield: 65%) of 1-(dibenzol[b,d]furan-4-yl)-N-((2-fluorophenyl)(4-(tributylsilyl)phenyl)phosphaneyl)-N-methyl-1-(4-(tributylsilyl)phenyl)phosphanamine.

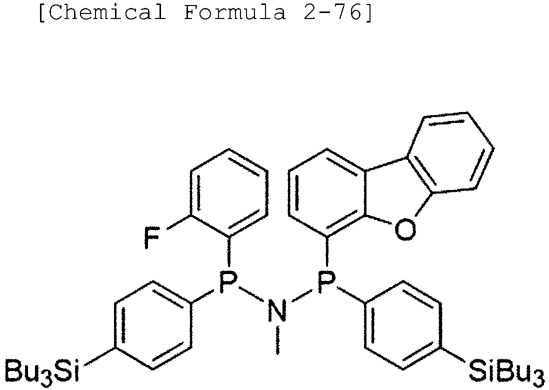

¹H NMR (500 MHz, CDCl₃) : δ 8.02 (m, 2H), 7.71 (m, 1H), 7.59(m, 1H), 7.49(m, 1H), 7.46(m, 1H), 7.45(m, 4H), 7.40 (m, 2H), 7.23(m, 1H), 7.21(m, 1H), 7.18(m, 5H), 2.48 (m, 3H), 1.43(m, 12H), 1.40(m, 12H), 1.26(m, 12H), 0.96(m, 18H)

### Comparative Synthesis Example 1: Synthesis of ligand compound represented by Chemical Formula 11

5.5 mmol (0.5 eq) of 3-methyl-2-butanamine, 22 mmol (2 eq) of triethylamine, and 10 ml of dichloromethane were charged into a dried flask in a nitrogen atmosphere, and stirring was started. Subsequently, after cooling to 0°C, 11 mmol (1 eq) of chlorobis(4-(tributlysilyl)phenyl)phosphane was slowly charged thereinto. After the reaction was completed, the precipitated solid was removed through a filter, the solvent was removed by reducing the pressure, and a compound represented by Chemical Formula 11 was obtained through column chromatography.

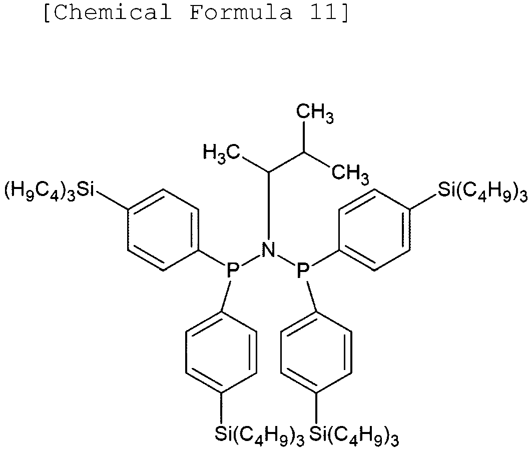

### N-(bis(4-(tributylsilyl)phenyl)phosphaneyl)-N-(3-methylbutan-2-yl)-1,1-bis(4-(tributylsilyl)phenyl)phosphanamine

¹H NMR (500 MHz, C₆D₆): δ 7.96-7.34(m, 16H), 3.64-3.53(m, 1H), 1.81-1.72(m, 1H), 1.38-1.29(m, 48H), 1.05(d, 3H), 0.91-0.84(m, 39H), 0.82-0.76(m, 24H), 0.55(d, 3H)

### Comparative Synthesis Example 2: Synthesis of ligand compound represented by Chemical Formula 12

5.5 mmol (0.5 eq) of cyclohexylamine, 22 mmol (2 eq) of triethylamine, and 10 ml of dichloromethane were charged into a dried flask in a nitrogen atmosphere, and stirring was started. Subsequently, after cooling to 0°C, 11 mmol (1 eq) of chlorobis(4-cyclohexylphenyl)phosphane was slowly charged thereinto. After the reaction was completed, the precipitated solid was removed through a filter, the solvent was removed by reducing the pressure, and a compound represented by Chemical Formula 12 was obtained through column chromatography.

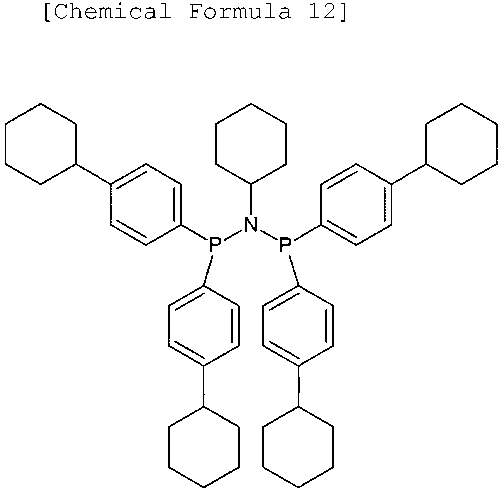

### N-(bis(4-cyclohexylphenyl)phosphaneyl)-N-cyclohexyl-1,1-bis(4-cyclohexylphenyl)phosphanamine

¹H NMR (500 MHz, C₆D₆): δ 7.58(br.s, 8H), 7.08(d, 8H), 3.47(pent, 1H), 2.35(t, 4H), 2.21-2.11(m, 2H), 1.80(d, 8H), 1.71-1.56(m, 16H), 1.44-1.11(m, 24H)

### Comparative Synthesis Example 3: Synthesis of ligand compound represented by Chemical Formula 13

5.5 mmol (0.5 eq) of cyclohexylamine, 22 mmol (2 eq) of triethylamine, and 10 ml of dichloromethane were charged into a dried flask in a nitrogen atmosphere, and stirring was started. Subsequently, after cooling to 0°C, 11 mmol (1 eq) of chlorobis(4-phenylphenyl)phosphane was slowly charged thereinto. After the reaction was completed, the precipitated solid was removed through a filter, the solvent was removed by reducing the pressure, and a compound represented by Chemical Formula 13 was obtained through column chromatography.

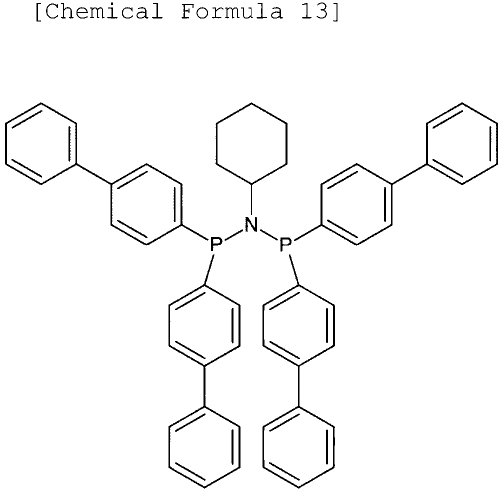

### 1,1-di([1,1'-biphenyl]-4-yl)-N-cyclohexyl-N-(di([1,1'-biphenyl]-4-yl)phosphaneyl)phosphanamine

¹H NMR(500 MHz, C₆D₆): δ 7.92-7.42(m, 24H), 7.17 (t, 8H), 7.10(t, 4H), 3.58(pent, 1H), 2.28-2.17(m, 2H), 1.84-1.77(m, 2H), 1.67-1.60(m, 2H), 1.48-1.42(m, 1H), 1.18-1.07(m, 3H)

### Comparative Synthesis Example 4: Synthesis of ligand compound represented by Chemical Formula 14

5.5 mmol (0.5 eq) of cyclohexylamine, 22 mmol (2 eq) of triethylamine, and 10 ml of dichloromethane were charged into a dried flask in a nitrogen atmosphere, and stirring was started. Subsequently, after cooling to 0°C, 11 mmol (1 eq) of chlorobis(3,5-dimethylphenyl)phosphane was slowly charged thereinto. After the reaction was completed, the precipitated solid was removed through a filter, the solvent was removed by reducing the pressure, and a compound represented by Chemical Formula 14 was obtained through column chromatography.

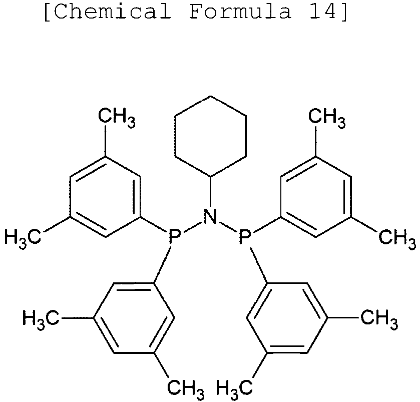

### N-(bis(3,5-dimethylphenyl)phosphaneyl)-N-cyclohexyl-1,1-bis(3,5-dimethylphenyl)phosphanamine

¹H NMR(500 MHz, C₆D₆): δ 7.38(br.s, 8H), 6.79(s, 4H), 3.63(pent, 1H), 2.24-2.13(m, 2H), 2.10(s, 24H), 1.77(d, 2H), 1.60(d, 2H), 1.45-1.39(m, 1H), 1.19-1.00(m, 3H)

### Examples and Comparative Examples

### Example 1

In an argon gas atmosphere, 0.5 mmol of chromium (III) chloride tetrahydrofuran (Cr(THF)₃Cl₃), 0.5 mmol of the ligand compound represented by Chemical Formula 2-1 according to Synthesis Example 1, and 0.5 mmol of N,N-dimethylanilinium tetrakis(pentafluorophenyl)borate (AB) as a co-catalyst were put in a flask. Then, 30 ml of dichloromethane was charged thereinto, stirring was carried out for 1 hour, and then the solvent was removed by vacuuming. Then, after dissolution in methylcyclohexane and then filtration, the solvent was removed again by vacuuming, and dissolution was carried out in methylcyclohexane to prepare a 0.15 mM (based on Cr) catalyst solution.

A Parr reactor having a capacity of 600 ml was prepared and subjected to vacuuming at 120°C for 2 hours. Then, the inside thereof was replaced with argon, and the temperature was lowered to 70°C. Thereafter, 180 ml of methylcyclohexane and 725 µmol of diisobutyl aluminum hydride as an activator in 2 ml were injected, and 5 ml (0.75 µmol Cr) of the catalyst solution was injected. After stirring at 1,000 rpm for 2 minutes, a valve of the ethylene line set to 40 bar was opened to fill the reactor with ethylene, and then stirring was carried out at 1,000 rpm for 60 minutes. The valve of the ethylene line was closed, and the reactor was cooled to 0°C using a dry ice/acetone bath. Then, unreacted ethylene was slowly vented, and then 0.5 ml of nonane (GC internal standard) was added thereto. After stirring for 10 seconds, 2 ml of the liquid portion of the reactor was taken and quenched with water, and the obtained organic portion was filtered through a PTFE syringe filter to make a GC-FID sample. Then, the distribution of the liquid product was subjected to GC analysis (Agilent 6890N, manufactured by Agilent Technologies, Inc., Alltech AT-5 30 m X 0.32 mm ID X 0.25 µm; series no. 12446)). In addition, 400 ml of ethanol/HCl(10 vol% of aqueous 12 M HCl solution) was added to the remaining reaction solution, the resultant mixture was stirred and filtered, and then the amount of the solid product was analyzed. The obtained polymer was dried overnight in a vacuum oven at 80°C.

### Examples 2 to 96 and Comparative Examples 1 to 4

The same method as in Example 1-1 was carried out, except that the kind of catalyst was changed as shown in Table 1 below.

**[Table 1]**

| | | | |
|---|---|---|---|
| Classificatio n | Ligand compound | Co-catalyst | Chromium source |
| Example 1 | Synthesis Example 1 (Chemical Formula 2-1) | AB | Cr (THF) ₃Cl₃ |
| Example 2 | Synthesis Example 2 (Chemical Formula 2-2) | AB | Cr (THF) ₃Cl₃ |
| Example 3 | Synthesis Example 3 (Chemical Formula 2-3) | AB | Cr (THF) ₃Cl₃ |
| Example 4 | Synthesis Example 4 (Chemical Formula 2-4) | AB | Cr (THF) ₃Cl₃ |
| Example 5 | Synthesis Example 5 (Chemical Formula 2-5) | AB | Cr (THF) ₃Cl₃ |
| Example 6 | Synthesis Example 6 (Chemical Formula 2-6) | AB | Cr (THF) ₃Cl₃ |
| Example 7 | Synthesis Example 7 (Chemical Formula 2-7) | AB | Cr (THF) ₃Cl₃ |
| Example 8 | Synthesis Example 8 (Chemical Formula 2-8) | AB | Cr (THF) ₃Cl₃ |
| Example 9 | Synthesis Example 9 (Chemical Formula 2-9) | AB | Cr (THF) ₃Cl₃ |
| Example 10 | Synthesis Example 10 (Chemical Formula 2-10) | AB | Cr (THF) ₃Cl₃ |
| Example 11 | Synthesis Example 11 (Chemical Formula 2-11) | AB | Cr (THF) ₃Cl₃ |
| Example 12 | Synthesis Example 12 (Chemical Formula 2-12) | AB | Cr (THF) ₃Cl₃ |
| Example 13 | Synthesis Example 13 (Chemical Formula 2-13) | AB | Cr (THF) ₃Cl₃ |
| Example 14 | Synthesis Example 14 (Chemical Formula 2-14) | AB | Cr (THF) ₃Cl₃ |
| Example 15 | Synthesis Example 15 (Chemical Formula 2-15) | AB | Cr (THF) ₃Cl₃ |
| Example 16 | Synthesis Example 16 (Chemical Formula 2-16) | AB | Cr (THF) ₃Cl₃ |
| Example 17 | Synthesis Example 17 (Chemical Formula 2-17) | AB | Cr (THF) ₃Cl₃ |
| Example 18 | Synthesis Example 18 (Chemical Formula 2-18) | AB | Cr (THF) ₃Cl₃ |
| Example 19 | Synthesis Example 19 (Chemical Formula 2-19) | AB | Cr (THF) ₃Cl₃ |
| Example 20 | Synthesis Example 20 (Chemical Formula 2-20) | AB | Cr (THF) ₃Cl₃ |
| Example 21 | Synthesis Example 21 (Chemical Formula 2-21) | AB | Cr (THF) ₃Cl₃ |
| Example 22 | Synthesis Example 22 (Chemical Formula 2-22) | AB | Cr (THF) ₃Cl₃ |
| Example 23 | Synthesis Example 23 (Chemical Formula 2-23) | AB | Cr (THF) ₃Cl₃ |
| Example 24 | Synthesis Example 24 (Chemical Formula 2-24) | AB | Cr (THF) ₃Cl₃ |
| Example 25 | Synthesis Example 25 (Chemical Formula 2-25) | AB | Cr (THF) ₃Cl₃ |
| Example 26 | Synthesis Example 26 (Chemical Formula 2-26) | AB | Cr (THF) ₃Cl₃ |
| Example 27 | Synthesis Example 27 (Chemical Formula 2-27) | AB | Cr (THF) ₃Cl₃ |
| Example 28 | Synthesis Example 28 (Chemical Formula 2-28) | AB | Cr (THF) ₃Cl₃ |
| Example 29 | Synthesis Example 29 (Chemical Formula 2-29) | AB | Cr (THF) ₃Cl₃ |
| Example 30 | Synthesis Example 30 (Chemical Formula 2-30) | AB | Cr (THF) ₃Cl₃ |
| Example 31 | Synthesis Example 31 (Chemical Formula 2-31) | AB | Cr (THF) ₃Cl₃ |
| Example 32 | Synthesis Example 32 (Chemical Formula 2-32) | AB | Cr (THF) ₃Cl₃ |
| Example 33 | Synthesis Example 33 (Chemical Formula 2-33) | AB | Cr (THF) ₃Cl₃ |
| Example 34 | Synthesis Example 34 (Chemical Formula 2-34) | AB | Cr (THF) ₃Cl₃ |
| Example 35 | Synthesis Example 35 (Chemical Formula 2-35) | AB | Cr (THF) ₃Cl₃ |
| Example 36 | Synthesis Example 36 (Chemical Formula 2-36) | AB | Cr (THF) ₃Cl₃ |
| Example 37 | Synthesis Example 37 (Chemical Formula 2-37) | AB | Cr (THF) ₃Cl₃ |
| Example 38 | Synthesis Example 38 (Chemical Formula 2-38) | AB | Cr (THF) ₃Cl₃ |
| Example 39 | Synthesis Example 39 (Chemical Formula 2-39) | AB | Cr (THF) ₃Cl₃ |
| Example 40 | Synthesis Example 40 (Chemical Formula 2-40) | AB | Cr (THF) ₃Cl₃ |
| Example 41 | Synthesis Example 41 (Chemical Formula 2-41) | AB | Cr (THF) ₃Cl₃ |
| Example 42 | Synthesis Example 42 (Chemical Formula 2-42) | AB | Cr (THF) ₃Cl₃ |
| Example 43 | Synthesis Example 43 (Chemical Formula 2-43) | AB | Cr (THF) ₃Cl₃ |
| Example 44 | Synthesis Example 44 (Chemical Formula 2-44) | AB | Cr (THF) ₃Cl₃ |
| Example 45 | Synthesis Example 45 (Chemical Formula 2-45) | AB | Cr (THF) ₃Cl₃ |
| Example 46 | Synthesis Example 46 (Chemical Formula 2-46) | AB | Cr (THF) ₃Cl₃ |
| Example 47 | Synthesis Example 47 (Chemical Formula 2-47) | AB | Cr (THF) ₃Cl₃ |
| Example 48 | Synthesis Example 48 (Chemical Formula 2-48) | AB | Cr (THF) ₃Cl₃ |
| Example 49 | Synthesis Example 49 (Chemical Formula 2-49) | AB | Cr (THF) ₃Cl₃ |
| Example 50 | Synthesis Example 50 (Chemical Formula 2-50) | AB | Cr (THF) ₃Cl₃ |
| Example 51 | Synthesis Example 51 (Chemical Formula 2-51) | AB | Cr (THF) ₃Cl₃ |
| Example 52 | Synthesis Example 52 (Chemical Formula 2-52) | AB | Cr (THF) ₃Cl₃ |
| Example 53 | Synthesis Example 53 (Chemical Formula 2-53) | AB | Cr (THF) ₃Cl₃ |
| Example 54 | Synthesis Example 54 (Chemical Formula 2-54) | AB | Cr (THF) ₃Cl₃ |
| Example 55 | Synthesis Example 55 (Chemical Formula 2-55) | AB | Cr (THF) ₃Cl₃ |
| Example 56 | Synthesis Example 56 (Chemical Formula 2-56) | AB | Cr (THF) ₃Cl₃ |
| Example 57 | Synthesis Example 57 (Chemical Formula 2-57) | AB | Cr (THF) ₃Cl₃ |
| Example 58 | Synthesis Example 58 (Chemical Formula 2-58) | AB | Cr (THF) ₃Cl₃ |
| Example 59 | Synthesis Example 59 (Chemical Formula 2-59) | AB | Cr (THF) ₃Cl₃ |
| Example 60 | Synthesis Example 60 (Chemical Formula 2-60) | AB | Cr (THF) ₃Cl₃ |
| Example 61 | Synthesis Example 61 (Chemical Formula 2-61) | AB | Cr (THF) ₃Cl₃ |
| Example 62 | Synthesis Example 62 (Chemical Formula 2-62) | AB | Cr (THF) ₃Cl₃ |
| Example 63 | Synthesis Example 63 (Chemical Formula 2-63) | AB | Cr (THF) ₃Cl₃ |
| Example 64 | Synthesis Example 64 (Chemical Formula 2-64) | AB | Cr (THF) ₃Cl₃ |
| Example 65 | Synthesis Example 65 (Chemical Formula 2-65) | AB | Cr (THF) ₃Cl₃ |
| Example 66 | Synthesis Example 66 (Chemical Formula 2-66) | AB | Cr (THF) ₃Cl₃ |
| Example 67 | Synthesis Example 67 (Chemical Formula 5-1) | AB | Cr (THF) ₃Cl₃ |
| Example 68 | Synthesis Example 68 (Chemical Formula 5-2) | AB | Cr (THF) ₃Cl₃ |
| Example 69 | Synthesis Example 69 (Chemical Formula 5-3) | AB | Cr (THF) ₃Cl₃ |
| Example 70 | Synthesis Example 70 (Chemical Formula 5-4) | AB | Cr (THF) ₃Cl₃ |
| Example 71 | Synthesis Example 71 (Chemical Formula 5-5) | AB | Cr (THF) ₃Cl₃ |
| Example 72 | Synthesis Example 72 (Chemical Formula 5-6) | AB | Cr (THF) ₃Cl₃ |
| Example 73 | Synthesis Example 73 (Chemical Formula 5-7) | AB | Cr (THF) ₃Cl₃ |
| Example 74 | Synthesis Example 74 (Chemical Formula 5-8) | AB | Cr (THF) ₃Cl₃ |
| Example 75 | Synthesis Example 75 (Chemical Formula 5-9) | AB | Cr (THF) ₃Cl₃ |
| Example 76 | Synthesis Example 76 (Chemical Formula 5-10) | AB | Cr (THF) ₃Cl₃ |
| Example 77 | Synthesis Example 77 (Chemical Formula 5-11) | AB | Cr (THF) ₃Cl₃ |
| Example 78 | Synthesis Example 78 (Chemical Formula 5-12) | AB | Cr (THF) ₃Cl₃ |
| Example 79 | Synthesis Example 79 (Chemical Formula 5-13) | AB | Cr (THF) ₃Cl₃ |
| Example 80 | Synthesis Example 80 (Chemical Formula 5-14) | AB | Cr (THF) ₃Cl₃ |
| Example 81 | Synthesis Example 81 (Chemical Formula 5-15) | AB | Cr (THF) ₃Cl₃ |
| Example 82 | Synthesis Example 82 (Chemical Formula 5-16) | AB | Cr (THF) ₃Cl₃ |
| Example 83 | Synthesis Example 83 (Chemical Formula 5-17) | AB | Cr (THF) ₃Cl₃ |
| Example 84 | Synthesis Example 84 (Chemical Formula 5-18) | AB | Cr (THF) ₃Cl₃ |
| Example 85 | Synthesis Example 85 (Chemical Formula 5-19) | AB | Cr (THF) ₃Cl₃ |
| Example 86 | Synthesis Example 86 (Chemical Formula 5-20) | AB | Cr (THF) ₃Cl₃ |
| Example 87 | Synthesis Example 87 (Chemical Formula 2-67) | AB | Cr (THF) ₃Cl₃ |
| Example 88 | Synthesis Example 88 (Chemical Formula 2-68) | AB | Cr (THF) ₃Cl₃ |
| Example 89 | Synthesis Example 89 (Chemical Formula 2-69) | AB | Cr (THF) ₃Cl₃ |
| Example 90 | Synthesis Example 90 (Chemical Formula 2-70) | AB | Cr (THF) ₃Cl₃ |
| Example 91 | Synthesis Example 91 (Chemical Formula 2-71) | AB | Cr (THF) ₃Cl₃ |
| Example 92 | Synthesis Example 92 (Chemical Formula 2-72) | AB | Cr (THF) ₃Cl₃ |
| Example 93 | Synthesis Example 93 (Chemical Formula 2-73) | AB | Cr (THF) ₃Cl₃ |
| Example 94 | Synthesis Example 94 (Chemical Formula 2-74) | AB | Cr (THF) ₃Cl₃ |
| Example 95 | Synthesis Example 95 (Chemical Formula 2-75) | AB | Cr (THF) ₃Cl₃ |
| Example 96 | Synthesis Example 96 (Chemical Formula 2-76) | AB | Cr (THF) ₃Cl₃ |
| Comparative Example 1 | Comparative Synthesis Example1 (Chemical Formula 11) | AB | Cr (THF) ₃Cl₃ |
| Comparative Example 2 | Comparative Synthesis Example2 (Chemical Formula 12) | AB | Cr (THF) ₃Cl₃ |
| Comparative Example 3 | Comparative Synthesis Example3 (Chemical Formula 13) | AB | Cr (THF) ₃Cl₃ |
| Comparative Example 4 | Comparative Synthesis Example4 (Chemical Formula 14) | AB | Cr (THF) ₃Cl₃ |

### Experimental Example

Table 2 below shows the results of the ethylene oligomerization reaction according to the above-described examples and comparative examples.

Catalytic activity (ton/mol·Cr/hr): The catalytic activity was calculated from the total product weight (ton), which is obtained by summing the weights (ton) of the obtained liquid product and the obtained solid product.

1-C6 and 1-C8 selectivity (% by weight): The contents of 1-hexene (1-C6) and 1-octene (1-C8) were calculated from the results obtained by analyzing the distribution of the liquid product by GC, and then the weight percent of 1-hexene or 1-octene based on the total weight of the product was calculated.

Solid (% by weight): The weight percent of the solid product based on the total weight of the product was calculated. This indicates the extent to which polyethylene in which the number of carbon atoms is about 40 or more is produced, where the polyethylene is an insoluble solid that does not dissolve in a solvent.

**[Table 2]**

| Classifica tion | Catalytic activity | Selectivity for 1-C6 and 1-C8 | | | Solid |
|---|---|---|---|---|---|
| | | 1-C6 | 1-C8 | Total | |
| | (ton/mol·Cr/h r) | (wt%) | (wt%) | (wt%) | (wt%) |
| Example 1 | 153 | 39.1 | 49.7 | 88.9 | 0.63 |
| Example 2 | 142 | 44.3 | 40.4 | 84.7 | 0.46 |
| Example 3 | 149 | 37.8 | 48.2 | 86.0 | 0.44 |
| Example 4 | 145 | 43.3 | 40.3 | 83.6 | 0.64 |
| Example 5 | 153 | 37.6 | 51.2 | 88.9 | 0.61 |
| Example 6 | 141 | 39.1 | 48.6 | 87.7 | 0.53 |
| Example 7 | 143 | 37.5 | 50.8 | 88.4 | 0.65 |
| Example 8 | 134 | 42.3 | 45.2 | 87.5 | 0.53 |
| Example 9 | 133 | 38.1 | 47.6 | 85.7 | 0.50 |
| Example 10 | 150 | 36.0 | 51.8 | 87.8 | 0.41 |
| Example 11 | 155 | 37.0 | 50.1 | 87.2 | 0.40 |
| Example 12 | 175 | 37.9 | 51.0 | 88.9 | 0.58 |
| Example 13 | 161 | 44.2 | 42.1 | 86.3 | 0.56 |
| Example 14 | 173 | 39.2 | 49.3 | 88.5 | 0.54 |
| Example 15 | 168 | 43.1 | 40.1 | 83.2 | 0.68 |
| Example 16 | 172 | 38.4 | 50.7 | 89.2 | 0.37 |
| Example 17 | 164 | 40.4 | 49.2 | 89.5 | 0.37 |
| Example 18 | 170 | 38.0 | 51.9 | 89.9 | 0.37 |
| Example 19 | 150 | 41.9 | 46.1 | 88.0 | 0.47 |
| Example 20 | 159 | 38.7 | 48.1 | 86.8 | 0.54 |
| Example 21 | 171 | 37.3 | 50.4 | 87.6 | 0.52 |
| Example 22 | 189 | 37.1 | 50.1 | 87.2 | 0.30 |
| Example 23 | 178 | 55.0 | 33.7 | 88.7 | 0.56 |
| Example 24 | 157 | 60.2 | 25.2 | 85.3 | 0.49 |
| Example 25 | 170 | 55.1 | 31.6 | 86.8 | 0.34 |
| Example 26 | 164 | 58.9 | 21.8 | 80.6 | 0.57 |
| Example 27 | 171 | 53.9 | 33.8 | 87.7 | 0.49 |
| Example 28 | 170 | 55.4 | 31.3 | 86.7 | 0.68 |
| Example 29 | 165 | 51.9 | 35.0 | 86.9 | 0.33 |
| Example 30 | 149 | 57.9 | 30.7 | 88.6 | 0.39 |
| Example 31 | 157 | 55.3 | 31.8 | 87.1 | 0.47 |
| Example 32 | 172 | 53.1 | 34.4 | 87.4 | 0.65 |
| Example 33 | 186 | 53.6 | 34.9 | 88.6 | 0.46 |
| Example 34 | 174 | 46.6 | 39.1 | 85.7 | 0.52 |
| Example 35 | 158 | 53.0 | 30.8 | 83.8 | 0.45 |
| Example 36 | 169 | 46.9 | 37.2 | 84.1 | 0.32 |
| Example 37 | 168 | 50.9 | 29.6 | 80.5 | 0.51 |
| Example 38 | 174 | 46.4 | 42.6 | 89.0 | 0.50 |
| Example 39 | 161 | 50.2 | 39.3 | 89.6 | 0.55 |
| Example 40 | 172 | 45.7 | 41.2 | 86.9 | 0.65 |
| Example 41 | 149 | 52.1 | 37.6 | 89.7 | 0.67 |
| Example 42 | 163 | 48.1 | 40.1 | 88.2 | 0.43 |
| Example 43 | 170 | 44.7 | 41.3 | 86.0 | 0.63 |
| Example 44 | 181 | 46.7 | 41.6 | 88.3 | 0.40 |
| Example 45 | 179 | 46.4 | 40.8 | 87.2 | 0.56 |
| Example 46 | 168 | 52.9 | 31.1 | 84.0 | 0.36 |
| Example 47 | 175 | 46.4 | 38.4 | 84.8 | 0.38 |
| Example 48 | 171 | 50.7 | 30.0 | 80.6 | 0.49 |
| Example 49 | 178 | 45.6 | 42.3 | 87.9 | 0.33 |
| Example 50 | 164 | 48.3 | 38.5 | 86.9 | 0.48 |
| Example 51 | 167 | 46.5 | 43.5 | 90.0 | 0.55 |
| Example 52 | 162 | 51.4 | 37.6 | 89.0 | 0.66 |
| Example 53 | 163 | 47.4 | 39.0 | 86.4 | 0.63 |
| Example 54 | 174 | 46.0 | 42.0 | 88.0 | 0.37 |
| Example 55 | 182 | 46.1 | 41.7 | 87.9 | 0.53 |
| Example 56 | 148 | 59.2 | 23.9 | 83.1 | 0.32 |
| Example 57 | 129 | 63.9 | 15.6 | 79.6 | 0.32 |
| Example 58 | 134 | 58.1 | 20.4 | 78.5 | 0.61 |
| Example 59 | 138 | 64.0 | 12.1 | 76.1 | 0.34 |
| Example 60 | 145 | 57.8 | 25.0 | 82.7 | 0.62 |
| Example 61 | 134 | 60.3 | 21.0 | 81.3 | 0.59 |
| Example 62 | 140 | 57.7 | 23.7 | 81.4 | 0.54 |
| Example 63 | 125 | 62.3 | 20.1 | 82.4 | 0.52 |
| Example 64 | 126 | 59.4 | 21.0 | 80.4 | 0.37 |
| Example 65 | 147 | 57.8 | 25.3 | 83.1 | 0.49 |
| Example 66 | 151 | 59.0 | 23.8 | 82.8 | 0.33 |
| Example 67 | 135 | 39.7 | 45.8 | 85.4 | 0.48 |
| Example 68 | 123 | 45.1 | 39.8 | 84.9 | 0.53 |
| Example 69 | 129 | 38.6 | 46.1 | 84.7 | 0.56 |
| Example 70 | 124 | 43.9 | 37.7 | 81.7 | 0.60 |
| Example 71 | 137 | 38.6 | 51.0 | 89.6 | 0.34 |
| Example 72 | 153 | 39.1 | 47.8 | 86.9 | 0.69 |
| Example 73 | 139 | 45.8 | 40.1 | 86.0 | 0.54 |
| Example 74 | 153 | 38.5 | 48.1 | 86.7 | 0.43 |
| Example 75 | 137 | 44.5 | 37.9 | 82.4 | 0.50 |
| Example 76 | 157 | 37.0 | 50.4 | 87.4 | 0.51 |
| Example 77 | 156 | 49.2 | 37.8 | 86.9 | 0.38 |
| Example 78 | 135 | 54.1 | 29.8 | 83.9 | 0.64 |
| Example 79 | 150 | 49.3 | 37.7 | 87.1 | 0.35 |
| Example 80 | 147 | 53.2 | 28.8 | 81.9 | 0.40 |
| Example 81 | 148 | 46.4 | 40.7 | 87.2 | 0.55 |
| Example 82 | 161 | 48.7 | 36.2 | 84.9 | 0.32 |
| Example 83 | 135 | 54.7 | 29.3 | 83.9 | 0.46 |
| Example 84 | 151 | 48.3 | 38.6 | 86.9 | 0.32 |
| Example 85 | 141 | 53.2 | 26.9 | 80.1 | 0.59 |
| Example 86 | 150 | 48.2 | 40.9 | 89.1 | 0.54 |
| Example 87 | 149 | 42.5 | 45.7 | 88.2 | 0.43 |
| Example 88 | 152 | 39.5 | 48.5 | 88.0 | 0.53 |
| Example 89 | 150 | 41.6 | 44.4 | 86.0 | 0.65 |
| Example 90 | 155 | 37.6 | 49.6 | 87.2 | 0.39 |
| Example 91 | 149 | 38.5 | 48.2 | 86.7 | 0.45 |
| Example 92 | 150 | 39.0 | 50.3 | 89.3 | 0.67 |
| Example 93 | 144 | 40.4 | 47.4 | 87.9 | 0.43 |
| Example 94 | 142 | 38.6 | 48.0 | 86.6 | 0.66 |
| Example 95 | 154 | 36.6 | 50.3 | 86.9 | 0.43 |
| Example 96 | 154 | 38.3 | 50.2 | 88.5 | 0.67 |
| Comparativ e Example 1 | 113 | 52.4 | 38.4 | 90.8 | 2.70 |
| Comparativ e Example 2 | 42 | 45.7 | 43.0 | 88.7 | 1.20 |
| Comparativ e Example 3 | 0 | - | - | - | - |
| Comparativ e Example 4 | 0 | - | - | - | - |

As shown in Table 2 above, it has been confirmed that in a case where the ethylene oligomerization reaction is carried out using a catalyst composition containing the ligand compound according to the present invention, the phenyl located at a terminal of the diphosphino aminyl moiety has a specific substituent at the ortho position and has, at each of the meta position and the para position as a substituent, an alkyl group having a specific number of carbon atoms or a silyl group substituted with an alkyl group having a specific number of carbon atoms, and in addition, the steric strain around the PNP functional group is controlled due to the substituent substituted on the nitrogen atom, which improves all of the catalytic activity, the selectivity, and the stability.

From these results, it has been confirmed that in a case where ethylene oligomerization is carried out using an organic chromium compound and a catalyst composition, which contain the ligand compound according to the present invention, it is possible to produce a linear alpha-olefin with high 1-hexene and 1-octene selectivity while maintaining excellent productivity due to high catalytic activity.

## Claims

1. A ligand compound represented by Chemical Formula 1: 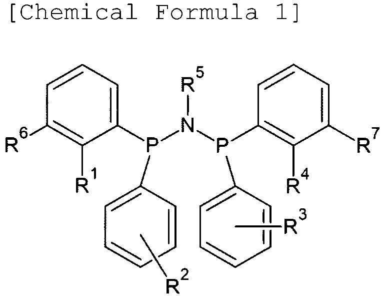 in Chemical Formula 1,
R¹ is a halogen group, an alkyl group having 1 to 10 carbon atoms, which is substituted with a halogen group or unsubstituted, an alkoxy group having 1 to 10 carbon atoms, which is substituted with a halogen group or unsubstituted, an alkylthio group having 1 to 10 carbon atoms, which is substituted with a halogen group or unsubstituted, an alkyl sulfonate group having 1 to 10 carbon atoms, which is substituted with a halogen group or unsubstituted, a trialkylsilyl group (alkyl groups of the trialkylsilyl group are each independently an alkyl group having 1 to 10 carbon atoms), or binds to R⁶ to form a monocyclic or polycyclic aromatic hydrocarbon ring or a monocyclic or polycyclic heterocyclic ring,
R⁶ is hydrogen in a case where R⁶ does not bind to R¹ to form a monocyclic or polycyclic aromatic hydrocarbon ring or a monocyclic or polycyclic heterocyclic ring,
R² and R³ are each independently hydrogen, an alkyl group having 5 to 20 carbon atoms, a cycloalkyl group having 5 to 20 carbon atoms, an alkoxyalkyl group having 2 to 20 carbon atoms, an arylalkoxyalkyl group having 7 to 30 carbon atoms, or a trialkylsilyl group (alkyl groups of the trialkylsilyl group are each independently an alkyl group having 1 to 10 carbon atoms), where R² and R³ are not hydrogen at the same time,
R⁴ is a halogen group, an alkyl group having 1 to 10 carbon atoms, which is substituted with a halogen group or unsubstituted, an alkoxy group having 1 to 10 carbon atoms, which is substituted with a halogen group or unsubstituted, an alkylthio group having 1 to 10 carbon atoms, which is substituted with a halogen group or unsubstituted, an alkyl sulfonate group having 1 to 10 carbon atoms, which is substituted with a halogen group or unsubstituted, a trialkylsilyl group (alkyl groups of the trialkylsilyl group are each independently an alkyl group having 1 to 10 carbon atoms), or binds to R⁷ to form a monocyclic or polycyclic aromatic hydrocarbon ring or a monocyclic or polycyclic heterocyclic ring,
R⁷ is hydrogen in a case where R⁷ does not bind to R⁴ to form a monocyclic or polycyclic aromatic hydrocarbon ring or a monocyclic or polycyclic heterocyclic ring, and
R⁵ is an alkyl group having 1 to 20 carbon atoms, an alkyl group having 1 to 20 carbon atoms, which is substituted with an aryl group having 6 to 10 carbon atoms, a cycloalkyl group having 5 to 10 carbon atoms, or a cycloalkyl group having 5 to 10 carbon atoms, which is fused with an aryl group having 6 to 10 carbon atoms.

2. The ligand compound of claim 1,
wherein R¹ is fluorine, an alkyl group having 1 to 5 carbon atoms, which is substituted with fluorine or unsubstituted, an alkoxy group having 1 to 5 carbon atoms, which is substituted with fluorine or unsubstituted, an alkylthio group having 1 to 5 carbon atoms, which is substituted with fluorine or unsubstituted, an alkyl sulfonate group having 1 to 5 carbon atoms, which is substituted with fluorine or unsubstituted, a trialkylsilyl group (alkyl groups of the trialkylsilyl group are each independently an alkyl group having 1 to 5 carbon atoms), or binds to R⁶ to form a monocyclic or polycyclic heterocyclic ring, and
R⁴ is fluorine, an alkyl group having 1 to 5 carbon atoms, which is substituted with fluorine or unsubstituted, an alkoxy group having 1 to 5 carbon atoms, which is substituted with fluorine or unsubstituted, an alkylthio group having 1 to 5 carbon atoms, which is substituted with fluorine or unsubstituted, an alkyl sulfonate group having 1 to 5 carbon atoms, which is substituted with fluorine or unsubstituted, a trialkylsilyl group (alkyl groups of the trialkylsilyl group are each independently an alkyl group having 1 to 5 carbon atoms), or binds to R⁷ to form a monocyclic or polycyclic heterocyclic ring.

3. The ligand compound of claim 1,
wherein R¹ is a fluoro group, a trifluoromethyl group, a methoxy group, a methyl group, a trifluoromethoxy group, a trifluoromethylthio group, a methylthio group, a methyl sulfonate group, or a trimethylsilyl group, or binds to R⁶ to form furan or dibenzofuran, and
R⁴ is a fluoro group, a trifluoromethyl group, a methoxy group, a methyl group, a trifluoromethoxy group, a trifluoromethylthio group, a methylthio group, a methyl sulfonate group, or a trimethylsilyl group, or binds to R⁷ to form furan or dibenzofuran.

4. The ligand compound of claim 1,
wherein R² and R³ are each independently an alkyl group having 8 to 12 carbon atoms, a tripropylsilyl group, or a tributylsilyl group.

5. The ligand compound of claim 1,
wherein R² and R³ are each independently an n-decyl group, a tripropylsilyl group, or a tributylsilyl group.

6. The ligand compound of claim 1,
wherein R⁵ is an alkyl group having 3 to 5 carbon atoms, an alkyl group having 1 to 5 carbon atoms, which is substituted with an aryl group having 6 to 10 carbon atoms, a cycloalkyl group having 5 to 8 carbon atoms, or a cycloalkyl group having 5 to 8 carbon atoms, which is fused with an aryl group having 6 to 10 carbon atoms.

7. The ligand compound of claim 1,
wherein the ligand compound represented by Chemical Formula 1 is represented by any one of Chemical Formula 2 to Chemical Formula 5: 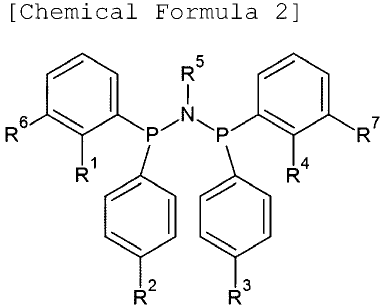 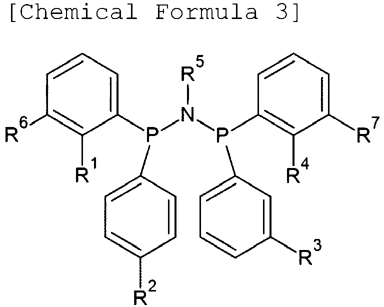 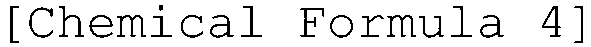 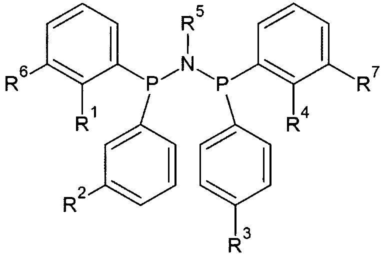 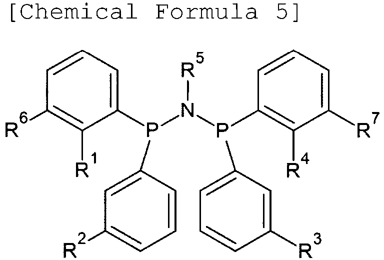
in Chemical Formula 2 to Chemical Formula 5, R¹ to R⁷ are respectively the same as those defined in claim 1.

8. The ligand compound of claim 1,
wherein the ligand compound represented by Chemical Formula 1 is represented by any one of Chemical Formula 2-1 to Chemical Formula 2-76 or Chemical Formula 5-1 to Chemical Formula 5-20:

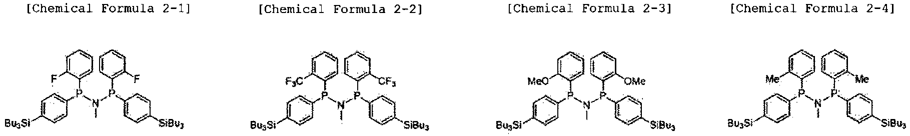

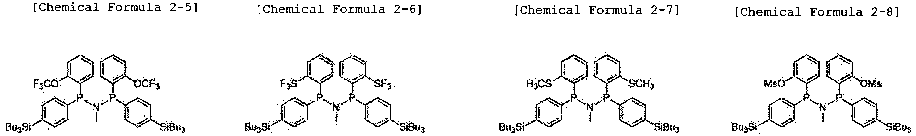

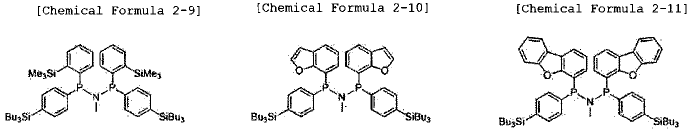

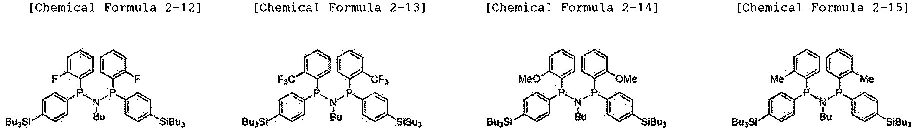

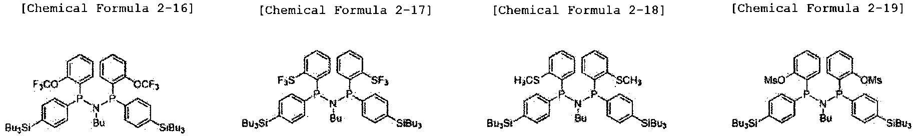

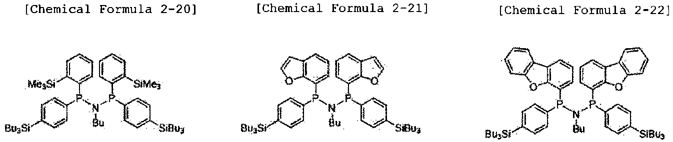

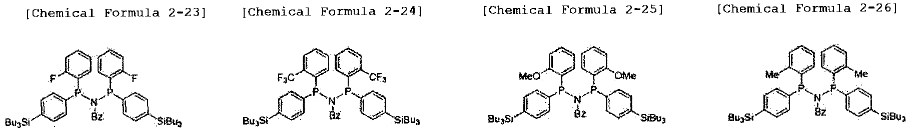

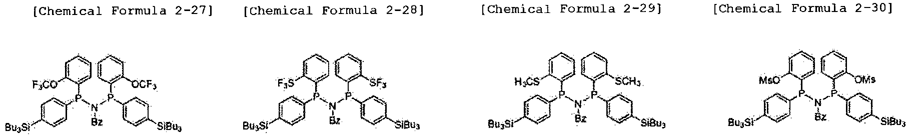

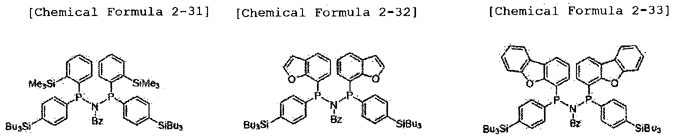

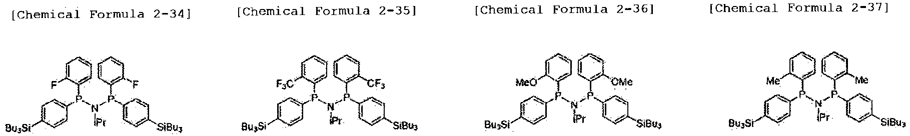

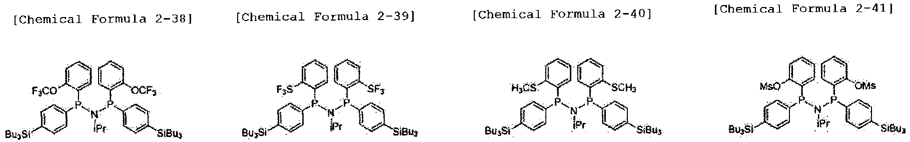

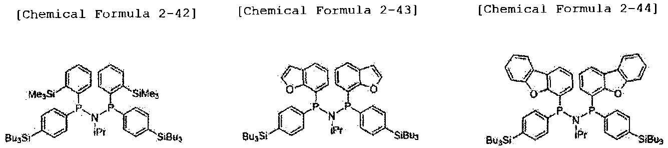

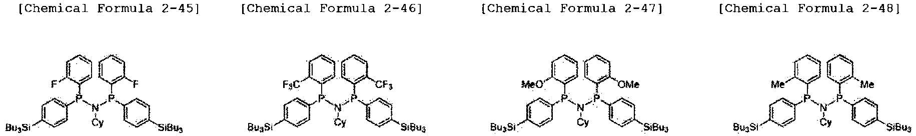

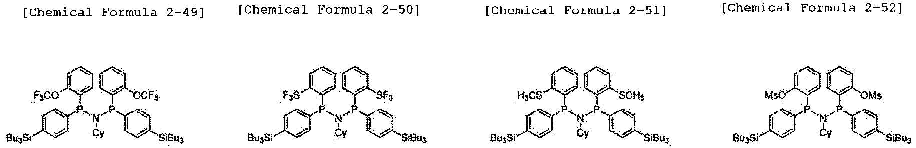

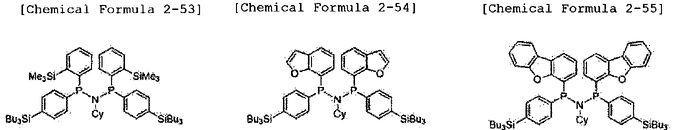

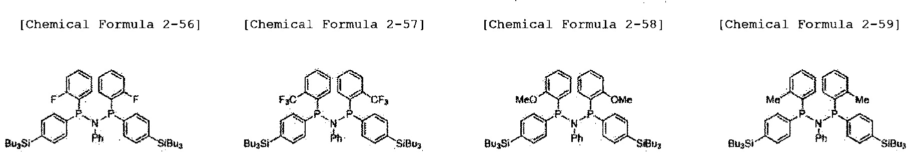

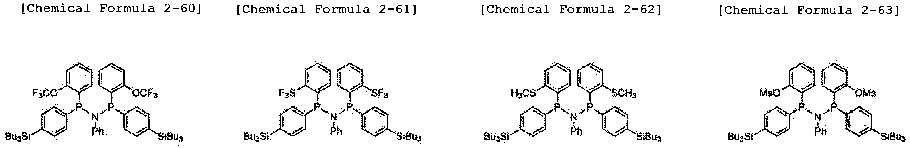

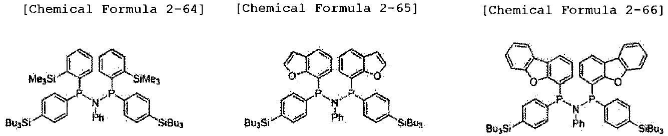

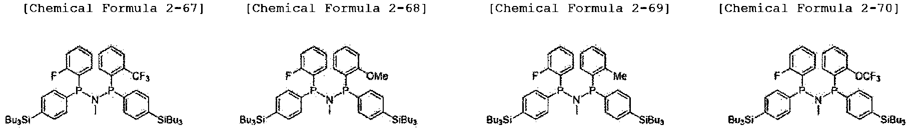

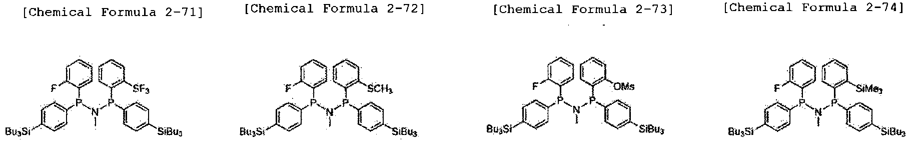

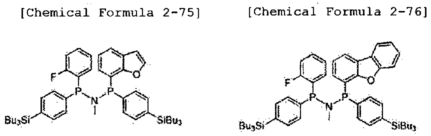

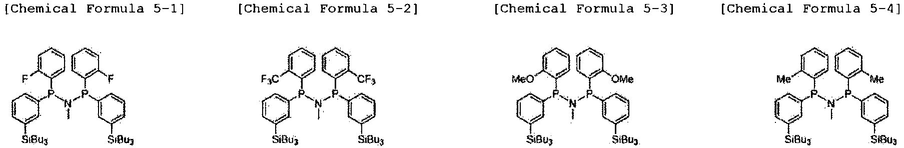

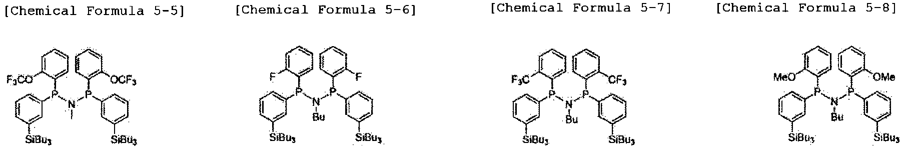

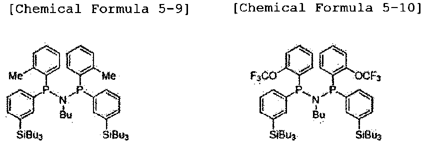

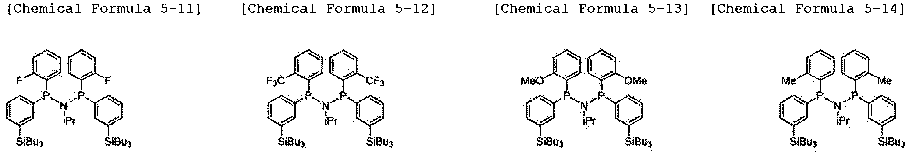

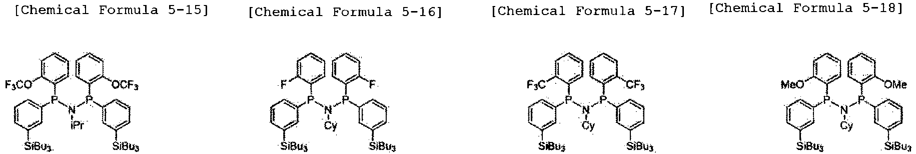

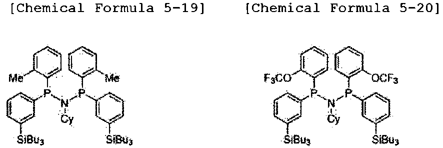

9. An organic chromium compound comprising:
the ligand compound of claim 1; and
chromium coordinated to the ligand compound.

10. The organic chromium compound of claim 9,
wherein the organic chromium compound has a form in which an unshared electron pair of any one or more of N and two P's is coordinated to chromium in the ligand compound represented by Chemical Formula 1.

11. A catalyst composition comprising:
the ligand compound of claim 1;
chromium; and
a co-catalyst.

12. The catalyst composition of claim 11,
wherein the chromium is derived from a chromium source, and
the chromium source contains one or more selected from the group consisting of chromium (III) acetylacetonate, chromium (III) chloride tetrahydrofuran, chromium (III) 2-ethylhexanoate, chromium (III) acetate, chromium (III) butyrate, chromium (III) pentanoate, chromium (III) laurate, chromium (III) tris(2,2,6,6-tetramethyl-3.5-heptanedionate), and chromium (III) stearate.

13. The catalyst composition of claim 11,
wherein the co-catalyst is at least one or more selected from the group consisting of compounds represented by Chemical Formula 6 to Chemical Formula 9:
[Chemical Formula 6] -[Al(R¹³)-O]ₐ-
in Chemical Formula 6,
R¹³'s are each independently a halogen group, a hydrocarbyl group having 1 to 20 carbon atoms, or a hydrocarbyl group having 1 to 20 carbon atoms, which is substituted with a halogen group, and
a is an integer of 2 or more,
[Chemical Formula 7] E(R¹⁴)₃
in Chemical Formula 7,
E is aluminum or boron, and
R¹⁴'s are each independently hydrogen, a halogen group, a hydrocarbyl group having 1 to 20 carbon atoms, or a hydrocarbyl group having 1 to 20 carbon atoms, which is substituted with a halogen group,
[Chemical Formula 8] [L-H]⁺[G(Y)₄]⁻
[Chemical Formula 9] [L]⁺[G(Y)₄]⁻
in Chemical Formulae 8 and 9,
L is a neutral or cationic Lewis acid,
[LH]⁺ is Brønsted acid,
G is a Group 13 element, and
Y's are each independently a substituted or unsubstituted alkyl group having 1 to 20 carbon atoms or a substituted or unsubstituted aryl group having 6 to 20 carbon atoms, where in a case where the alkyl group or the aryl group is substituted, a substituent is a halogen group a hydrocarbyl group having 1 to 20 carbon atoms, an alkoxy group having 1 to 20 carbon atoms, or an aryloxy group having 6 to 20 carbon atoms.

14. A method for producing a linear alpha-olefin, the method comprising:
a step (S10) of oligomerizing ethylene in the presence of the catalyst composition of claim 11.

15. The method of claim 14,
wherein the linear alpha-olefin is 1-hexene, 1-octene, or a mixture thereof
